# EUROPÄISCHE PATENTSCHRIFT

(11) **EP 2 590 930 B1**
(45) Veröffentlichungstag und Bekanntmachung des Hinweises auf die Patenterteilung: **25.02.2015**
(21) Anmeldenummer: 11729315.9
(22) Anmeldetag: 05.07.2011
(51) Int. Cl.: C07C 59/72, C07C 255/54, C07D 213/64, C07D 213/65, C07D 305/06, A61K 31/4439, A61K 31/426, A61K 31/421, A61K 31/34, A61K 31/381, A61K 31/192

(54) **SPIROCYCLISCH SUBSTITUIERTE 1,3-PROPANDIOXIDDERIVATE, VERFAHREN ZU IHRER HERSTELLUNG UND IHRE VERWENDUNG ALS ARZNEIMITTEL**
SPIROCYCLICALLY SUBSTITUTED 1,3-PROPANE DIOXIDE DERIVATIVES, METHODS FOR THE PRODUCTION THEREOF AND USE OF THE SAME AS MEDICAMENT
DÉRIVÉS 1,3-PROPANEDIOXYDE À SUBSTITUTION SPIROCYCLIQUE, PROCÉDÉ DE PRÉPARATION ET UTILISATION COMME MÉDICAMENT

(30) Priorität: 05.07.2010 EP 10305734
(43) Veröffentlichungstag der Anmeldung: 15.05.2013
(73) Patentinhaber: SANOFI, 75013 Paris (FR)
(72) Erfinder: KEIL, Stefanie, 65926 Frankfurt am Main (DE); DEFOSSA, Elisabeth, 65926 Frankfurt am Main (DE); DIETRICH, Viktoria, 65926 Frankfurt am Main (DE); STENGELIN, Siegfried, 65926 Frankfurt am Main (DE); HERLING, Andreas, 65926 Frankfurt am Main (DE); HASCHKE, Guido, 65926 Frankfurt am Main (DE); KLABUNDE, Thomas, 65926 Frankfurt am Main (DE)
(74) Vertreter: Then, Johann
(86) Internationale Anmeldenummer: PCT/EP2011/061336
(87) Internationale Veröffentlichungsnummer: WO 2012/004270

(56) Entgegenhaltungen:
- WO-A1-2005/063729
- WO-A2-2008/054675

## Beschreibung

Die Erfindung betrifft spirocyclisch substituierte 1,3-Propandioxidderivate, sowie deren physiologisch verträgliche Salze.

Es sind bereits strukturähnliche Verbindungen im Stand der Technik beschrieben (siehe WO00/64888) sowie deren Verwendung als PPAR Agonisten oder Antagonisten.

Der Erfindung lag die Aufgabe zugrunde, Verbindungen zur Verfügung zu stellen, die eine therapeutisch verwertbare Wirkung entfalten. Weiter bestand die Aufgabe darin, neue Verbindungen zu finden, die zur Behandlung von Hyperglykämie und von Diabetes geeignet sind. Weiter bestand die Aufgabe darin, neue Verbindungen zu finden, die den GPR40 Rezeptor aktivieren und so zur Behandlung von Hyperglykämie und von Diabetes geeignet sind.

Die Erfindung betrifft daher Verbindungen der Formel I, 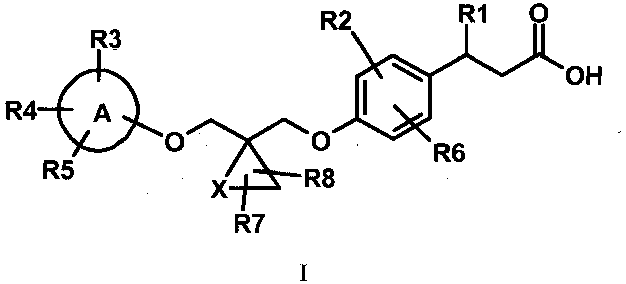 worin bedeuten
- R1: O-(C₁-C₆)-Alkyl, (C₂-C₆)-Alkinyl, (C₂-C₆)-Alkinylen-(C₃-C₁₀)-Cycloalkyl, wobei der O-(C₁-C₆)-Alkylrest, der (C₂-C₆)-Alkinylrest und der (C₂-C₆)-Alkinylen-(C₃-C₁₀)-Cycloalkylrest einfach oder mehrfach mit Fluor substituiert sein können;
- R2, R6: unabhängig voneinander H, F, Cl, Br, CN, (C₁-C₆)-Alkyl, O-(C₁-C₆)-Alkyl, CO-(C₁-C₆)-Alkyl, wobei der (C₁-C₆)-Alkylrest, O-(C₁-C₆)-Alkylrest und der CO-(C₁-C₆)-Alkylrest einfach oder mehrfach mit Fluor substituiert sein können;
- R3, R4,: R5 unabhängig voneinander H, F, Cl, Br, I, NO₂, CN, O-(C₁-C₆)-Alkyl, (C₁-C₆)-Alkyl, (C₁-C₃)-Alkylen-(C₃-C₆)-cycloalkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₁₀)-Cycloalkyl, oder ein 4 bis 12-gliedriger Heterocyclus, wobei der (C₁-C₆)-Alkylrest, der O-(C₁-C₆)-Alkylrest, der (C₁-C₃)-Alkylen-(C₃-C₆)-cycloalkylrest, der NH(C₁-C₆)-Alkylrest, der N((C₁-C₆)-Alkyl)₂rest, der SO₂-CH₃-rest, der SO₂-NH(C₁-C₆)-Alkylrest, SO₂-N((C₁-C₆)-Alkyl)₂-rest, COO-(C₁-C₆)-Alkylrest, der CONH(C₁-C₆)-Alkylrest, und der CON((C₁-C₆)-Alkyl)₂rest jeweils ein oder mehrfach mit F substituiert sein können und wobei der (C₆-C₁₀)-Arylrest, der (C₃-C₁₀)-Cycloalkylrest und der 4 bis 12-gliedrige Heterocyclusrest jeweils ein bis 3-fach substituiert sein können mit
F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, S O₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂ oder SF₅;
- R7, R8: unabhängig voneinander H, F;
- A: (C₆-C₁₀)-Aryl, (C₃-C₁₀)-Cycloalkyl, oder ein 4 bis 12-gliedriger Heterocyclus;
- X: -CH₂-, -CH₂-CH₂-, -CH₂O-, -OCH₂-, -CH₂S, -SCH₂-, -CH₂SO-, -SOCH₂-, -CH₂SO₂-, -SO₂CH₂-;
und deren physiologisch verträgliche Salze.

Eine weitere Ausführungsform betrifft Verbindungen der Formel I, worin ein oder mehrere Reste die folgenden Bedeutungen haben:
- R1: (C₂-C₆)-Alkinyl;
- R2, R6: unabhängig voneinander H, F, Cl, Br, CN, (C₁-C₆)-Alkyl, O-(C₁-C₆)-Alkyl, CO-(C₁-C₆)-Alkyl, wobei der (C₁-C₆)-Alkylrest, O-(C₁-C₆)-Alkylrest und der CO-(C₁-C₆)-Alkylrest einfach oder mehrfach mit Fluor substituiert sein können;
- R3, R4, R5: unabhängig voneinander H, F, Cl, Br, I, NO₂, CN, O-(C₁-C₆)-Alkyl, (C₁-C₆)-Alkyl, (C₁-C₃)-Alkylen-(C₃-C₆)-cycloalkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂ COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₁₀)-Cycloalkyl, oder ein 4 bis 12-gliedriger Heterocyclus, wobei der (C₁-C₆)-Alkylrest, der O-(C₁-C₆)-Alkylrest, der (C₁-C₃)-Alkylen-(C₃-C₆)-cycloalkylrest, der NH(C₁-C₆)-Alkylrest, der N((C₁-C₆)-Alkyl)₂rest, der SO₂-CH₃-rest, der SO₂-NH(C₁-C₆)-Alkylrest, SO₂-N((C₁-C₆)-Alkyl)₂-rest, COO-(C₁-C₆)-Alkylrest, der CONH(C₁-C₆)-Alkylrest, und der CON((C₁-C₆)-Alkyl)₂rest jeweils ein oder mehrfach mit F substituiert sein können und wobei der (C₆-C₁₀)-Arylrest, der (C₃-C₁₀)-Cycloalkylrest und der 4 bis 12-gliedrige Heterocyclusrest jeweils ein bis 3-fach substituiert sein können mit
F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, S O₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂ oder SF₅;
- R7, R8: unabhängig voneinander H, F;
- A: (C₆-C₁₀)-Aryl, (C₃-C₁₀)-Cycloalkyl, oder ein 4 bis 12-gliedriger Heterocyclus;
- X: -CH₂-, -CH₂-CH₂-, -CH₂O-, -OCH₂-, -CH₂S, -SCH₂-, -CH₂SO-, -SOCH₂-, -CH₂SO₂-, -SO₂CH₂-;
und deren physiologisch verträgliche Salze.

Eine weitere Ausführungsform betrifft Verbindungen der Formel I, worin ein oder mehrere Reste die folgenden Bedeutungen haben:
- R1: (C₂-C₆)-Alkinyl;
- R2, R6: unabhängig voneinander H, F, Cl, Br, CN, (C₁-C₆)-Alkyl, O-(C₁-C₆)-Alkyl, CO-(C₁-C₆)-Alkyl, wobei der (C₁-C₆)-Alkylrest, O-(C₁-C₆)-Alkylrest und der CO-(C₁-C₆)-Alkylrest einfach oder mehrfach mit Fluor substituiert sein können;
- R3, R4, R5: unabhängig voneinander H, F, Cl, Br, I, NO₂, CN, O-(C₁-C₆)-Alkyl, (C₁-C₆)-Alkyl, (C₁-C₃)-Alkylen-(C₃-C₆)-cycloalkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₁₀)-Cycloalkyl, oder ein 4 bis 12-gliedriger Heterocyclus, wobei der (C₁-C₆)-Alkylrest, der O-(C₁-C₆)-Alkylrest, der (C₁-C₃)-Alkylen-(C₃-C₆)-cycloalkylrest, der NH(C₁-C₆)-Alkylrest, der N((C₁-C₆)-Alkyl)₂rest, der SO₂-CH₃-rest, der SO₂-NH(C₁-C₆)-Alkylrest, SO₂-N((C₁-C₆)-Alkyl)₂-rest, COO-(C₁-C₆)-Alkylrest, der CONH(C₁-C₆)-Alkylrest, und der CON((C₁-C₆)-Alkylhrestjeweils ein oder mehrfach mit F substituiert sein können und wobei der (C₆-C₁₀)-Arylrest, der (C₃-C₁₀)-Cycloalkylrest und der 4 bis 12-gliedrige Heterocyclusrest jeweils ein bis 3-fach substituiert sein können mit
F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, S O₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂ oder SF₅;
- R7, R8: unabhängig voneinander H, F;
- A: Phenyl, Pyridyl;
- X: -CH₂-, -CH₂-CH₂-, -CH₂O-, -OCH₂-, -CH₂S, -SCH₂-, -CH₂SO-, -SOCH₂-, -CH₂SO₂-, -SO₂CH₂-;
und deren physiologisch verträgliche Salze.

Eine weitere Ausführungsform betrifft Verbindungen der Formel I, worin ein oder mehrere Reste die folgenden Bedeutungen haben:
- R1: (C₂-C₆)-Alkinyl;
- R2, R6: H;
- R3, R4: unabhängig voneinander H, F, Cl, Br, I, CF₃, CN, CF₃, OCF₃, (C₁-C₆)-Alkyl;
- R5: H;
- R7, R8: H;
- A: Phenyl, Pyridyl;
- X: -CH₂-, -CH₂-CH₂-;
und deren physiologisch verträgliche Salze.

Eine weitere Ausführungsform betrifft Verbindungen der Formel I, worin ein oder mehrere Reste die folgenden Bedeutungen haben:
- R1: (C₂-C₆)-Alkinyl;
- R2, R6: H;
- R3, R4: unabhängig voneinander H, F, Cl, Br, I, CF₃, CN, CF₃, OCF₃, (C₁-C₆)-Alkyl;
- R5: H;
- R7, R8: H;
- A: Phenyl;
- X: -CH₂-, -CH₂-CH₂-;
und deren physiologisch verträgliche Salze.

Eine weitere Ausführungsform betrifft Verbindungen der Formel I, worin ein oder mehrere Reste die folgenden Bedeutungen haben:
- R1: -(C₂-C₆)-Alkinyl;
- R2, R6: H;
- R3, R4: unabhängig voneinander H, F, Cl, Br, I, CF₃, CN, CF₃, OCF₃, (C₁-C₆)-Alkyl;
- R5: H;
- R7, R8: H;
- A: Pyridyl;
- X: -CH₂-, -CH₂-CH₂-;
und deren physiologisch verträgliche Salze.

Eine weitere Ausführungsform betrifft Verbindungen der Formel I, worin ein oder mehrere Reste die folgenden Bedeutungen haben:
- R1: -(C₂-C₆)-Alkinyl;
- R2, R6: H;
- R3, R4: unabhängig voneinander H, F, Cl, Br, I, CF₃, CN, CF₃, OCF₃, (C₁-C₆)-Alkyl;
- R5: H;
- R7, R8: H;
- A: Phenyl;
- X: -CH₂-;
und deren physiologisch verträgliche Salze.

Eine weitere Ausführungsform betrifft Verbindungen der Formel I, worin ein oder mehrere Reste die folgenden Bedeutungen haben:
- R1: -(C₂-C₆)-Alkinyl;
- R2, R6: H;
- R3, R4: unabhängig voneinander H, F, Cl, Br, I, CF₃, CN, CF₃, OCF₃, (C₁-C₆)-Alkyl;
- R5: H;
- R7, R8: H;
- A: Pyridyl;
- X: -CH₂-;
und deren physiologisch verträgliche Salze.

Eine weitere Ausführungsform betrifft Verbindungen der Formel I, worin ein oder mehrere Reste die folgenden Bedeutungen haben:
- R1: (C₂-C₆)-Alkinyl;
- R2, R6: H;
- R3, R4: unabhängig voneinander H, F, Cl, Br, I, CF₃, CN, CF₃, OCF₃, (C₁-C₆)-Alkyl;
- R5: H;
- R7, R8: H;
- A: Phenyl;
- X: -CH₂-, -CH₂-CH₂-;
und deren physiologisch verträgliche Salze.

Eine weitere Ausführungsform betrifft Verbindungen der Formel I, worin ein oder mehrere Reste die folgenden Bedeutungen haben:
- R1: (C₂-C₆)-Alkinyl;
- R2, R6: H;
- R3, R4: unabhängig voneinander H, F, Cl, Br, I, CF₃, CN, CF₃, OCF₃, (C₁-C₆)-Alkyl;
- R5: H;
- R7, R8: H;
- A: 2-Pyridyl, 3-Pyridyl;
- X: -CH₂-, -CH₂-CH₂-;
und deren physiologisch verträgliche Salze.

Eine weitere Ausführungsform betrifft Verbindungen der Formel I, worin ein oder mehrere Reste die folgenden Bedeutungen haben:
- X: -CH₂-;
und alle anderen Gruppen und Zahlen wie in der allgemeinen Definition der Verbindungen der Formel I oder in einer der angegebenen Ausführungsformen der Erfindung oder Definitionen von Strukturelementen definiert sind.

Eine weitere Ausführungsform betrifft Verbindungen der Formel I, worin ein oder mehrere Reste die folgenden Bedeutungen haben:
- X: - CH₂-CH₂-;
und alle anderen Gruppen und Zahlen wie in der allgemeinen Definition der Verbindungen der Formel I oder in einer der angegebenen Ausführungsformen der Erfindung oder Definitionen von Strukturelementen definiert sind.

Eine weitere Ausführungsform betrifft Verbindungen der Formel I, worin ein oder mehrere Reste die folgenden Bedeutungen haben:
- R1: Propinylrest;
und alle anderen Gruppen und Zahlen wie in der allgemeinen Definition der Verbindungen der Formel I oder in einer der angegebenen Ausführungsformen der Erfindung oder Definitionen von Strukturelementen definiert sind.

Eine weitere Ausführungsform betrifft Verbindungen der Formel I, worin ein oder mehrere Reste die folgenden Bedeutungen haben:
- R1: -OCH₃;
und alle anderen Gruppen und Zahlen wie in der allgemeinen Definition der Verbindungen der Formel I oder in einer der angegebenen Ausführungsformen der Erfindung oder Definitionen von Strukturelementen definiert sind.

Eine weitere Ausführungsform betrifft Verbindungen der Formel I, worin ein oder mehrere Reste die folgenden Bedeutungen haben:
- A: Phenylrest;
und alle anderen Gruppen und Zahlen wie in der allgemeinen Definition der Verbindungen der Formel I oder in einer der angegebenen Ausführungsformen der Erfindung oder Definitionen von Strukturelementen definiert sind.

Eine weitere Ausführungsform betrifft Verbindungen der Formel I, worin ein oder mehrere Reste die folgenden Bedeutungen haben:
- A: 2-Pyridyl, 3-Pyridyl;
und alle anderen Gruppen und Zahlen wie in der allgemeinen Definition der Verbindungen der Formel I oder in einer der angegebenen Ausführungsformen der Erfindung oder Definitionen von Strukturelementen definiert sind.

Können Reste oder Substituenten mehrfach in den Verbindungen der Formeln I auftreten, so können sie alle unabhängig voneinander die angegebene Bedeutung haben und gleich oder verschieden sein.

Die Alkyl- und Alkinylreste in den Resten R1, R2, R3, R4, R5 und R6 können sowohl geradkettig wie verzweigt sein.

Die Erfindung betrifft Verbindungen der Formel I in Form ihrer Salze, Racemate, racemischen Mischungen und reinen Enantiomere, und deren Diastereomere und Mischungen davon.

Gegenstand der Erfindung sind weiterhin sowohl Stereoisomerengemische der Formel I, als auch die reinen Stereoisomere der Formel I, sowie Diastereomerengemische der Formel I als auch die reinen Diastereomere. Die Trennung der Gemische erfolgt z.B. auf chromatographischem Weg.

Die vorliegende Erfindung umfasst alle möglichen tautomeren Formen der Verbindungen der Formel I.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren sowie organischer Säuren

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstoffkette verstanden, wie z.B. Methyl, Ethyl, iso-Propyl, tert.-Butyl, Hexyl. Die Alkylreste können einfach oder mehrfach wie oben beschrieben substituiert sein.

Die Erfindung umfasst auch Solvate, Hydrate und Alkoholaddukte der Verbindungen der Formel I.

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 100 mg, typischerweise von 1 ng bis 100 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:
Alle Antidiabetika, die in der Roten Liste 2010, Kapitel 12 genannt sind; alle Abmagerungsmittel/Appetitzügler, die in der Roten Liste 2010, Kapitel 1 genannt sind; alle Diuretika, die in der Roten Liste 2010, Kapitel 36 genannt sind; alle Lipidsenker, die in der Roten Liste 2010, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Erfolgt die Gabe der Wirkstoffe durch getrennte Verabreichung der Wirkstoffe, so kann diese gleichzeitig oder nacheinander erfolgen. Die meisten der nachfolgend aufgeführten Wirkstoffe sind in USP Dictionary of USAN and International Drug Names, US Pharmacopeia, Rockville 2006, offenbart.

Antidiabetika umfassen Insulin und Insulinderivate, wie z.B. Lantus^{®} (siehe www.lantus.com) oder HMR 1964 oder Levemir® (insulin detemir), Humalog^{(R)} (Insulin Lispro), Insulin Degludec, Insulin Aspart, poly-Ethylen-glycosidiertes (PEGyliertes) Insulin Lispro wie in WO2009152128 beschrieben, Humulin^{(R)}, VIAject™, SuliXen^{(R)}, VIAject™ oder solche, wie sie in WO2005005477 (Novo Nordisk) beschrieben sind, schnell wirkende Insuline (siehe US 6,221,633), inhalierbare Insuline, wie z. B. Exubera^{®}, Nasulin™, oder orale Insuline, wie z. B. IN-105 (Nobex) oder Oral-lyn™ (Generex Biotechnology) oder Technosphere^{(R)} Insulin (MannKind) oder Cobalamin™ orales Insulin oder ORMD-0801 oder Insuline oder Insulinvorstufen (insulin precursors), wie sie in WO2007128815, WO2007128817, WO2008034881, WO2008049711, WO2008145721, WO2009034117, WO2009060071, WO2009133099 beschrieben sind oder Insuline, die transdermal verabreicht werden können; daneben sind auch umfasst solche Insulinderivate, die durch einen bifunktionellen Linker an Albumin gebunden sind wie sie z.B. in WO2009121884 beschrieben sind;

GLP-1-Derivate und GLP-1 Agonisten wie z.B. Exenatide oder spezielle Zubereitungen davon, wie sie z.B. in WO2008061355, WO2009080024, WO2009080032 beschrieben sind, Liraglutide, Taspoglutide (R-1583), Albiglutide, Lixisenatide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), inhalierbares GLP-1 (MKC-253 der Firma MannKind), AVE-0010, BIM-51077 (R-1583, ITM-077), PC-DAC:Exendin-4 (ein Exendin-4 Analogon, welches kovalent an rekombinantes menschliches Albumin gebunden ist), biotinyliertes Exendin (WO2009107900), eine spezielle Formulierung von Exendin-4 wie sie in US2009238879 beschrieben ist, CVX-73, CVX-98 und CVx-96 (GLP-1 Analoga, welche kovalent an einen monoklonalen Antikörper gebunden sind, der spezifische Bindungsstellen für das GLP-1 Peptid aufweist), CNTO-736 (ein GLP-1 Analogon, welches an eine Domäne gebunden ist, welche den Fc-Teil eines Antikörpers beinhaltet), PGC-GLP-1 (GLP-1 gebunden an einen Nanocarrier), Agonisten oder Modulatoren wie sie z.B. bei D. Chen et al., Proc. Natl. Acad. Sci. USA 104 (2007) 943 beschrieben sind, solche wie sie in WO2006124529, WO2007124461, WO2008062457, WO2008082274, WO2008101017, WO2008081418, WO2008112939, WO2008112941, WO2008113601, WO2008116294, WO2008116648, WO2008119238, WO2008148839, US2008299096, WO2008152403, WO2009030738, WO2009030771, WO2009030774, WO2009035540, WO2009058734, WO2009111700, WO2009125424, WO2009129696, WO2009149148 beschrieben sind, Peptide wie z.B. Obinepitide (TM-30338), oral wirksame GLP-1 Analoga (z.B. NN9924 von Novo Nordisk), Amylinrezeptor Agonisten, wie sie z.B. in WO2007104789, WO2009034119 beschrieben sind, Analoga des humanen GLP-1, wie sie in WO2007120899, WO2008022015, WO2008056726 beschrieben sind, chimäre pegylierte Peptide, die sowohl GLP-1- wie auch Glucagonreste enthalten und wie sie z.B. in WO2008101017, WO2009155257, WO2009155258 beschrieben sind, glycosylierte GLP-1 Derivate wie sie in WO2009153960 beschrieben sind, sowie oral wirksame hypoglykämische Wirkstoffe.

Antidiabetika umfassen auch Gastrinanaloga wie z.B. TT-223.

Weiterhin umfassen Antidiabetika poly- oder monoklonale Antikörper, welche z.B. gegen Interleukin-1-beta (IL-1ß), wie z.B. XOMA-052, gerichtet sind.

Antidiabetika umfassen weiterhin Peptide, welche an den humanen Pro-Insel Peptidrezeptor (human pro-islet petide (HIP) receptor) binden können wie sie z.B. in WO2009049222 beschrieben sind.

Antidiabetika umfassen auch Agonisten des Glukose-abhängigen insulinotropen Polypeptids (GIP) Rezeptors wie sie z.B. in WO2006121860 beschrieben sind.

Antidiabetika umfassen auch das Glukose-abhängige insulinotrope Polypeptid (GIP) wie auch analoge Verbindungen wie sie z.B. in WO2008021560, WO2010016935, WO2010016936, WO2010016938, WO2010016940, WO2010016944 beschrieben sind.

Weiterhin eingeschlossen sind Analoga und Derivate des humanen pankreatischen Polypeptids (human pancreatic polypeptide) wie sie z.B. in WO2009007714 beschrieben sind.

Antidiabetika umfassen ferner verkapselte Insulin-produzierende Schweinezellen wie z.B. Diabecell(R).

Antidiabetika umfassen auch Analoga und Derivate des Fibroblastenwachstumsfaktors 21 (FGF-21, fibroblast growth factor 21) wie sie z.B. in WO2009149171, WO2010006214 beschrieben sind.

Die oral wirksamen hypoglykämischen Wirkstoffe umfassen vorzugsweise Sulfonylharnstoffe, Biguanidine,
Meglitinide,
Oxadiazolidindione,
Thiazolidindione,
PPAR- und RXR-Modulatoren,
Glukosidase-Inhibitoren,
Hemmstoffe der Glykogenphosphorylase,
Glucagonrezeptor-Antagonisten,
Glukokinaseaktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT),
GLP-1-Agonisten, I
Kaliumkanalöffner, wie z.B. Pinacidil, Cromakalim, Diazoxid, Diazoxid Cholinsalz oder solche wie sie bei R. D. Carr et al., Diabetes 52, 2003, 2513.2518, bei J. B. Hansen et al, Current Medicinal Chemistry 11, 2004, 1595-1615, bei T. M. Tagmose et al., J. Med. Chem. 47, 2004, 3202-3211 oder bei M. J. Coghlan et al., J. Med. Chem. 44, 2001, 1627-1653 beschrieben sind, oder diejenigen, die in WO 97/26265 und WO 99/03861 von Novo Nordisk A/S offenbart wurden,
Wirkstoffe, die auf den ATP-abhängigen Kaliumkanal der Betazellen wirken,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufhahme, des Glukosetransports und der Glukoserückresorption, Modulatoren der natrium-abhängigen Glukosetransporter 1 oder 2 (SGLT1, SGLT2),
Hemmstoffe der 11-beta-Hydroxysteroid-Dehydrogenase-1 (11β-HSD1),
Inhibitoren der Protein-Tyrosin-Phosphatase-1B (PTP-1B),
Nikotinsäurerezeptoragonisten,
Inhibitoren der hormon-sensitiven bzw. endothelialen Lipasen,
Hemmstoffen der Acetyl-CoA Carboxylase (ACC1 und/oder ACC2) oder Inhibitoren der GSK-3 beta.
Weiterhin sind umfasst den Fettstoffwechsel verändernde Verbindungen wie antihyperlipidämische Wirkstoffe und antilipidämische Wirkstoffe,
HMGCoA-Reduktase-Inhibitoren,
Famesoid X Rezeptor (FXR) Modulatoren,
Fibrate,
Cho lesterinresreptionsinhibitoren,
CETP-Inhibitoren,
Gallensäureresorptionsinhibitoren,
MTP-Inhibitoren,
Agonisten des Estrogenrezeptors gamma (ERR y Agonisten),
Sigma-1 Rezeptorantagonisten,
Antagonisten des Somatostatin 5 Rezeptors (SST5 Rezeptor);
Verbindungen, die die Nahrungsmitteleinnahme verringern und
Verbindungen, die die Thermogenese erhöhen.

Bei einer Ausführungsform der Erfindung wird die Verbindung der Formel I in Kombination mit Insulin verabreicht.

Bei einer anderen Ausführungsform der Erfindung wird die verbindung der Formel I in Kombination mit einem Insulin-Sensitizer wie z.B. PN-2034 oder ISIS-113715 verabreicht.

Bei einer Ausführungsform wird die Verbindung der Formel I in Kombination mit einem Wirkstoff, der auf den ATP-abhängigen Kaliumkanal der Betazellen wirkt, z.B. Sulfonylharnstoffe, wie z.B. Tolbutamid, Glibenclamid, Glipizid, Gliclazide oder Glimepirid oder solche Zubereitungen wie sie z.B. in EP2103302 beschrieben sind, verabreicht.

Bei einer Ausführungsform wird die Verbindung der Formel I in Kombination mit einer Tablette verabreicht, die sowohl Glimeprid enthält, welches schnell freigesetzt wird wie auch Metformin enthält, welches über einen längeren Zeitraum freigesetzt wird (wie z.B. in US2007264331, WO2008050987, WO2008062273 beschrieben).

Bei einer Ausführungsform wird die Verbindung der Formel I in Kombination mit einem Biguanid, wie z.B. Metformin oder einem seiner Salze, verabreicht.

Bei einer weiteren Ausführungsform wird die Verbindung der Formel I in Kombination mit einem Guanidin, wie z.B. Benzylguanidin oder einem seiner Salze, oder solchen Guanidinen wie sie in WO2009087395 beschrieben sind, verabreicht.

Bei wieder einer Ausführungsform wird die Verbindung der Formel I in Kombination mit einem Meglitinid, wie z.B. Repaglinide, Nateglinide oder Mitiglinide verabreicht.

Bei einer weiteren Ausführungsform wird die Verbindung der Formel I mit einer Kombination von Mitiglinide mit einem Glitazon, z.B. Pioglitazon Hydrochlorid, verabreicht.

Bei einer weiteren Ausführungsform wird die Verbindung der Formel I mit einer Kombination von Mitiglinide mit einem alpha-Glukosidaseinhibitor verabreicht.

Bei einer weiteren Ausführungsform wird die Verbindung der Formel I in Kombination mit antidiabetischen Verbindungen, wie sie in WO2007095462, WO2007101060, WO2007105650 beschrieben sind, verabreicht.

Bei einer weiteren Ausführungsform wird die Verbindung der Formel I in Kombination mit antihypoglykämischen Verbindungen, wie sie in WO2007137008, WO2008020607 beschrieben sind, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon), DRL-17564, DRF-2593 (Balaglitazon), INT-131, T-2384 oder solchen, wie sie in WO2005086904, WO2007060992, WO2007100027, WO2007103252, WO2007122970, WO2007138485, WO2008006319, WO2008006969, WO2008010238, WO2008017398, WO2008028188, WO2008066356, WO2008084303, WO2008089461-WO2008089464, WO2008093639, WO2008096769, WO2008096820, WO2008096829, US2008194617, WO2008099944, WO2008108602, WO2008109334, WO2008110062, WO2008126731, WO2008126732, WO2008137105, WO2009005672, WO2009038681, WO2009046606, WO2009080821, WO2009083526, WO2009102226, WO2009128558, WO2009139340 beschrieben sind, verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit Tandemact™, einer festen Kombination von Pioglitazon mit Glimeprid, verabreicht.

Bei einer weiteren Ausführungsform der Erfindung wird die Verbindung der Formel I in Kombination mit einer festen Kombination von Pioglitazon Hydrochlorid mit einem Angiotensin II Agonisten, wie z.B. TAK-536, verabreicht.

Bei einer Ausführungsform der Erfindung wird die Verbindung der Formel I in Kombination mit einem PPAR alpha Agonisten bzw. gemischten PPAR alpha/PPAR delta Agonisten, wie z.B. GW9578, GW-590735, K-111, LY-674, KRP-101, DRF-10945, LY-518674, CP-900691, BMS-687453, BMS-711939 oder solchen wie sie in WO2001040207, WO2002096894, WO2005097076, WO2007056771, WO2007087448, WO2007089667, WO2007089557, WO2007102515, WO2007103252, JP2007246474, WO2007118963, WO2007118964, WO2007126043, WO2008006043, WO2008006044, WO2008012470, WO2008035359, WO2008087365, WO2008087366, WO2008087367, WO2008117982, JP2009023975, WO2009033561, WO2009047240, WO2009072581, WO2009080248, WO2009080242, WO2009149819, WO2009149820, WO2009147121, WO2009153496, WO2010008299, WO2010014771 beschrieben sind, verabreicht.

Bei einer Ausführungsform der Erfindung wird die Verbindung der Formel I in Kombination mit einem gemischten PPAR alpha/gamma Agonisten, wie z.B. Naveglitazar, Aleglitazar, LY-510929, ONO-5129, E-3030, AVE 8042, AVE 8134, AVE 0847, CKD-501 (Lobeglitazon Sulfat), MBX-213, KY-201, BMS-759509 oder wie in WO 00/64888, WO 00/64876, WO03/020269, WO2004024726, WO2007099553, US2007276041, WO2007085135, WO2007085136, WO2007141423, WO2008016175, WO200805333.1, WO2008109697, WO2008109700, WO2008108735, WO2009026657, WO2009026658, WO2009149819, WO2009149820 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 oder wie sie in WO2006059744, WO2006084176, WO2006029699, WO2007039172-WO2007039178, WO2007071766, WO2007101864, US2007244094, WO2007119887, WO2007141423, US2008004281, WO2008016175, WO2008066356, WO2008071311, WO2008084962, US2008176861, WO2009012650, US2009137671, WO2009080223, WO2009149819, WO2009149820, WO2010000353 beschrieben sind, verabreicht.

Bei einer Ausführungsform der Erfindung wird die Verbindung der Formel I in Kombination mit einem pan-SPPARM (selective PPAR modulator alpha, gamma, delta), wie z.B. GFT-505, Indeglitazar oder solchen wie sie in WO2008035359, WO2009072581 beschrieben sind, verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht.

Bei einer Ausführungsform wird die Verbindung der Formel I in Kombination mit einem α-Glukosidase-Inhibitor, wie z.B. Miglitol oder Acarbose oder solchen, wie sie z.B. in WO2007114532, WO2007140230, US2007287674, US2008103201, WO2008065796, WO2008082017, US2009076129 beschrieben sind, verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932, WO2008062739, WO2008099000, WO2008113760, WO2009016118, WO2009016119, WO2009030715, WO2009045830, WO2009045831, WO2009127723 beschrieben, verabreicht.

Bei einer anderen Ausführungsform wird die Verbindung der Formel I in Kombination mit einem Inhibitor der Interaktion der Leberglykogenphosphorylase mit dem Protein PPP1R3 (GL-Untereinheit der Glykogen-assoziierten Proteinphosphatase 1 (PP1)), wie z.B. in WO2009030715 beschrieben, verabreicht.

Bei einer Ausführungsform wird die Verbindung der Formel I in Kombination mit GlucagonRezeptor-Antagonisten, wie z.B. A-770077 oder NNC-25-2504 oder wie in WO2004100875, WO2005065680, WO2006086488, WO2007047177, WO2007106181, WO2007111864, WO2007120270, WO2007120284, WO2007123581, WO2007136577, WO2008042223, WO2008098244, WO2009057784, WO2009058662, WO2009058734, WO2009110520, WO2009120530, WO2009140342, WO2010019828 beschrieben, verabreicht.

Bei einer weiteren Ausführungsform wird die Verbindung der Formel I in Kombination mit einer Antisense-Verbindung, z.B. ISIS-325568, verabreicht, welche die Produktion des Glucagonrezeptors inhibiert.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923, WO2006112549, WO2006125972, WO2007017549, WO2007017649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847, WO2007061923, WO2007075847, WO2007089512, WO2007104034, WO2007117381, WO2007122482, WO2007125103, WO2007125105, US2007281942, WO2008005914, WO2008005964, WO2008043701, WO2008044777, WO2008047821, US2008096877, WO2008050117, WO2008050101, WO2008059625, US2008146625, WO2008078674, WO2008079787, WO2008084043, WO2008084044, WO2008084872, WO2008089892, WO2008091770, WO2008075073, WO2008084043, WO2008084044, WO2008084872, WO2008084873, WO2008089892, WO2008091770, JP2008189659, WO2008104994, WO2008111473, WO2008116107, WO2008118718, WO2008120754, US2008280875, WO2008136428, WO2008136444, WO2008149382, WO2008154563, WO2008156174, WO2008156757, US2009030046, WO2009018065, WO2009023718, WO2009039944, WO2009042435, WO2009046784, WO2009046802, WO2009047798, WO2009063821, WO2009081782, WO2009082152, WO2009083553, WO2009091014, US2009181981, WO2009092432, WO2009099080, WO2009106203, WO2009106209, WO2009109270, WO2009125873, WO2009127544, WO2009127546, WO2009128481, WO2009133687, WO2009140624, WO2010013161, WO2010015849, WO2010018800 beschrieben sind, verabreicht.

Bei einer Ausführungsform wird die Verbindung der Formel I in Kombination mit einem Inhibitor der Glukoneogenese, wie sie z. B. in FR-225654, WO2008053446 beschrieben sind, verabreicht.

Bei einer Ausführungsform wird die Verbindung der Formel I in Kombination mit Inhibitoren der Fructose-1,6-bisphosphatase (FBPase) wie z.B. MB-07729, CS-917 (MB-06322) oder MB-07803 oder solchen wie sie in WO2006023515, WO2006104030, WO2007014619, WO2007137962, WO2008019309, WO2008037628, WO2009012039, EP2058308, WO2009068467, WO2009068468 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Sitagliptin Phosphat, Saxagliptin (BMS-477118), GSK-823093, PSN-9301, SYR-322, SYR-619, TA-6666, TS-021, GRC-8200 (Melogliptin), GW-825964X, KRP-104, DP-893, ABT-341, ABT-279 oder ein anderes Salz davon, S-40010, S-40755, PF-00734200, BI-1356, PHX-1149, DSP-7238, Alogliptin Benzoat, Linagliptin, Melogliptin, Carmegliptin oder solchen Verbindungen wie sie in WO2003074500, WO2003106456, WO2004037169, WO200450658, WO2005037828, WO2005058901, WO2005012312, WO2005/012308, WO2006039325, WO2006058064, WO2006015691, WO2006015701, WO2006015699, WO2006015700, WO2006018117, WO2006099943, WO2006099941, JP2006160733, WO2006071752, WO2006065826, WO2006078676, WO2006073167, WO2006068163, WO2006085685, WO2006090915, WO2006104356, WO2006127530, WO2006111261, US2006890898, US2006803357, US2006303661, WO2007015767 (LY-2463665), WO2007024993, WO2007029086, WO2007063928, WO2007070434, WO2007071738, WO2007071576, WO2007077508, WO2007087231, WO2007097931, WO2007099385, WO2007100374, WO2007112347, WO2007112669, WO2007113226, WO2007113634, WO2007115821, WO2007116092, US2007259900, EP1852108, US2007270492, WO2007126745, WO2007136603, WO2007142253, WO2007148185, WO2008017670, US2008051452, WO2008027273, WO2008028662, WO2008029217, JP2008031064, JP2008063256, WO2008033851, WO2008040974, WO2008040995, WO2008060488, WO2008064107, WO2008066070, WO2008077597, JP2008156318, WO2008087560, WO2008089636, WO2008093960, WO2008096841, WO2008101953, WO2008118848, WO2008119005, WO2008119208, WO2008120813, WO2008121506, WO2008130151, WO2008131149, WO2009003681, WO2009014676, WO2009025784, WO2009027276, WO2009037719, WO2009068531, WO2009070314, WO2009065298, WO2009082134, WO2009082881, WO2009084497, WO2009093269, WO2009099171, WO2009099172, WO2009111239, WO2009113423, WO2009116067, US2009247532, WO2010000469, WO2010015664 beschrieben sind, verabreicht.

Bei einer Ausführungsform wird die Verbindung der Formel I in Kombination mit Janumet™, einer festen Kombination von Sitagliptin Phosphat mit Metformin Hydrochlorid, verabreicht.

Bei einer Ausführungsform wird die Verbindung der Formel I in Kombination mit Eucreas^{®}, einer festen Kombination von Vildagliptin mit Metformin Hydrochlorid, verabreicht.

Bei einer weiteren Ausführungsform wird die Verbindung der Formel I in Kombination mit einer festen Kombination von Alogliptin Benzoat mit Pioglitazone verabreicht.

Bei einer Ausführungsform wird die Verbindung der Formel I in Kombination mit einer festen Kombination von eines Salzes von Sitagliptin mit Metformin Hydrochlorid, verabreicht.

Bei einer Ausführungsform wird die Verbindung der Formel I in Kombination mit einer Kombination eines DPP-IV-Inhibitors mit omega-3-Fettsäuren oder omega-3-Fettsäureestern, wie z.B. in WO2007128801 beschrieben, verabreicht.

Bei einer Ausführungsform wird die Verbindung der Formel I in Kombination mit einer Kombination eines DPP-IV-Inhibitors mit Metformin Hydrochlorid, wie z.B. in WO2009121945 beschrieben, verabreicht.

Bei einer Ausführungsform wird die Verbindung der Formel I in Kombination mit einer Kombination eines DPP-IV-Inhibitors mit einem GPR-119-Agonisten, wie z.B. in WO2009123992 beschrieben, verabreicht.

Bei einer Ausführungsform wird die Verbindung der Formel I in Kombination mit einer Kombination eines DPP-IV-Inhibitors mit Miglitol, wie z.B. in WO2009139362 beschrieben, verabreicht.

Bei einer Ausführungsform wird die Verbindung der Formel I in Kombination mit einer festen Kombination von einem Salz von Sitagliptin mit Metformin Hydrochlorid verabreicht.

Bei einer Ausführungsform wird die Verbindung der Formel I in Kombination mit einer festen Kombination von Alopliptin Benzoat mit Pioglitazon Hydrochlorid verabreicht.

Bei einer Ausführungsform wird die Verbindung der Formel I in Kombination mit einer die Insulinsekretion verstärkende Substanz, wie z. B. KCP-265 (WO2003097064), oder solchen wie sie in WO2007026761, WO2008045484, US2008194617, WO2009109259, WO2009109341 beschrieben sind, verabreicht.

Bei einer Ausführungsform wird die Verbindung der Formel I in Kombination mit Agonisten des glucose-abhängigen insulinotropischen Rezeptors (GDIR) wie z. B. APD-668 verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform wird die Verbindung der Formel I in Kombination mit Modulatoren des natrium-abhängigen Glukosetransporters 1 und/oder 2 (SGLT1, SGLT2), wie z.B. KGA-2727, T-1095, SGL-0010, AVE 2268, SAR 7226, SGL-5083, SGL-5085, SGL-5094, ISIS-388626, Sergliflozin, Dapagliflozin oder Remogliflozin Etanobat, Canagliflozin oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895, WO2007080170, WO2007093610, WO2007126117, WO2007128480, WO2007129668, US2007275907, WO2007136116, WO2007143316, WO2007147478, WO2008001864, WO2008002824, WO2008013277, WO2008013280, WO2008013321, WO2008013322, WO2008016132, WO2008020011, JP2008031161, WO2008034859, WO2008042688, WO2008044762, WO2008046497, WO2008049923, WO2008055870, WO2008055940, WO2008069327, WO2008070609, WO2008071288, WO2008072726, WO2008083200, WO2008090209, WO2008090210, WO2008101586, WO2008101939, WO2008116179, WO2008116195, US2008242596, US2008287529, WO2009026537, WO2009049731, WO2009076550, WO2009084531, WO2009096503, WO2009100936, WO2009121939, WO2009124638, WO2009128421, WO2009135673, WO2010009197, WO2010018435, WO2010018438 oder von A. L. Handion in Expert Opin. Ther. Patents (2005) 15(11), 1531-1540 beschrieben sind, verabreicht.

Bei einer weiteren Ausführungsform der Erfindung wird die Verbindung der Formel I in Kombination mit einer festen Kombination eines SGLT-Inhibitors mit einem DPP-IV Inhibitor, wie in WO2009091082 beschrieben, verabreicht.

Bei einer Ausführungsform wird die Verbindung der Formel I in Kombination mit einem Stimulator des Glukosetransports, wie z.B. in WO2008136392, WO2008136393 beschrieben, verabreicht.

Bei einer Ausführungsform wird die Verbindung der Formel I in Kombination mit Hemmstoffen der 11-beta-Hydroxysteroid-Dehydrogenase-1 (11ß-HSD1), wie z. B. BVT-2733, JNJ-25918646, INCB-13739, INCB-20817, DIO-92 ((-)-Ketoconazol) oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005063247, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138508, WO2006138695, WO2006133926, WO2007003521, WO2007007688, US2007066584, WO2007029021, WO2007047625, WO2007051811, WO2007051810, WO2007057768, WO2007058346, WO2007061661, WO2007068330, WO2007070506, WO2007087150, WO2007092435, WO2007089683, WO2007101270, WO2007105753, WO2007107470, WO2007107550, WO2007111921, US2007207985, US2007208001, WO2007115935, WO2007118185, WO2007122411, WO2007124329, WO2007124337, WO2007124254, WO2007127688, WO2007127693, WO2007127704, WO2007127726, WO2007127763, WO2007127765, WO2007127901, US2007270424, JP2007291075, WO2007130898, WO2007135427, WO2007139992, WO2007144394, WO2007145834. WO2007145835, WO2007146761, WO2008000950, WO2008000951, WO2008003611, WO2008005910, WO2008006702, WO2008006703, WO2008011453, WO2008012532, WO2008024497, WO2008024892, WO2008032164, WO2008034032, WO2008043544, WO2008044656, WO2008046758, WO2008052638, WO2008053194, WO2008071169, WO2008074384, WO2008076336, WO2008076862, WO2008078725, WO2008087654, WO2008088540, WO2008099145, WO2008101885, WO2008101886, WO2008101907, WO2008101914, WO2008106128, WO2008110196, WO2008119017, WO2008120655, WO2008127924, WO2008130951, WO2008134221, WO2008142859, WO2008142986, WO2008157752, WO2009001817, WO2009010416, WO2009017664, WO2009020140, WO2009023180, WO2009023181, WO2009023664, WO2009026422, WO2009038064, WO2009045753, WO2009056881, WO2009059666, WO2009061498, WO2009063061, WO2009070497, WO2009074789, WO2009075835, WO2009088997, WO2009090239, WO2009094169, WO2009098501, WO2009100872, WO2009102428, WO2009102460, WO2009102761, WO2009106817, WO2009108332, WO2009112691, WO2009112845, WO2009114173, WO2009117109, US2009264401, WO2009118473, WO2009131669, WO2009132986, WO2009134384, WO2009134387, WO2009134392, WO2009134400, WO2009135581, WO2009138386, WO2010006940, WO2010010157, WO2010010174, WO2010011917 beschrieben sind, verabreicht.

Bei einer Ausführungsform wird die Verbindung der Formel I in Kombination mit Inhibitoren der Protein-Tyrosin-Phosphatase-1B (PTP-1B), wie sie z. B. in WO200119830-31, WO200117516, WO2004506446, WO2005012295, WO2005116003, WO2005116003, WO2006007959, DE 10 2004 060542.4, WO2007009911, WO2007028145, WO2007067612-615, WO2007081755, WO2007115058, US2008004325, WO2008033455, WO2008033931, WO2008033932, WO2008033934, WO2008089581, WO2008148744, WO2009032321, WO2009109999, WO2009109998 beschrieben sind, verabreicht.

Bei einer weiteren Ausführungsform wird die Verbindung der Formel I in Kombination mit Stimulatoren der Tyrosin-Kinase-B (Trk-B), wie sie z. B. in WO2010014613 beschrieben sind, verabreicht.

Bei einer weiteren Ausführungsform wird die Verbindung der Formel I in Kombination mit Beta 3-Agonisten (auch Beta-3 Adrenoceptor Agonisten genannt), wie z.B. in Physiol. Behav. 2004 Sep 15;82(2-3):489-96, J Clin Invest (1998) 101: 2387-93, Curr. Pharma. Des.2001 Sep;7(14):1433-49., Bioorganic & Medicinal Chemistry Letters Band 14, Ausgabe 13, 5 Juli 2004, Seiten 3525-3529 (BMS-201620) beschrieben sind, verabreicht.

Bei einer Ausführungsform der Erfindung wird die Verbindung der Formel I in Kombination mit einem Agonisten des GPR109A (HM74A Rezeptor Agonisten; NAR-Agonisten (Nikotinsäurerezeptoragonisten)), wie z.B. Nicotinsäure oder ,,extended release niacin" in Verbindung mit MK-0524A (Laropiprant) oder MK-0524 oder solchen Verbindungen, wie sie in WO2004041274, WO2006045565, WO2006045564, WO2006069242, WO2006085108, WO2006085112, WO2006085113, WO2006124490, WO2006113150, WO2007002557, WO2007017261, WO2007017262, WO2007017265, WO2007015744, WO2007027532, WO2007092364, WO2007120575, WO2007134986, WO2007150025, WO2007150026, WO2008016968, WO2008051403, WO2008086949, WO2008091338, WO2008097535, WO2008099448, US2008234277, WO2008127591 beschrieben sind, verabreicht.

Bei einer anderen Ausführungsform der Erfindung wird die Verbindung der Formel I in Kombination mit einer festen Kombination von Niacin mit Simvastatin verabreicht.

Bei einer anderen Ausführungsform der Erfindung wird die Verbindung der Formel I in Kombination mit Nicotinsäure oder "extended release niacin" in Verbindung mit MK-0524A (Laropiprant) verabreicht.

Bei einer weiteren Ausführungsform der Erfindung wird die Verbindung der Formel I in Kombination mit Nicotinsäure oder "extended release niacin" in Verbindung mit MK-0524A (Laropiprant) und mit Simvastatin verabreicht.

Bei einer Ausführungsform der Erfindung wird die Verbindung der Formel I in Kombination mit Nicotinsäure oder einem anderen Nicotinsäurerezeptoragonisten und einem Prostaglandin DP Rezeptorantagonisten, wie z.B. solchen wie sie in WO2008039882 beschrieben sind, verabreicht.

Bei einer anderen Ausführungsform der Erfindung wird die Verbindung der Formel I in Kombination mit einer festen Kombination von Niacin mit Meloxicam, wie z.B. in WO2009149056 beschrieben, verabreicht.

Bei einer anderen Ausführungsform der Erfindung wird die Verbindung der Formel I in Kombination mit einem Agonisten des GPR116, wie sie z.B. in WO2006067531, WO2006067532 beschrieben sind, verabreicht.

Bei einer Ausführungsform wird die Verbindung der Formel I in Kombination mit Modulatoren des GPR40, wie sie z.B. in WO2007013689, WO2007033002, WO2007106469, US2007265332, WO2007123225, WO2007131619, WO2007131620, WO2007131621, US2007265332, WO2007131622, WO2007136572, WO2008001931, WO2008030520, WO2008030618, WO2008054674, WO2008054675, WO2008066097, US2008176912, WO2008130514, WO2009038204, WO2009039942, WO2009039943, WO2009048527, WO2009054479, WO2009058237, WO2009111056, WO2010012650 beschrieben sind, verabreicht.

Bei einer Ausführungsform wird die Verbindung der Formel I in Kombination mit Modulatoren des GPR119 (G-Protein-gekoppelter Glukose-abhängiger insulinotroper Rezeptor), wie z.B. PSN-119-1, PSN-821, PSN-119-2, MBX-2982 oder solchen wie sie z. B. in WO2004065380, WO2005061489 (PSN-632408), WO2006083491, WO2007003960-62 und WO2007003964, WO2007035355, WO2007116229, WO2007116230, WO2008005569, WO2008005576, WO2008008887, WO2008008895, WO2008025798, WO2008025799, WO2008025800, WO2008070692, WO2008076243, WO200807692, WO2008081204, WO2008081205, WO2008081206, WO2008081207, WO2008081208, WO2008083238, WO2008085316, WO2008109702, WO2008130581, WO2008130584, WO2008130615, WO2008137435, WO2008137436, WO2009012275, WO2009012277, WO2009014910, WO2009034388, WO2009038974, WO2009050522, WO2009050523, WO2009055331, WO2009105715, WO2009105717, WO2009105722, WO2009106561, WO2009106565, WO2009117421, WO2009125434, WO2009126535, WO2009129036, US2009286812, WO2009143049, WO2009150144, WO2010001166, WO2010004343, WO2010004344, WO2010004345, WO2010004346, WO2010004347, WO2010004348, WO2010008739, WO2010006191, WO2010009183, WO2010009195, WO2010009207, WO2010009208, WO2010014593 beschrieben sind, verabreicht.

Bei einer weiteren Ausführungsform wird die Verbindung der Formel I in Kombination mit Modulatoren des GPR120, wie sie z.B. in EP1688138, WO2008066131, WO2008066131, WO2008103500, WO2008103501, WO2008139879, WO2009038204, WO2009147990, WO2010008831 beschrieben sind, verabreicht.

Bei einer anderen Ausführungsform wird die Verbindung der Formel I in Kombination mit Antagonisten des GPR105, wie sie z.B. in WO2009000087, WO2009070873 beschrieben sind, verabreicht.

Bei einer weiteren Ausführungsform wird die Verbindung der Formel I in Kombination mit Agonisten des GPR43, wie z.B. ESN-282 verabreicht.

Bei einer Ausführungsform wird die Verbindung der Formel I in Kombination mit Inhibitoren der hormon-sensitiven Lipase (HSL) und/oder Phospholipasen, wie z. B. in WO2005073199, WO2006074957, WO2006087309, WO2006111321, WO2007042178, WO2007119837, WO2008122352, WO2008122357, WO2009009287 beschrieben, verabreicht.

Bei einer Ausführungsform wird die Verbindung der Formel I in Kombination mit Inhibitoren der endothelialen Lipase, wie z. B. in WO2007110216 beschrieben, verabreicht.

Bei einer Ausführungsform wird die Verbindung der Formel I in Kombination mit einem Phospholipase A2 Inhibitor wie z.B. Darapladib oder A-002 oder solchen, wie sie in WO2008048866, WO20080488867, US2009062369 beschrieben sind, verabreicht.

Bei einer Ausführungsform wird die Verbindung der Formel I in Kombination mit Myricitrin, einem Lipase-Inhibitor (WO2007119827), verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117, WO2007073117, WO2007083978, WO2007120102, WO2007122634, WO2007125109, WO2007125110, US2007281949, WO2008002244, WO2008002245, WO2008016123, WO2008023239, WO2008044700, WO2008056266, WO2008057940, WO2008077138, EP1939191, EP1939192, WO2008078196, WO2008094992, WO2008112642, WO2008112651, WO2008113469, WO2008121063, WO2008121064, EP-1992620, EP-1992621, EP1992624, EP-1992625, WO2008130312, WO2009007029, EP2020232, WO2009017452, WO2009035634, WO2009035684, WO2009038385, WO2009095787, WO2009095788, WO2009095789, WO2009095792, WO2009145814, US2009291982, WO2009154697, WO2009156857, WO2009156859, WO2009156860, WO2009156861, WO2009156863, WO2009156864, WO2009156865, WO2010013168, WO2010014794 beschrieben.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Phosphoinositidkinase-3 (PI3K), wie z.B. solchen, wie in WO2008027584, WO2008070150, WO2008125833, WO2008125835, WO2008125839, WO2009010530, WO2009026345, WO2009071888, WO2009071890, WO2009071895 beschrieben, verabreicht.

Bei einer Ausführungsform wird die Verbindung der Formel I in Kombination mit einem Inhibitor der Serum/Glucocorticoid regulierten Kinase (SGK), wie z. B. in WO2006072354, WO2007093264, WO2008009335, WO2008086854, WO2008138448 beschrieben, verabreicht.

Bei einer Ausführungsform wird die Verbindung der Formel I in Kombination mit einem Modulator des Glucocorticoidrezeptors, wie z. B. in WO2008057855, WO2008057856, WO2008057857, WO2008057859, WO2008057862, WO2008059867, WO2008059866, WO2008059865, WO2008070507, WO2008124665, WO2008124745, WO2008146871, WO2009015067, WO2009040288, WO2009069736, WO2009149139 beschrieben, verabreicht.

Bei einer Ausführungsform wird die Verbindung der Formel I in Kombination mit einem Modulator des Mineralocorticoidrezeptors (MR), wie z. B. Drospirenone, oder solchen wie sie in WO2008104306, WO2008119918 beschrieben sind, verabreicht.

Bei einer Ausführungsform wird die Verbindung der Formel I in Kombination mit einem Inhibitor der Protein Kinase C beta (PKC beta), wie z. B. Ruboxistaurin, oder solchen wie sie in WO2008096260, WO2008125945 beschrieben sind, verabreicht.

Bei einer Ausführungsform wird die Verbindung der Formel I in Kombination mit einem Inhibitor der Protein Kinase D, wie z. B. Doxazosin (WO2008088006), verabreicht.

Bei einer weiteren Ausführungsform wird die Verbindung der Formel I in Kombination mit einem Aktivator/Modulator der AMP-aktivierten Proteinkinase (AMPK), wie sie z. B. in WO2007062568, WO2008006432, WO2008016278, WO2008016730, WO2008020607, WO2008083124, WO2008136642, WO2009019445, WO2009019446, WO2009019600, WO2009028891, WO2009065131, WO2009076631, WO2009079921, WO2009100130, WO2009124636, WO2009135580, WO2009152909 beschrieben sind, verabreicht.

Bei einer Ausführungsform wird die Verbindung der Formel I in Kombination mit einem Inhibitor der Ceramidkinase, wie sie z. B. in WO2007112914, WO2007149865 beschrieben sind, verabreicht.

Bei einer weiteren Ausführungsform wird die Verbindung der Formel I in Kombination mit einem Inhibitor der MAPK-interagierenden Kinase 1 oder 2 (MNK1 oder 2), wie sie z.B. in WO2007104053, WO2007115822, WO2008008547, WO2008075741 beschrieben sind, verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022057, WO2004022553, WO2005097129, WO2005113544, US2007244140, WO2008099072, WO2008099073, WO2008099073, WO2008099074, WO2008099075, WO2009056693, WO2009075277, WO2009089042, WO2009120801 beschrieben sind, verabreicht.

Bei einer anderen Ausführungsform wird die Verbindung der Formel I in Kombination mit Inhibitoren der NF-kappaB (NFKB) Aktivierung, wie sie z. B. Salsalate verabreicht.

Bei einer weiteren Ausführungsform wird die Verbindung der Formel I in Kombination mit Inhibitoren der ASK-1 (apoptosis signal-regulating kinase 1), wie sie z. B. in WO2008016131, WO2009123986 beschrieben sind, verabreicht.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, Pitavastatin, L-659699, BMS-644950, NCX-6560 oder solchen, wie sie in US2007249583, WO2008083551, WO2009054682 beschrieben sind, verabreicht.

Bei einer weiteren Ausführungsform der Erfindung wird die Verbindung der Formel I in Kombination mit einem Farnesoid X Rezeptor (FXR) Modulatoren, wie z.B. WAY-362450 oder solchen wie in WO2003099821, WO2005056554, WO2007052843, WO2007070796, WO2007092751, JP2007230909, WO2007095174, WO2007140174, WO2007140183, WO2008000643, WO2008002573, WO2008025539, WO2008025540, JP2008214222, JP2008273847, WO2008157270, US2008299118, US2008300235, WO2009005998, WO2009012125, WO2009027264, WO2009062874, US2009131409, US2009137554, US2009163552, WO2009127321, EP2128158 beschrieben, verabreicht.

Bei einer anderen Ausführungsform der Erfindung wird die Verbindung der Formel I in Kombination mit einem Liganden des Leber X Rezeptors (liver X receptor; LXR), wie z.B. in WO2007092965, WO2008041003, WO2008049047, WO2008065754, WO2008073825, US2008242677, WO2009020683, US2009030082, WO2009021868, US2009069373, WO2009024550, WO2009040289, WO2009086123, WO2009086129, WO2009086130, WO2009086138, WO2009107387, US2009247587, WO2009133692, WO2008138438, WO2009144961, WO2009150109 beschrieben, verabreicht.

Bei einer Ausführungsform der Erfindung wird die Verbindung der Formel I in Kombination mit einem Fibrat, wie z.B. Fenofibrat, Clofibrat, Bezafibrat, oder solchen wie sie in WO2008093655 beschrieben sind, verabreicht.

Bei einer Ausführungsform der Erfindung wird die Verbindung der Formel I in Kombination mit Fibraten, wie z.B. dem Cholinsalz von Fenofibrat (SLV-348; Trilipix^{™}), verabreicht.

Bei einer Ausführungsform der Erfindung wird die Verbindung der Formel I in Kombination mit Fibraten, wie z.B. dem Cholinsalz von Fenofibrat (Trilipix^{™}) und einem HMGCoA Reduktase Inhibitor, wie z.B. Rosuvastatin, verabreicht.

Bei einer weiteren Ausführungsform der Erfindung wird die Verbindung der Formel I in Kombination mit Bezafibrat und Diflunisal verabreicht.

Bei einer weiteren Ausführungsform der Erfindung wird die Verbindung der Formel I in Kombination mit einer festen Kombination von Fenofibrat oder einem Salz davon mit Simvastatin, Rosuvastatin, Fluvastatin, Lovastatin, Cerivastatin, Pravastatin, Pitavastatin oder Atorvastatin verabreicht.

Bei einer weiteren Ausführungsform der Erfindung wird die Verbindung der Formel I in Kombination mit Synordia (R), einer festen Kombination von Fenofibrat mit Metformin, verabreicht.

Bei einer anderen Ausführungsform der Erfindung wird die Verbindung der Formel I in Kombination mit einer festen Kombination von Metformin mit einem MTP-Inhibitor, wie in WO2009090210 beschrieben, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464, WO2005000353 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) oder wie in WO2002050060, WO2002050068, WO2004000803, WO2004000804, WO2004000805, WO2004087655, WO2004097655, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163, WO2007059871, US2007232688, WO2007126358, WO2008033431, WO2008033465, WO2008052658, WO2008057336, WO2008085300, WO2008104875, US2008280836, WO2008108486 beschrieben, verabreicht.

Bei einer Ausführungsform der Erfindung wird die Verbindung der Formel I in Kombination mit einem NPC1L1-Antagonisten, wie z.B. solchen, wie sie in WO2008033464, WO2008033465 beschrieben sind, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Atorvastatin, verabreicht.

Bei einer Ausführungsform der Erfindung wird die Verbindung der Formel I in Kombination mit einer festen Kombination von Ezetimibe mit Fenofibrat verabreicht.

Bei einer Ausführungsform der Erfindung ist der weitere Wirkstoff ein Diphenylazetidinonderivat, wie z.B. in US 6,992,067 oder US 7,205,290 beschrieben.

Bei einer weiteren Ausführungsform der Erfindung ist der weitere Wirkstoff ein Diphenylazetidinonderivat, wie z.B. in US 6,992,067 oder US 7,205,290 beschrieben, kombiniert mit einem Statin, wie z.B. Simvastatin, Fluvastatin, Pravastatin, Lovastatin, Cerivastatin, Atorvastatin, Pitavastatin oder Rosuvastatin.

Bei einer Ausführungsform der Erfindung wird die Verbindung der Formel I in Kombination mit einer festen Kombination von Lapaquistat, einem Squalensynthase-Inhibitor, mit Atorvastatin verabreicht.

Bei einer weiteren Ausführungsform der Erfindung wird die Verbindung der Formel I in Kombination mit einem Konjugat bestehend aus dem HMGCoA-Reduktaseinhibitor Atorvastatin mit dem Renininhibitor Aliskiren (WO2009090158) verabreicht.

Bei einer Ausführungsform der Erfindung wird die Verbindung der Formel I in Kombination mit einem CETP-Inhibitor, wie z.B. Torcetrapib, Anacetrapib oder JTT-705 (Dalcetrapib) oder solchen wie sie in WO2006002342, WO2006010422, WO2006012093, WO2006073973, WO2006072362, WO2007088996, WO2007088999, US2007185058, US2007185113, US2007185154, US2007185182, WO2006097169, WO2007041494, WO2007090752, WO2007107243, WO2007120621, US2007265252, US2007265304, WO2007128568, WO2007132906, WO2008006257, WO2008009435, WO2008018529, WO2008058961, WO2008058967, WO2008059513, WO2008070496, WO2008115442, WO2008111604, WO2008129951, WO2008141077, US2009118287, WO2009062371, WO2009071509 beschrieben sind, verabreicht.

Bei einer Ausführungsform der Erfindung wird die Verbindung der Formel I in Kombination mit Gallensäureresorptionsinhibitoren (Inhibitoren des intestinalen Gallensäuretransporters (IBAT)) (siehe z.B. US 6,245,744, US 6,221,897 oder WO00/61568), wie z.B. HMR 1741 oder solchen wie in DE 10 2005 033099.1 und DE 10 2005 033100.9, DE 10 2006 053635, DE 10 2006 053637, WO2007009655-56, WO2008058628, WO2008058629, WO2008058630, WO2008058631 beschrieben, verabreicht.

Bei einer Ausführungsform wird die Verbindung der Formel I in Kombination mit Agonisten des GPBAR1 (G-protein-coupled-bile-acid-receptor-1; TGR5), wie z.B. INT-777 oder solchen wie sie z.B. in US20060199795, WO2007110237, WO2007127505, WO2008009407, WO2008067219, WO2008067222, FR2908310, WO2008091540, WO2008097976, US2009054304, WO2009026241, WO2009146772, WO2010014739, WO2010014836 beschrieben sind, verabreicht.

Bei einer Ausführungsform wird die Verbindung der Formel I in Kombination mit Modulatoren der Histon-Deacetylase, wie z.B. Ursodeoxycholsäure wie in WO2009011420 beschrieben, verabreicht.

Bei einer Ausführungsform wird die Verbindung der Formel I in Kombination mit Inhibitoren/Modulatoren des TRPM5 Kanals (TRP-Cation-Channel-M5), wie sie z.B. in WO2008097504, WO2009038722 beschrieben sind, verabreicht.

Bei einer Ausführungsform wird die Verbindung der Formel I in Kombination mit Inhibitoren/Modulatoren des TRPA1 Kanals (TRP-Cation-Channel-A1), wie sie z.B. in US2009176883, WO2009089083, WO2009144548 beschrieben sind, verabreicht.

Bei einer Ausführungsform wird die Verbindung der Formel I in Kombination mit Inhibitoren/Modulatoren des TRPV3 Kanals (TRP-Cation-Channel-V3), wie sie z.B. in WO2009084034, WO2009130560 beschrieben sind, verabreicht.

Bei einer Ausführungsform der Erfindung wird die Verbindung der Formel I in Kombination mit einem polymeren Gallensäureadsorber, wie z.B. Cholestyramin, Colesevelam Hydrochlorid, verabreicht.

Bei einer Ausführungsform der Erfindung wird die Verbindung der Formel I in Kombination mit Colesevelam Hydrochlorid und Metformin oder einem Sulfonylharnstoff oder Insulin verabreicht.

Bei einer Ausführungsform der Erfindung wird die Verbindung der Formel I in Kombination mit Tocotrienol und Insulin oder einem Insulinderivat verabreicht.

Bei einer Ausführungsform der Erfindung wird die Verbindung der Formel I in Kombination mit einem Phytosterole enthaltenden Kaugummi (Reductol^{™}) verabreicht.

Bei einer Ausführungsform der Erfindung wird die Verbindung der Formel I in Kombination mit einem Inhibitor des mikrosomalen Triglycerid-Transfer-Proteins (MTTP-Inhibitor), wie z.B. Implitapide , BMS-201038, R-103757, AS-1552133, SLx-4090, AEGR-733, JTT-130 oder solchen wie in WO2005085226, WO2005121091, WO2006010423, WO2006113910, WO2007143164, WO2008049806, WO2008049808, WO2008090198, WO2008100423, WO2009014674 beschrieben, verabreicht.

Bei einer weiteren Ausführungsform der Erfindung wird die Verbindung der Formel I in Kombination mit einer Kombinbation eines Cholesterolabsorptionsinhibitors, wie z.B. Ezetimibe, und einem Inhibitor des Triglycerid-Transfer-Proteins (MTP-Inhibitor), wie z.B. Implitapide, wie in WO2008030382 oder in WO2008079398 beschrieben, verabreicht.

Bei einer Ausführungsform der Erfindung wird die Verbindung der Formel I in Kombination mit einem antihypertriglyceridämischen Wirkstoff, wie z.B. solchen wie sie in WO2008032980 beschrieben sind, verabreicht.

Bei einer anderen Ausführungsform der Erfindung wird die Verbindung der Formel I in Kombination mit einem Antagonisten des Somatostatin 5 Rezeptors (SST5 Rezeptor), wie z.B. solchen wie sie in WO2006094682 beschrieben sind, verabreicht.

Bei einer Ausführungsform der Erfindung wird die Verbindung der Formel I in Kombination mit einem ACAT-Inhibitor, wie z.B. Avasimibe, SMP-797 oder KY-382 oder solchen, wie sie in WO2008087029, WO2008087030, WO2008095189, WO2009030746, WO2009030747, WO2009030750, WO2009030752, WO2009070130, WO2009081957, WO2009081957 beschrieben sind, verabreicht.

Bei einer weiteren Ausführungsform der Erfindung wird die Verbindung der Formel I in Kombination mit einem Inhibitor der Leber-Carnitin Palmitoyltransferase-1 (L-CPT1), wie sie z.B. in WO2007063012, WO2007096251 (ST-3473), WO2008015081, US2008103182, WO2008074692, WO2008145596, WO2009019199, WO2009156479, WO2010008473 beschrieben sind, verabreicht.

Bei einer anderen Ausführungsform der Erfindung wird die Verbindung der Formel I in Kombination mit einem Inhibitor der Carnitin-O-Palmitoyltransferase-II (CPT2), wie sie z.B. in US2009270500, US2009270505, WO2009132978, WO2009132979 beschrieben sind, verabreicht.

Bei einer weiteren Ausführungsform der Erfindung wird die Verbindung der Formel I in Kombination mit einem Modulator der Serin-Palmitoyltransferase (SPT), wie sie z.B. in WO2008031032, WO2008046071, WO2008083280, WO2008084300 beschrieben sind, verabreicht.

Bei einer Ausführungsform der Erfindung wird die Verbindung der Formel I in Kombination mit einem Squalen Synthetase Inhibitor, wie z.B. BMS-188494, TAK-475 (Lapaquistat Acetat) oder wie in WO2005077907, JP2007022943, WO2008003424, WO2008132846, WO2008133288, WO2009136396 beschrieben, verabreicht.

Bei einer Ausführungsform der Erfindung wird die Verbindung der Formel I in Kombination mit ISIS-301012 (Mipomersen), einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit Apolipoprotein (ApoB) SNALP, einem therapeutischen Produkt, welches eine siRNA (gerichtet gegen das ApoB-Gen) enthält, verabreicht.

Bei einer Ausführungsform der Erfindung wird die Verbindung der Formel I in Kombination mit einem Stimulator des ApoA-1 Gens, wie er z.B. in WO2008092231 beschrieben ist, verabreicht.

Bei einer Ausführungsform der Erfindung wird die Verbindung der Formel I in Kombination mit einem Modulator der Synthese von Apolipoprotein C-III, wie .B. ISIS-APOCIIIRx, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738, WO2008020607 beschrieben, verabreicht.

Bei einer anderen Ausführungsform der Erfindung wird die Verbindung der Formel I in Kombination mit einem HDL-Cholesterol-erhöhenden Agens, wie z.B. solchen wie sie in WO2008040651, WO2008099278, WO2009071099, WO2009086096, US2009247550 beschrieben sind, verabreicht.

Bei einer Ausführungsform der Erfindung wird die Verbindung der Formel I in Kombination mit einem ABCA1 Expressionsverstäker, wie sie z.B. in WO2006072393, WO2008062830, WO2009100326 beschrieben, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit einem Adenosin A1 Rezeptor Agonisten (Adenosin A1 R), wie z.B. CVT-3619 oder solchen wie sie z.B. in EP1258247, EP1375508, WO2008028590, WO2008077050, WO2009050199, WO2009080197, WO2009100827, WO2009112155 beschrieben sind, verabreicht.

Bei einer Ausführungsform der Erfindung wird die Verbindung der Formel I in Kombination mit einem Adenosin A2B Rezeptor Agonisten (Adenosin A2B R) wie z.B. ATL-801 verabreicht.

Bei einer anderen Ausführungsform der Erfindung wird die Verbindung der Formel I in Kombination mit einem Modulator der Adenosin A2A und/oder Adenosin A3 Rezeptoren, wie z.B. in WO2007111954, WO2007121918, WO2007121921, WO2007121923, WO2008070661, WO2009010871 beschrieben, verabreicht.

Bei einer weiteren Ausführungsform der Erfindung wird die Verbindung der Formel I in Kombination mit einem Liganden der Adenosin A1/A2B Rezeptoren, wie z.B. in WO2008064788, WO2008064789, WO2009080198, WO2009100827, WO2009143992 beschrieben, verabreicht.

Bei einer Ausführungsform der Erfindung wird die Verbindung der Formel I in Kombination mit einem Adenosin A2B Rezeptor Antagonisten (Adenosin A2B R), wie sie in

US2007270433, WO2008027585, WO2008080461, WO2009037463, WO2009037467, WO2009037468, WO2009118759 beschrieben sind, verabreicht.

Bei einer Ausführungsform wird die Verbindung der Formel I in Kombination mit Hemmstoffen der Acetyl-CoA Carboxylase (ACC1 und/oder ACC2) wie z. B. solchen wie in WO199946262, WO200372197, WO2003072197, WO2005044814, WO2005108370, JP2006131559, WO2007011809, WO2007011811, WO2007013691, WO2007095601-603, WO2007119833, WO2008065508, WO2008069500, WO2008070609, WO2008072850, WO2008079610, WO2008088688, WO2008088689, WO2008088692, US2008171761, WO2008090944, JP2008179621, US2008200461, WO2008102749, WO2008103382, WO2008121592, WO2009082346, US2009253725, JP2009196966, WO2009144554, WO2009144555, WO2010003624, WO2010002010 beschrieben, verabreicht.

Bei einer anderen Ausführungsform wird die Verbindung der Formel I in Kombination mit Modulatoren der mikrosomalen Acyl-CoA:Glycerol-3-Phosphat-Acyltransferase 3 (GPAT3, beschrieben in WO2007100789) oder mit Modulatoren der mikrosomalen Acyl-CoA:Glycerol-3-Phosphat-Acyltransferase 4 (GPAT4, beschrieben in WO2007100833) oder mit Modulatoren der mitochondrialen Glycerol-3-Phosphat-O-Acyltransferase, beschrieben in WO2010005922, verabreicht.

Bei einer weiteren Ausführungsform wird die Verbindung der Formel I in Kombination mit Modulatoren der Xanthin-Oxidoreductase (XOR) verabreicht.

Bei einer anderen Ausführungsform wird die Verbindung der Formel I in Kombination mit Inhibitoren der löslichen Epoxidhydrolase (sEH), wie sie z.B. in WO2008051873, WO2008051875, WO2008073623, WO2008094869, WO2008112022, WO2009011872, WO2009049154, WO2009049157, WO2009049165, WO2009073772, WO2009097476, WO2009111207, WO2009129508, WO2009151800 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A) oder Velneperit oder solche wie sie in WO2009110510 beschrieben sind;
NPY-5 Rezeptorantagonisten/-rezeptormodulatoren wie L-152804 oder die Verbindung "NPY-5-BY" der Firma Banyu oder wie sie z. B. in WO2006001318, WO2007103295, WO2007125952, WO2008026563, WO2008026564, WO2008052769, WO2008092887, WO2008092888, WO2008092891, WO2008129007, WO2008134228, WO2009054434, WO2009095377, WO2009131096 beschrieben sind;
NPY-4-Rezeptorantagonisten wie sie z. B. in WO2007038942 beschrieben sind;
NPY-2-Rezeptorantagonisten/-modulatoren wie sie z. B. in WO2007038943, WO2009006185, US2009099199, US2009099243, US2009099244, WO2009079593, WO2009079597 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424, WO2006095166, WO2008003947, WO2009080608 beschrieben sind;
NPY-2-Rezeptoragonisten wie sie z.B. in WO2009080608 beschrieben sind;
Derivaten des Peptids Obestatin wie sie WO2006096847 beschrieben sind;
CB1R (Cannabinoid Rezeptor 1) Antagonisten/inverse Agonisten, wie z.B. Rimonabant, Surinabant (SR147778), SLV-319 (Ibipinabant), AVE-1625, Taranabant (MK-0364) oder Salze davon, Otenabant (CP-945,598), Rosonabant, V-24343 oder solche Verbindungen wie sie in z. B. EP 0656354, WO 00/15609, WO2001/64632-64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006018662, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007018460, WO2007016460, WO2007020502, WO2007026215, WO2007028849, WO2007031720, WO2007031721, WO2007036945, WO2007038045, WO2007039740, US20070015810, WO2007046548, WO2007047737, WO2007057687, WO2007062193, WO2007064272, WO2007079681, WO2007084319, WO2007084450, WO2007086080, EP1816125, US2007213302, WO2007095513, WO2007096764, US2007254863, WO2007119001, WO2007120454, WO2007121687, WO2007123949, US2007259934, WO2007131219, WO2007133820, WO2007136571, WO2007136607, WO2007136571, US7297710, WO2007138050, WO2007139464, WO2007140385, WO2007140439, WO2007146761, WO2007148061, WO2007148062, US2007293509, WO2008004698, WO2008017381, US2008021031, WO2008024284, WO2008031734, WO2008032164, WO2008034032, WO2008035356, WO2008036021, WO2008036022, WO2008039023, WO2998043544, WO2008044111, WO2008048648, EP1921072-A1, WO2008053341, WO2008056377, WO2008059207, WO2008059335, WO2008062424, WO2008068423, WO2008068424, WO2008070305, WO2008070306, WO2008074816, WO2008074982, WO2008075012, WO2008075013, WO2008075019, WO2008075118, WO2008076754, WO2008081009, WO2008084057, EP1944295, US2008090809, US2008090810, WO2008092816, WO2008094473, WO2008094476, WO2008099076, WO2008099139, WO2008101995, US2008207704, WO2008107179, WO2008109027, WO2008112674, WO2008115705, WO2008118414, WO2008119999, WO200812000, WO2008121257, WO2008127585, WO2008129157, WO2008130616, WO2008134300, US2008262066, US2008287505, WO2009005645, WO2009005646, WO2009005671, WO2009023292, WO2009023653, WO2009024819, WO2009033125, EP2042175, WO2009053548-WO2009053553, WO2009054923, WO2009054929, WO2009059264, WO2009073138, WO2009074782, WO2009075691, WO2009078498, WO2009087285, WO2009074782, WO2009097590, WO2009097995, WO2009097996, WO2009097998, WO2009097999, WO2009098000, WO2009106708, US2009239909, WO2009118473, US2009264436, US2009264476, WO2009130234, WO2009131814, WO2009131815, US2009286758, WO2009141532, WO2009141533, WO2009153569, WO2010003760, WO2010012437, WO2010019762 beschrieben sind;
Cannabinoid Rezeptor 1 / Cannabinoid Rezeptor 2 (CB1,/CB2) modulierende Verbindungen wie z.B. delta-9-Tetrahydrocannabivarin oder solchen wie sie z.B. in WO2007001939, WO2007044215, WO2007047737, WO2007095513, WO2007096764, WO2007112399, WO2007112402, WO2008122618, WO2009007697, WO2009012227, WO2009087564, WO2009093018, WO2009095752, WO2009120660, WO2010012964 beschrieben sind;
Cannabinoid Rezeptor 2 (CB2) modulierende Verbindungen wie z.B. solchen wie sie z.B. in WO2008063625, WO2008157500, WO2009004171, WO2009032754, WO2009055357, WO2009061652, WO2009063495, WO2009067613, WO2009114566 beschrieben sind;
Modulatoren der FAAH (fatty acid amide hydrolase) wie sie z.B. in WO2007140005, WO2008019357, WO2008021625, WO2008023720, WO2008030532, WO2008129129, WO2008145839, WO2008145843, WO2008147553, WO2008153752, WO2009011904, WO2009048101, WO2009084970, WO2009105220, WO2009109504, WO2009109743, WO2009117444, WO2009127944, WO2009138416, WO2009151991, WO2009152025, WO2009154785, WO2010005572, WO2010017079 beschrieben sind;
Inhibitoren der Fettsäuresynthase (fatty acid synthase; FAS), wie sie z.B. in WO2008057585, WO2008059214, WO2008075064, WO2008075070, WO2008075077, WO2009079860 beschrieben sind;
Inhibitoren der LCE (long chain fatty acid elongase)/Long-Chain-Fatty-Acid-CoA-Ligase, wie sie z.B. in WO2008120653, WO2009038021, WO2009044788, WO2009081789, WO2009099086 beschrieben sind;
Vanilloid-1-Rezeptor Modulatoren (Modulatoren des TRPV1), wie sie z.B. in WO2007091948, WO2007129188, WO2007133637, WO2008007780, WO2008010061, WO2008007211, WO2008010061, WO2008015335, WO2008018827, WO2008024433, WO2008024438, WO2008032204, WO2008050199, WO2008059339, WO2008059370, WO2008066664, WO2008075150, WO2008090382, WO2008090434, WO2008093024, WO2008107543, WO2008107544, WO2008110863, WO2008125295, WO2008125296, WO2008125337,, WO2008125342, WO2008132600, WO2008133973, WO2009010529, WO2009010824, WO2009016241, WO2009023539, WO2009038812, WO2009050348, WO2009055629, WO2009055749, WO2009064449, WO2009081222, WO2009089057, WO2009109710 WO2009112677, WO2009112678, WO2009112679, WO2009121036, WO2009124551, WO2009136625, WO2010002209 beschrieben sind;
Modulatoren, Liganden, Antagonisten oder inverse Agonisten der Opioidrezeptoren, wie z.B. GSK-982 oder solche wie sie z.B. in WO2007047397, WO2008021849, WO2008021851, WO2008032156, WO2008059335, WO2008125348, WO2008125349, WO2008142454, WO2009030962, WO2009103552, WO2009115257 beschrieben sind;
Modulatoren des "orphan opioid (ORL-1) receptor" wie sie z.B. in US2008249122, WO2008089201 beschrieben sind;
Agonisten des Prostaglandinrezeptors, wie z.B. Bimatoprost oder solchen Verbindungen wie sie in WO2007111806 beschrieben sind;
MC4-Rezeptor Agonisten (Melanocortin-4 Rezeptor Agonisten, MC4R Agonisten wie z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141, MK-0493 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2004089307, WO2005042516, WO2005040109, WO2005030797, US20040224901, WO200501921, WO200509184, WO2005000339, EP1460069, WO2005047253, WO2005047251, WO2005118573, EP1538159, WO2004072076, WO2004072077, WO2006021655-57, WO2007009894, WO2007015162, WO2007041061, WO2007041052, JP2007131570, EP-1842846, WO2007096186, WO2007096763, WO2007141343, WO2008007930, WO2008017852, WO2008039418, WO2008087186, WO2008087187, WO2008087189, WO2008087186-WO2008087190, WO2008090357, WO2008142319, WO2009015867, WO2009061411, US2009076029, US2009131465, WO2009071101, US2009305960, WO2009144432, WO2009151383, WO2010015972 beschrieben sind;
MC4-Rezeptor Modulatoren (Melanocortin-4 Rezeptor Modulatoren) wie sie z.B. in WO2009010299, WO2009074157 beschrieben sind;
Orexin-Rezeptor 1 Antagonisten (OX1R Antagonisten), Orexin-Rezeptor 2 Antagonisten (OX2R Antagonisten) oder gemischte OX1R/OX2R Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224, WO2007085718, WO2007088276, WO2007116374, WO2007122591, WO2007126934, WO2007126935, WO2008008517, WO2008008518, WO2008008551, WO2008020405, WO2008026149, WO2008038251, US2008132490, WO2008065626, WO2008078291, WO2008087611, WO2008081399, WO2008108991, WO2008107335, US2008249125, WO2008147518, WO2008150364, WO2009003993, WO2009003997, WO2009011775, WO2009016087, WO2009020642, WO2009058238, US2009186920, US2009203736, WO2009092642, WO2009100994, WO2009104155, WO2009124956, WO2009133522, WO2009156951, WO2010017260 beschrieben sind);
Histamin H3 Rezeptor Antagonisten/inverse Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893, WO2005123716, US2005171181 (z.B. PF-00389027), WO2006107661, WO2007003804, WO2007016496, WO2007020213, WO2007049798, WO2007055418, WO2007057329, WO2007062999, WO2007065820, WO2007068620, WO2007068641, WO2007075629, WO2007080140, WO2007082840, WO2007088450, WO2007088462, WO2007094962, WO2007099423, WO2007100990, WO2007105053, WO2007106349, WO2007110364, WO2007115938, WO2007131907, WO2007133561, US2007270440, WO2007135111, WO2007137955, US2007281923, WO2007137968, WO2007138431, WO2007146122, WO2008005338, WO2008012010, WO2008015125, WO2008045371, EP1757594, WO2008068173, WO2008068174, US20080171753, WO2008072703, WO2008072724, US2008188484, US2008188486, US2008188487, WO2008109333, WO2008109336, WO2008126886, WO2008154126, WO2008151957, US2008318952, WO2009003003, WO2009013195, WO2009036132, WO2009039431, WO2009045313, WO2009058300, WO2009063953, WO2009067401, WO2009067405, WO2009067406, US2009163464, WO2009100120, WO2009105206, WO2009121812, WO2009126782, WO2010011653, WO2010011657 beschrieben sind);
Histamin H1 / Histamin H3 Modulatoren, wie z. B. Betahistin bzw. seinem Dihydrochlorid;
Modulatoren des Histamin H3 Transporters oder der Histamin H3 / Serotonin Transporter wie sie z.B. in WO2008002816, WO2008002817, WO2008002818, WO2008002820 beschrieben sind;
Modulatoren des vesikulären Monoamintransporters 2 (vesicular monoamine transporter 2 (VMAT2)) wie sie z.B. in WO2009126305 beschrieben sind;
Histamin H4 Modulatoren wie sie z.B. in WO2007117399, US2009156613 beschrieben sind;
CRF-Antagonisten (z.B. [2-Methyl-9-(2,4,6-trimethyl-phenyl)-9H-1,3,9-triaza-fluoren-4-yl]-dipropyl-amin (WO 00/66585) oder solche CRF1-Antagonisten, wie sie in WO2007105113, WO2007133756, WO2008036541, WO2008036579, WO2008083070, WO2010015628, WO2010015655 beschrieben sind);
CRF BP-Antagonisten (z.B. Urocortin);
Urocortin-Agonisten;
Modulatoren des beta-3 Adrenoceptors wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553, WO2002038543, WO2002038544, WO2007048840-843, WO2008015558, EP1947103, WO2008132162 beschrieben sind;
MSH (Melanocyt-stimulicrendes Hormon)-Agonisten;
MCH (melanin-konzentrierendes Hormon) Rezeptor Antagonisten (wie z. B. NBI-845, A-761, A-665798, A-798, ATC-0175, T-226296, T-71 (AMG-071, AMG-076), GW-856464, NGD-4715, ATC-0453, ATC-0759, GW-803430 oder solche Verbindungen, wie sie in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649, WO2007092416; WO2007093363-366, WO2007114902, WO2007114916, WO2007141200, WO2007142217, US2007299062, WO2007146758, WO2007146759, WO2008001160, WO2008016811, WO2008020799, WO2008022979, WO2008038692, WO2008041090, WO2008044632, WO2008047544, WO2008061109, WO2008065021, WO2008068265, WO2008071646, WO2008076562, JP2008088120, WO2008086404, WO2008086409, US2008269110, WO2008140239, WO2009021740, US2009011994, US2009082359, WO2009041567, WO2009076387, WO2009089482, WO2009103478, WO2009119726, WO2009120655, WO2009123194, WO2009137270, WO2009146365, WO2009154132 beschrieben sind);
CCK-A (CCK-1) Modulatoren (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl} -essigsäure Trifluoressigsäuresalz (WO 99/15525) oder SR-146131 (WO 0244150) oder SSR-125180) oder solchen, wie sie in WO2005116034, WO2007120655, WO2007120688, WO2007120718, WO2008091631 beschrieben sind;
Serotonin-Wiederaufnahme-Inhibitoren (z.B. Dexfenfluramine) oder solchen wie sie in WO2007148341, WO2008034142, WO2008081477, WO2008120761, WO2008141081, WO2008141082, WO2008145135, WO2008150848, WO2009043834, WO2009077858 beschrieben sind;
gemischte Serotonin-/Dopamin-Wiederaufnahme-Inhibitoren (z.B. Bupropion) oder solche wie sie in WO2008063673 beschrieben sind oder feste Kombinationen von Bupropion mit Naltrexon oder Bupropion mit Zonisamid;
gemischte Wiederaufnahmeinhibitoren wie z.B. DOV-21947 oder solche wie sie in WO2009016214, WO2009016215, WO2009077584, WO2009098208, WO2009098209, WO2009106769, WO2009109517, WO2009109518, WO2009109519, WO2009109608, WO2009145357, WO2009149258 beschrieben sind;
gemischte Serotonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
gemischte Dopamin/Norepinephrin/Acetylcholin-Wiederaufnahme-Inhibitoren (z.B. Tesofensine) oder solchen wie sie z.B. in WO2006085118, WO2008150480 beschrieben sind;
Dopaminantagonisten wie sie z.B. in WO2008079838, WO2008079839, WO2008079847, WO2008079848 beschrieben sind;
Norepinephrin-Wiederaufnahme-Inhibitoren wie sie z.B. in US2008076724, WO2009062318 beschrieben sind;
5-HT1A Rezeptor Modulatoren wie sie z.B. in WO2009006227, WO2009137679, WO2009137732 beschrieben sind;
5-HT2A Rezeptor Antagonisten wie sie z.B. in WO2007138343 beschrieben sind;
5-HT2C Rezeptor Agonisten (wie z.B. Lorcaserin Hydrochlorid (APD-356) oder BVT-933 oder solche, wie sie in WO200077010, WO200077001-02, WO2005019180, WO2003064423, WO200242304, WO2005035533, WO2005082859, WO2006004937, US2006025601, WO2006028961, WO2006077025, WO2006103511, WO2007028132, WO2007084622, US2007249709; WO2007132841, WO2007140213, WO2008007661, WO2008007664, WO2008009125, WO2008010073, WO2008108445, WO2009063991, WO2009063992, WO2009063993, WO2009079765 beschrieben sind);
5-HT6 Rezeptor Modulatoren, wie z.B. E-6837, BVT-74316, PF-3246799 oder PRX-07034 oder solche wie sie z.B. in WO2005058858, WO2007054257, WO2007107373, WO2007108569, WO2007108742-744, WO2008003703, WO2008027073, WO2008034815, WO2008054288, EP1947085, WO2008084491, WO2008084492, WO2008092665, WO2008092666, WO2008101247, WO2008110598, WO2008116831, WO2008116833, WO2008117169, WO2008136017, WO2008147812, EP2036888, WO2009013010, WO2009034581, WO2009053997, WO2009056632, WO2009073118, WO2009115515, WO2009135925, WO2009135927, WO2010000456, WO2010012806, EP2145887 beschrieben sind;
Agonisten des Estrogenrezeptors gamma (ERR Agonisten), wie sie z.B. in WO2007131005, WO2008052709 beschrieben sind;
Agonisten des Estrogenrezeptors alpha (ERR / ERR1 Agonisten), wie sie z.B. in WO2008109727 beschrieben sind;
Agonisten des Estrogenrezeptors beta (ERRß Agonisten), wie sie z.B. in WO2009055734, WO2009100335, WO2009127686 beschrieben sind;
Sigma-1 Rezeptorantagonisten, wie sie z.B. in WO2007098953, WO2007098961, WO2008015266, WO2008055932, WO2008055933, WO2009071657 beschrieben sind;
Muscarin 3 Rezeptor (M3R) Antagonisten, wie sie z.B. in WO2007110782, WO2008041184 beschrieben sind;
Bombesin-Rezeptor Agonisten (BRS-3 Agonisten), wie sie z.B. in WO2008051404, WO2008051405, WO2008051406, WO2008073311 beschrieben sind;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO01/85695));
Growth Hormone Secretagogue Receptor Antagonisten (Ghrelin Antagonisten) wie z. B. A-778193 oder solchen, wie sie in WO2005030734, WO2007127457, WO2008008286, WO2009056707 beschrieben sind;
Growth Hormone Secretagogue Receptor Modulatoren (Ghrelin-Modulatoren) wie z.B. JMV-2959, JMV-3002, JMV-2810, JMV-2951 oder solchen, wie sie in WO2006012577 (z.B. YIL-781 oder YIL-870), WO2007079239, WO2008092681, WO2008145749, WO2008148853, WO2008148854, WO2008148856, WO2009047558, WO2009071283, WO2009115503 beschrieben sind;
TRH-Agonisten (siehe z.B. EP 0 462 884);
entkoppelnde Protein 2- oder 3-Modulatoren (wie z.B. in WO2009128583 beschrieben);
chemische Entkoppler (z.B. WO2008059023, WO2008059024, WO2008059025, WO2008059026);
Leptinrezeptoragonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
Leptinrezeptormodulatoren wie sie z.B. in WO2009019427, WO2009071658, WO2009071668, WO2009071677, WO2009071678, WO2009147211, WO2009147216, WO2009147219, WO2009147221beschrieben sind;
DA-Agonisten (Bromocriptin, Bromocriptin Mesylat, Doprexin) oder solche wie sie in US2009143390 beschrieben sind;
Lipase/Amylase-Inhibitoren (z.B. WO00/40569, WO2008107184, WO2009049428, WO2009125819);
Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189, WO2006082952, WO2006120125, WO2006113919, WO2006134317, WO2007016538, WO2007060140, JP2007131584, WO2007071966, WO2007126957, WO2007137103, WO2007137107, WO2007138304, WO2007138311, WO2007141502, WO2007141517, WO2007141538, WO2007141545, WO2007144571, WO2008011130, WO2008011131, WO2008039007, WO2008048991, WO2008067257, WO2008099221, WO2008129319, WO2008141976, WO2008148840, WO2008148849, WO2008148851, WO2008148868, WO2009011285, WO2009016462, WO2009024821, US2009076275, WO2009040410, WO2009071483, WO2009081195, WO2009119534, WO2009126624, WO2009126861, WO2010007046, WO2010017040 beschrieben;
Inhibitoren der Monoacylglycerolacyltransferase (2-Acylglycerol-O-Acyltransferase; MGAT) wie sie z.B. in WO2008038768 beschrieben sind;
Inhibitoren der Fettsäuresynthase (FAS) wie z.B. C75 oder solchen, wie in WO2004005277, WO2008006113 beschrieben;
Inhibitoren der Stearoyl-CoA delta9 Desaturase (SCD1) wie sie z.B. in WO2007009236, WO2007044085, WO2007046867, WO2007046868, WO20070501124, WO2007056846, WO2007071023, WO2007130075, WO2007134457, WO2007136746, WO2007143597, WO2007143823, WO2007143824, WO2008003753, WO2008017161, WO2008024390, WO2008029266, WO2008036715, WO2008043087, WO2008044767, WO2008046226, WO2008056687, WO2008062276, WO2008064474, WO2008074824, WO2008074832, WO2008074833, WO2008074834, WO2008074835, WO2008089580, WO2008096746, WO2008104524, WO2008116898, US2008249100, WO2008120744, WO2008120759, WO2008123469, WO2008127349, WO2008128335, WO2008135141, WO2008139845, WO2008141455, US20080255130, US2008255161, WO2008141455, WO2009010560, WO2009016216, WO2009012573, WO2009024287, JP2009019013, WO2009037542, WO2009056556, WO2009060053, WO2009060054, WO2009070533, WO2009073973, WO2009103739, WO2009117659, WO2009117676, US2009253693, US2009253738, WO2009124259, WO2009126123, WO2009126527, WO2009129625, WO2009137201, WO2009150196, WO2009156484, WO2010006962, WO2010007482 beschrieben sind;
Inhibitoren der Fatty-Acid-Desaturase-1 (delta5 Desaturase) wie sie z.B. in WO2008089310 beschrieben sind;
Inhibitoren der Monoglycerid-Lipase (MGL) wie sie in WO2008145842 beschrieben sind;
hypoglykämische/hypertriglyceridämische Indolinverbindungen wie sie in WO2008039087, WO2009051119 beschrieben sind;
Inhibitoren des "Adipocyte fatty acid-binding protein aP2" wie z.B. BMS-309403 oder solchen wie sie in WO2009028248 beschrieben sind;
Aktivatoren der Adiponectinsekretion, wie z.B. in WO2006082978, WO2008105533, WO2008136173 beschrieben;
Promotoren der Adiponectinproduktion, wie z.B. in WO2007125946, WO2008038712 beschrieben;
modifizierte Adiponectine wie z.B. in WO2008121009 beschrieben;
Oxyntomodulin oder Analoga davon (wie z.B. TKS-1225);
Oleoyl-Estron
oder Agonisten oder partiellen Agonisten des Schilddrüsenhormonrezeptors (thyroid hormone receptor agonists) wie z. B: KB-2115 (Eprotirome), QRX-431 (Sobetirome) oder DITPA oder solche, wie in WO20058279, WO200172692, WO200194293, WO2003084915, WO2004018421, WO2005092316, WO2007003419, WO2007009913, WO2007039125, WO2007110225, WO2007110226, WO2007128492, WO2007132475, WO2007134864, WO2008001959, WO2008106213, JP2009155261 beschrieben
oder Agonisten des Schilddrüsenhormonrezeptors beta (TR-beta) wie z. B. MB-07811 oder MB-07344, oder solchen wie in WO2008062469 beschrieben, verabreicht.

Bei einer Ausführungsform der Erfindung wird die Verbindung der Formel I in Kombination mit einer Kombination von Eprotirome mit Ezetimibe verabreicht.

Bei einer Ausführungsform der Erfindung wird die Verbindung der Formel I in Kombination mit einem Inhibitor der Site-1 Protease (S1P), wie z.B. PF-429242, verabreicht.

Bei einer weiteren Ausführungsform der Erfindung wird die Verbindung der Formel I in Kombination mit einem Modulator des "Trace-Amine-Associated-Receptor-1" (TAAR1), wie sie z.B. in US2008146523, WO2008092785 beschrieben sind, verabreicht.

Bei einer Ausführungsform der Erfindung wird die Verbindung der Formel I in Kombination mit einem Inhibitor des Growth-Factor-Receptor-Bound-Protein-2 (GRB2), wie z.B. in WO2008067270 beschrieben, verabreicht.

Bei einer weiteren Ausführungsform der Erfindung wird die Verbindung der Formel I in Kombination mit einem RNAi (siRNA) Therapeutikum, welches gegen PCSK9 (Proprotein Convertase Subtilisin/Kexin Typ 9) gerichtet ist, verabreicht.

Bei einer Ausführungsform wird die Verbindung der Formel I in Kombination mit Omacor® oder Lovaza™ (Omega-3-Fettsäureester; hochkonzentrierte Ethylester der Eicosapentaensäure und der Docosahexaensäure) verabreicht.

Bei einer Ausführungsform wird die Verbindung der Formel I in Kombination mit Lycopin verabreicht.

Bei einer Ausführungsform der Erfindung wird die Verbindung der Formel I in Kombination mit einem Antioxidans, wie z.B. OPC-14117, AGI-1067 (Succinobucol), Probucol, Tocopherol, Ascorbinsäure, β-Caroten oder Selen oder solchen, wie sie in WO2009135918 beschrieben sind, verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinide und Metformin (PrandiMet (TM)), Insulin und einem Sulfonylharnstoff, Insulin und Metformin, Insulin und Troglitazon, Insulin und Lovastatin, etc. verabreicht.

Bei einer weiteren Ausführungsform wird die Verbindung der Formel I in Kombination mit einem Aktivator der löslichen Guanylatcyclase (soluble guanylate cyclase (sGC)) verabreicht wie sie z.B. in WO2009032249 beschrieben sind.

Bei einer anderen Ausführungsform wird die Verbindung der Formel I in Kombination mit einem Inhibitor der Carboanhydrase Typ 2 (Carbonic anhydrase type 2), wie z.B. solchen, wie in WO2007065948, WO2009050252 beschrieben, verabreicht.

Bei einer anderen Ausführungsform wird die Verbindung der Formel I in Kombination mit Topiramat oder einem Derivat davon, wie es in WO2008027557, US2009304789 beschrieben ist, verabreicht.

Bei einer weiteren Ausführungsform wird die Verbindung der Formel I in Kombination mit einer festen Kombination von Topiramat mit Phentermin (Qnexa^{™}) verabreicht.

Bei einer weiteren Ausführungsform wird die Verbindung der Formel I in Kombination mit einer Antisense-Verbindung, z.B. ISIS-377131, verabreicht, welche die Produktion des Glukokortikoidrezeptors inhibiert.

Bei einer anderen Ausführungsform wird die Verbindung der Formel I in Kombination mit einem Aldosteronsynthaseinhibitor und einem Antagonisten des Glucocorticoidrezeptors, einem Cortisolsyntheseinhibitor und/oder einem Antagonisten des Corticotropin-freisetzenden Faktors (corticotropin releasing factor), wie z.B. in EP1886695, WO2008119744 beschrieben, verabreicht.

Bei einer Ausführungsform wird die Verbindung der Formel I in Kombination mit einem Agonisten des RUP3 Rezeptors, wie z. B. in WO2007035355, WO2008005576 beschrieben, verabreicht.

Bei einer anderen Ausführungsform wird die Verbindung der Formel I in Kombination mit einem Aktivator des Gens, welches für die Ataxia Telangiectasia Mutated (ATM) Proteinkinase kodiert, wie z. B. Chloroquin, verabreicht.

Bei einer Ausführungsform wird die Verbindung der Formel I in Kombination mit einem Tau-Protein-Kinase-1-Inhibitor (TPK1 Inhibitor), wie z. B. in WO2007119463, WO2009035159, WO2009035162 beschrieben, verabreicht.

Bei einer Ausführungsform wird die Verbindung der Formel I in Kombination mit einem "c-Jun N-terminal kinase" Inhibitor (JNK-Inhibitor), wie z. B. BI-78D3 oder solchen wie in WO2007125405, WO2008028860, WO2008118626 beschrieben, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der neutralen Endopeptidase (NEP Inhibitoren), wie z.B. in WO2009138122, WO2009135526 beschrieben sind, verabreicht.

Bei einer Ausführungsform wird die Verbindung der Formel I in Kombination mit Modulatoren des Glukokortikoidrezeptors (GR), wie z.B. KB-3305 oder solchen Verbindungen wie sie z. B. in WO2005090336, WO2006071609, WO2006135826, WO2007105766, WO2008120661, WO2009040288, WO2009058944, WO2009108525, WO2009111214 beschrieben sind, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff ein Agonist des alpha 7-nikotinischen Acetylcholinrezeptors, wie sie z.B. in WO2009018551, WO2009071519, WO2009071576, WO2009071577 beschrieben sind.

Bei einer Ausführungsform ist der weitere Wirkstoff Trodusquemine.

Bei einer Ausführungsform ist der weitere Wirkstoff ein Modulator des Enzyms SIRT1 und/oder SIRT3 (einer NAD⁺-abhängigen Proteindeacetylase); dieser Wirkstoff kann z.B. Resveratrol in geeigneten Formulierungen sein, oder solche Verbindungen wie sie in WO2007019416 (z.B. SRT-1720), WO2008073451, WO2008156866, WO2008156869, WO2009026701, WO2009049018, WO2009058348, WO2009061453, WO2009134973, WO2009146358, WO2010003048 genannt sind.

Bei einer Ausführungsform der Erfindung ist der weitere Wirkstoff DM-71 (N-Acetyl-L-Cystein mit Bethanechol).

Bei einer Ausführungsform wird die Verbindung der Formel I in Kombination mit antihypercholesterolemisch wirkenden Verbindungen, wie sie z.B. in WO2004000803, WO2006000804, WO2004000805, WO2004087655, WO2005113496, WO2007059871, WO2007107587, WO2007111994, WO2008052658, WO2008106600, WO2008113796, US2008280836, WO2009113952, US2009312302 beschrieben sind, verabreicht.

Bei einer weiteren Ausführungsform wird die Verbindung der Formel I in Kombination mit Inhibitoren des SREBP (sterol regulatory element-binding protein), wie z.B. Fatostatin oder solchen wie sie z.B. in WO2008097835 beschrieben sind, verabreicht.

Bei einer anderen Ausführungsform wird die Verbindung der Formel I in Kombination mit einem cyclischen Peptidagonisten des VPAC2 Rezeptors, wie sie z.B. in WO2007101146, WO2007133828 beschrieben sind, verabreicht.

Bei einer weiteren Ausführungsform wird die Verbindung der Formel I in Kombination mit einem Agonisten des Endothelinrezeptors, wie sie z.B. in WO2007112069 beschrieben sind, verabreicht.

Bei einer weiteren Ausführungsform wird die Verbindung der Formel I in Kombination mit AKP-020 (Bis(ethylmaltolato)oxovanadium-IV) verabreicht.

Bei einer anderen Ausführungsform wird die Verbindung der Formel I in Kombination mit gewebe-selektiven Androgenrezeptor Modulatoren ("tissue-selective androgen receptor modulators"; SARM), wie sie z.B. in WO2007099200, WO2007137874 beschrieben sind, verabreicht.

Bei einer weiteren Ausführungsform wird die Verbindung der Formel I in Kombination mit einem AGE (advanced glycation endproduct) Inhibitor, wie sie z.B. in JP2008024673 beschrieben sind, verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer anderen Ausführungsform der Erfindung ist der weitere Wirkstoff Metreleptin (rekombinantes Methionyl-Leptin) kombiniert mit Pramlintide.

Bei einer weiteren Ausführungsform der Erfindung ist der weitere Wirkstoff das Tetrapeptid ISF-402.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin oder solche Derivate wie sie in WO2008034142 beschrieben sind.

Bei einer Ausführungsform ist der weitere Wirkstoff Mazindol oder Phentermin.

Bei einer weiteren Ausführungsform ist der weitere Wirkstoff Geniposidinsäure (geniposidic acid; WO2007100104) oder Derivate davon (JP2008106008).

Bei einer anderen Ausführungsform ist der weitere Wirkstoff ein Agonist des Neuropeptids FF2 wie er z.B. in WO2009038012 beschrieben ist.

Bei einer Ausführungsform ist der weitere Wirkstoff ein nasal verabreichter Calciumkanalblocker wie z.B. Diltiazem oder solche, wie sie in US 7,138,107 beschrieben sind.

Bei einer Ausführungsform ist der weitere Wirkstoff ein Inhibitor des Natrium-Calcium-Ionen-Austausches wie z.B. solche, wie sie in WO2008028958, WO2008085711 beschrieben sind.

Bei einer weiteren Ausführungsform ist der weitere Wirkstoff ein Blocker von Calciumkanälen wie z.B. des CaV3.2 oder CaV2.2 wie sie in WO2008033431, WO2008033447, WO2008033356, WO2008033460, WO2008033464, WO2008033465, WO2008033468, WO2008073461 beschrieben sind.

Bei einer Ausführungsform ist der weitere Wirkstoff ein Modulator eines Calciumkanals wie z.B. solche, wie sie in WO2008073934, WO2008073936, WO2009107660 beschrieben sind.

Bei einer Ausführungsform ist der weitere Wirkstoff ein Inhibitor des Calciummetabolismus wie z.B. solche, wie sie in US2009124680 beschrieben sind.

Bei einer Ausführungsform ist der weitere Wirkstoff ein Blocker des "T-type calcium channel" wie sie z.B. in WO2008033431, WO2008110008, US2008280900, WO2008141446, US2009270338, WO2009146540, US2009325979, WO2009146539 beschrieben sind.

Bei einer Ausführungsform ist der weitere Wirkstoff ein Inhibitor des KCNQ-Kaliumkanal-2 bzw. -3 wie z.B. solche, wie sie in US2008027049, US2008027090 beschrieben sind.

Bei einer Ausführungsform ist der weitere Wirkstoff ein Modulator des KCNN-Kaliumkanal-1, -2 bzw. -3 (Modulatoren des SK1-, SK2- und/oder SK3-Kanals) wie z.B. solche, wie sie in US2009036475 beschrieben sind.

Bei einer Ausführungsform ist der weitere Wirkstoff ein Inhibitor/Blocker des Kalium Kv1.3 Ionenkanals wie z.B. solchen, wie sie in WO2008040057, WO2008040058, WO2008046065, WO2009043117 beschrieben sind.

Bei einer Ausführungsform ist der weitere Wirkstoff ein Kaliumkanalmodulator wie z.B. solche, wie sie in WO2008135447, WO2008135448, WO2008135591, WO2009099820 beschrieben sind.

Bei einer weiteren Ausführungsform ist der weitere Wirkstoff ein hyperpolarisationsaktivierter und durch zyklisches Nukleotid gesteuerter Kalium-Natrium-Kanal Inhibitor ("hyperpolarisation-activated cyclic nucleotide-gated (HCN) potassium-sodium channel inhibitor") wie z.B. solche, wie sie in US2009069296 beschrieben sind.

Bei einer anderen Ausführungsform ist der weitere Wirkstoff ein Inhibitor des Natrium-Kalium-2-Chlorid (NKCC1) Co-Transporters wie z.B. solche, wie sie in WO2009130735 beschrieben sind.

Bei einer anderen Ausführungsform ist der weitere Wirkstoff ein Inhibitor spannungsgeleiteten Natiumkanals (völtage-gated sodium channel inhibitor) wie z.B. solche, wie sie in WO2009049180, WO2009049181 beschrieben sind.

Bei einer anderen Ausführungsform ist der weitere Wirkstoff ein Modulator des MCP-1 Rezeptors (monocyte chemoattractant protein-1 (MCP-1)) wie z.B. solche, wie sie in WO2008014360, WO2008014381 beschrieben sind.

Bei einer Ausführungsform ist der weitere Wirkstoff ein Modulator des Somatostatinrezeptors 3 (SSTR3) wie z.B. solche, wie sie in WO2009011836 beschrieben sind.

Bei einer Ausführungsform ist der weitere Wirkstoff ein Modulator des Somatostatinrezeptors 5 (SSTR5) wie z.B. solche, wie sie in WO2008019967, US2008064697, US2008249101, WO2008000692, US2008293756, WO2008148710 beschrieben sind.

Bei einer Ausführungsform ist der weitere Wirkstoff ein Modulator des Somatostatinrezeptors 2 (SSTR2) wie z.B. solche, wie sie in WO2008051272 beschrieben sind.

Bei einer Ausführungsform ist der weitere Wirkstoff eine Verbindung, welche in der Lage ist, die Menge des Retinol-bindenden Proteins 4 (RBP4) zu reduzieren, wie z.B. solche, wie sie in WO2009051244, WO2009145286 sind.

Bei einer Ausführungsform ist der weitere Wirkstoff ein Erythropoietin-mimetisches Peptid, welches als Erythropoietin (EPO) Rezeptoragonist agiert. Solche Moleküle sind z.B. in WO2008042800 beschrieben.

Bei einer weiteren Ausführungsform ist der weitere Wirkstoff ein Anorektikum/eine hypoglykämische Verbindung wie z.B. solche, wie sie in WO2008035305, WO2008035306, WO2008035686 beschrieben sind.

Bei einer Ausführungsform ist der weitere Wirkstoff ein Induktor der Liponsäuresynthetase wie z.B. solche, wie sie in WO2008036966, WO2008036967 beschrieben sind.

Bei einer Ausführungsform ist der weitere Wirkstoff ein Stimulator der endothelialen Nitric-Oxid-Synthase (eNOS) wie z.B. solche, wie sie in WO2008058641, WO2008074413 beschrieben sind.

Bei einer Ausführungsform ist der weitere Wirkstoff ein Modulator des Kohlenhydrat- und/oder Lipidstoffwechsels wie z.B. solche, wie sie in WO2008059023, WO2008059024, WO2008059025, WO2008059026 beschrieben sind.

Bei einer weiteren Ausführungsform ist der weitere Wirkstoff ein Angiotensin II Rezeptorantagonist wie z.B. solche, wie sie in WO2008062905, WO2008067378, WO2008062905 beschrieben sind.

Bei einer Ausführungsform ist der weitere Wirkstoff ein Agonist des Sphingosin-1-Phosphatrezeptors (S1P) wie z.B. solche, wie sie in WO2008064315, WO2008074820. WO2008074821, WO2008135522, WO2009019167, WO2009043013, WO2009080663, WO2009085847, WO2009151529, WO2009151621, WO2009151626, WO2009154737 beschrieben sind.

Bei einer Ausführungsform ist der weitere Wirkstoff ein Mittel, welches die Magenentleerung retardiert wie z.B. 4-Hydroxyisoleucin (WO2008044770).

Bei einer Ausführungsform ist der weitere Wirkstoff ein Trytophan-5-Hydroxylase-Inhibitor-1 (TPH1 Inhibitor), welcher die gastrointestinale Motilität moduliert wie z.B. in WO2009014972 beschrieben.

Bei einer Ausführungsform ist der weitere Wirkstoff eine Muskel-relaxierende Substanz wie sie z.B. in WO2008090200 beschrieben ist.

Bei einer weiteren Ausführungsform ist der weitere Wirkstoff ein Inhibitor der Monoaminoxidase B (MAO-B) wie z.B. solche, wie sie in WO2008092091, WO2009066152 beschrieben sind.

Bei einer weiteren Ausführungsform ist der weitere Wirkstoff ein Inhibitor der Monoaminoxidase A (MAO-A) wie z.B. solche, wie sie in WO2009030968 beschrieben sind.

Bei einer anderen Ausführungsform ist der weitere Wirkstoff ein Inhibitor der Bindung von Cholesterol und/oder Triglyceriden an das SCP-2 Protein (sterol carrier protein-2) wie z.B. solche, wie sie in US2008194658 beschrieben sind.

Bei einer weiteren Ausführungsform ist der weitere Wirkstoff eine Verbindung, welche an die β-Untereinheit des trimeren GTP-bindenden Proteins bindet, z.B. solchen wie sie in WO2008126920 beschrieben sind.

Bei einer Ausführungsform ist der weitere Wirkstoff ein Inhibitor des Harnsäureanionaustauschers-1 (urate-anion-exchanger-inhibitor-1), wie sie z.B. in WO2009070740 beschrieben sind.

Bei einer Ausführungsform ist der weitere Wirkstoff ein Modulator des ATP-Transporters, wie z.B. in WO2009108657 beschrieben.

Bei einer anderen Ausführungsform ist der weitere Wirkstoff Lisofylline, welcher Autoimmunschäden an insulinproduzierenden Zellen verhindert.

Bei einer noch anderen Ausführungsform ist der weitere Wirkstoff ein Extrakt aus Bidens pilosa mit dem Inhaltsstoff Cytopiloin wie in EP1955701 beschrieben.

Bei einer Ausführungsform ist der weitere Wirkstoff ein Inhibitor der Glucosylceramid-Synthase wie z.B. in WO2008150486 beschrieben.

Bei einer weiteren Ausführungsform der Erfindung ist der weitere Wirkstoff ein Glycosidaseinhibitor wie z.B. in WO2009117829, WO2009155753 beschrieben.

Bei einer anderen Ausführungsform ist der weitere Wirkstoff ein Inhaltsstoff der Pflanze *Hoodia Gordonii* wie er in US2009042813, EP2044852 beschrieben ist.

Bei einer Ausführungsform ist der weitere Wirkstoff ein Antidiabetikum wie z.B. D-Tagatose.

Bei einer Ausführungsform ist der weitere Wirkstoff ein Zinkkomplex von Curcumin wie er in WO2009079902 beschrieben ist.

Bei einer Ausführungsform ist der weitere Wirkstoff ein Inhibitor des "cAMP response element binding protein" (CREB) wie er in WO2009143391 beschrieben ist.

Bei einer anderen Ausführungsform ist der weitere Wirkstoff ein Antagonist des Bradykinin B1 Rezeptors wie er in WO2009124746 beschrieben ist.

Bei einer weiteren Ausführungsform ist der weitere Wirkstoff eine Verbindung, die in der Lage ist, die diabetische periphere Neuropathie (DPN) zu modulieren. Solche Modulatoren sind z.B. FK-1706 oder SB-509 oder solche wie sie in WO1989005304, WO2009092129, WO2010002956 beschrieben sind.

Bei einer Ausführungsform ist der weitere Wirkstoff eine Verbindung, die in der Lage ist, die diabetische Nephropathie zu modulieren. Solche Verbindungen sind z.B. in WO2009089545, WO2009153261 beschrieben.

Bei einer Ausführungsform ist der weitere Wirkstoff ein Inhibitor (z.B. ein Anti-CD38 Antikörper) von CD38 wie in US2009196825 beschrieben.

Bei einer Ausführungsform ist der weitere Wirkstoff ein Inhibitor des humanen Fibroblastenwachstumsfaktor-Rezeptor 4 (human fibroblast growth factor receptor 4 (FGFR4)) wie z.B. in WO2009046141 beschrieben.

Bei einer weiteren Ausführungsform der Erfindung ist der weitere Wirkstoff eine die Betazelle schützende Verbindung wie z.B. 14-alpha-Lipolyl-andrographolide (AL-1).

Bei einer noch anderen Ausführungsform der Erfindung ist der weitere Wirkstoff das INGAP Peptid (islet neogenesis associated protein), ein Peptid, welches die Insulinproduktion in Patienten mit Diabetes Mellitus wieder herstellt.

Bei einer Ausführungsform der Erfindung ist der weitere Wirkstoff ein Modulator des CFTR (cystic fibrosis transmembrane conductance regulator) wie er z.B. in US2009246137, US2009264433, US2009264441, US2009264471, US2009264481, US2009264486, WO2010019239 beschrieben ist.

Bei einer Ausführungsform der Erfindung ist der weitere Wirkstoff eine Verbindung, welche die Insulinfreisetzung stimuliert/moduliert, wie z.B. solche wie sie in WO2009109258, WO2009132739, US2009281057, WO2009157418 beschrieben sind.

Bei einer Ausführungsform der Erfindung ist der weitere Wirkstoff ein Extrakt aus *Hippophae rhamnoides,* wie er z.B. in WO2009125071 beschrieben ist.

Bei einer Ausführungsform der Erfindung ist der weitere Wirkstoff ein aus *Huanglian* und *Ku Ding Cha,* wie er z.B. in WO2009133458 beschrieben ist.

Bei einer anderen Ausführungsform der Erfindung ist der weitere Wirkstoff ein Wurzelextrakt aus *Cipadessa baccifera,* wie er in US2009238900 beschrieben ist.

Bei einer Ausführungsform der Erfindung sind die weiteren Wirkstoffe Borapetoside A und/oder Borapetoside C, welche aus der Pflanze SDH-V, einer Species von *Tinospora crispa*, isoliert werden können, wie sie z.B. in US2010016213 beschrieben sind.

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

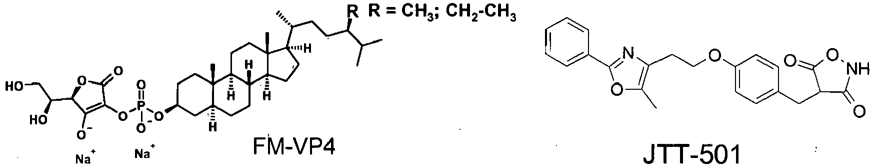

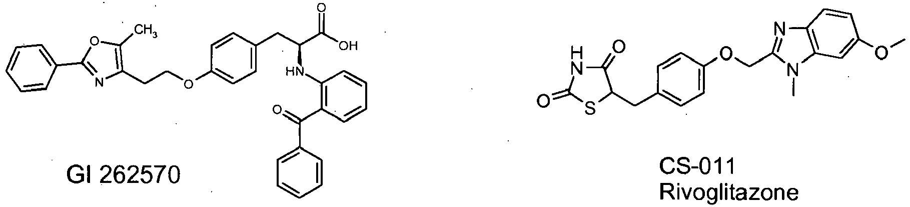

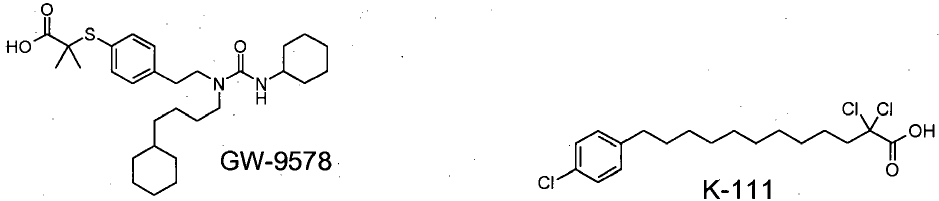

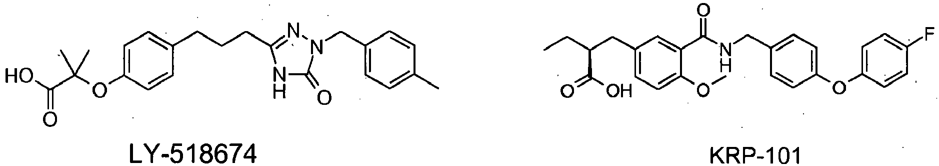

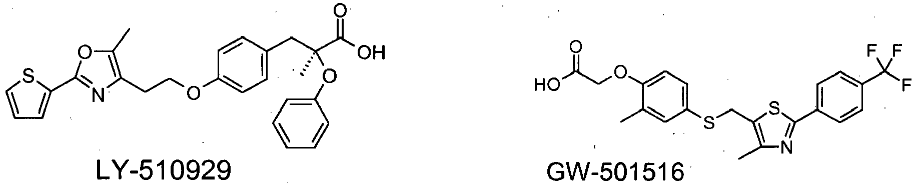

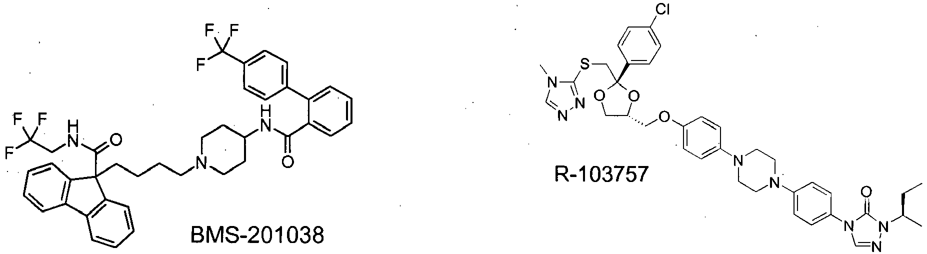

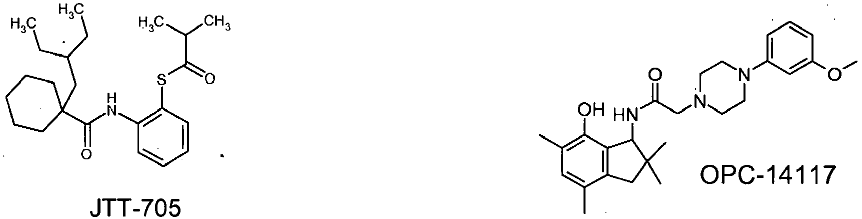

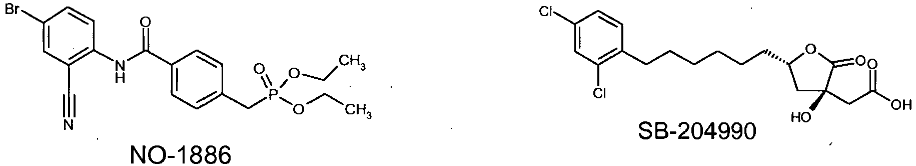

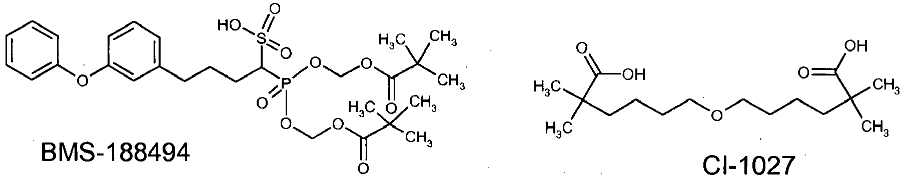

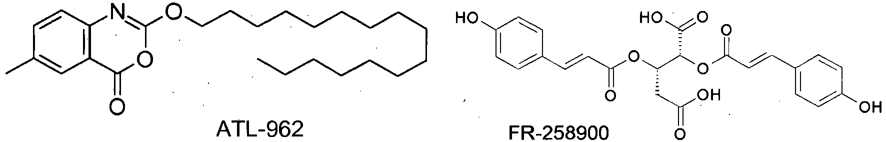

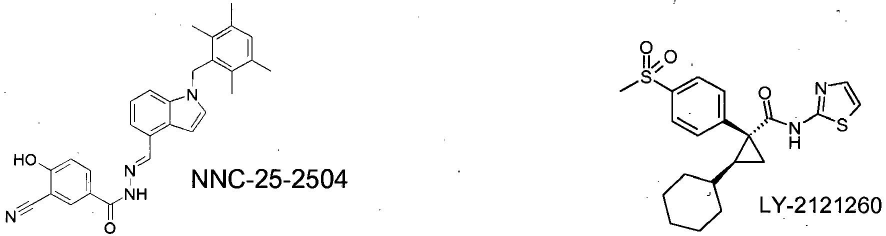

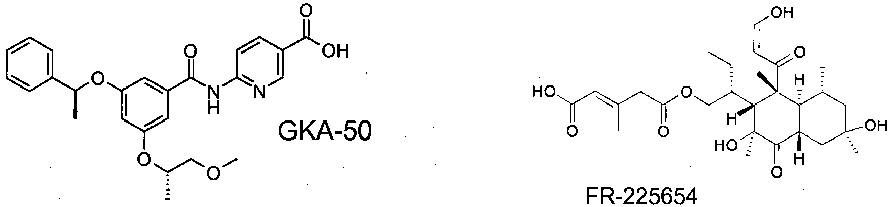

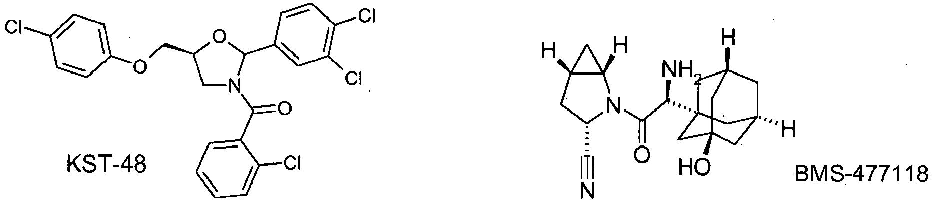

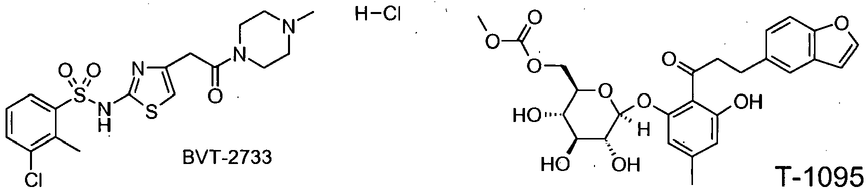

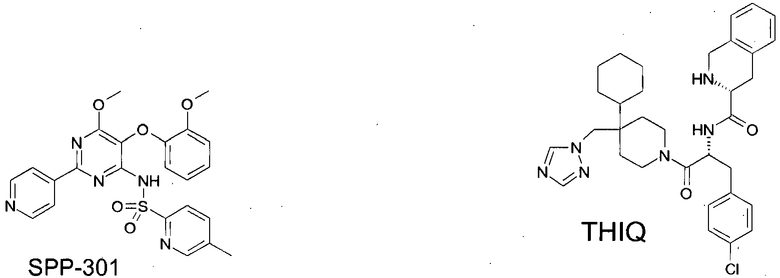

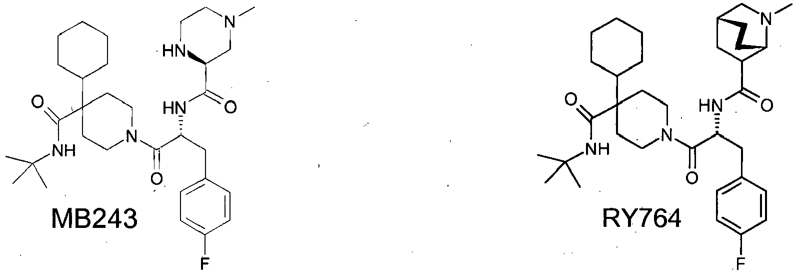

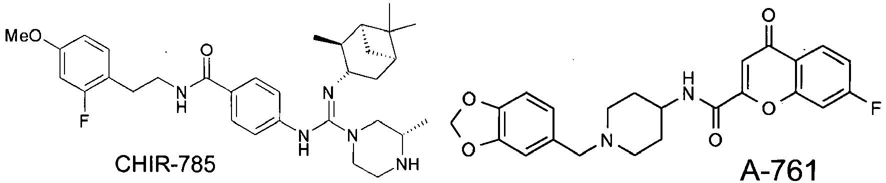

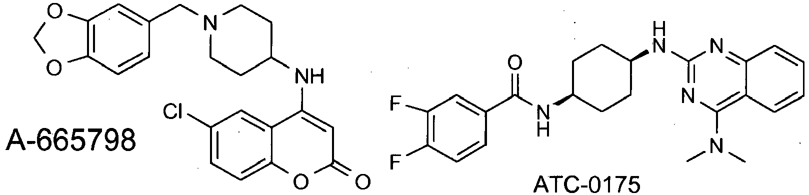

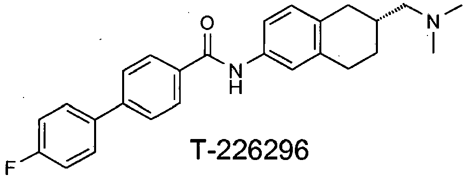

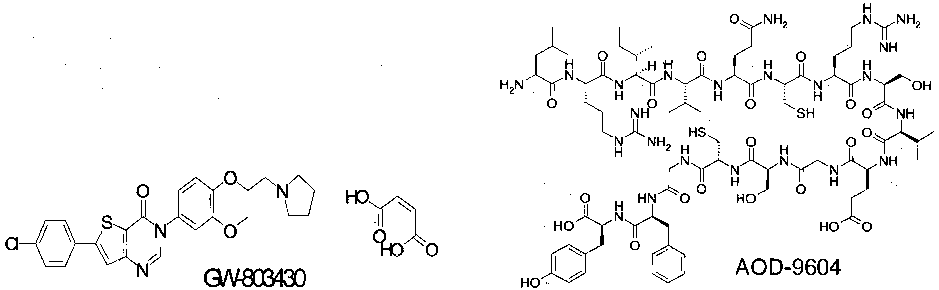

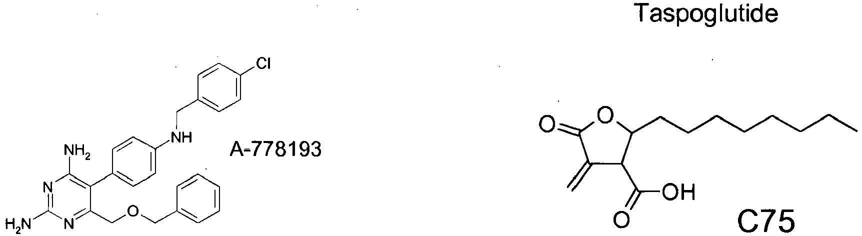

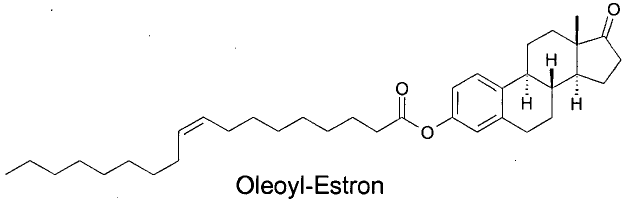

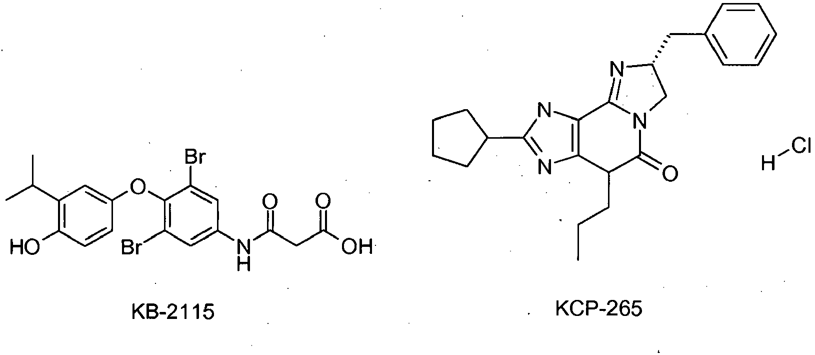

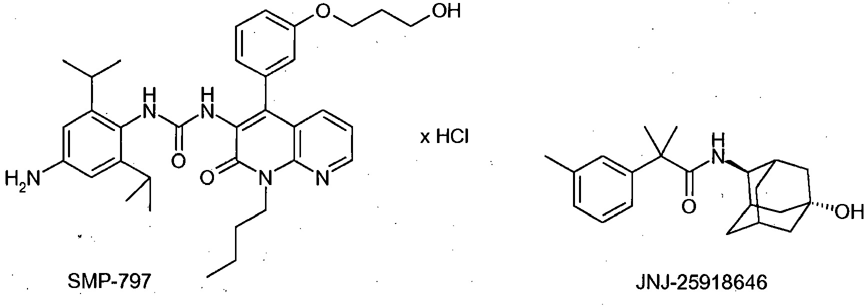

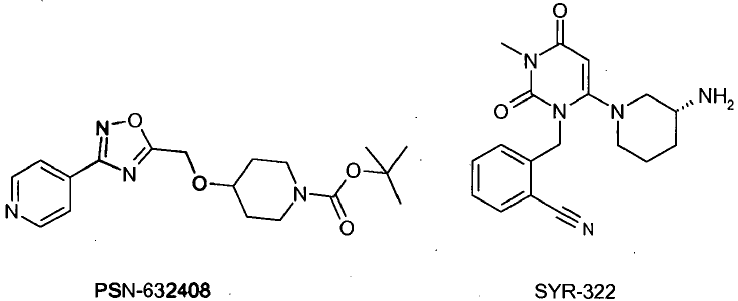

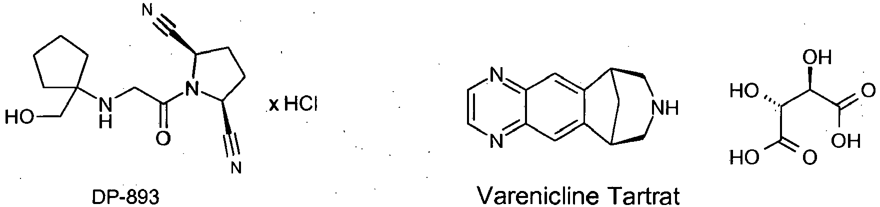

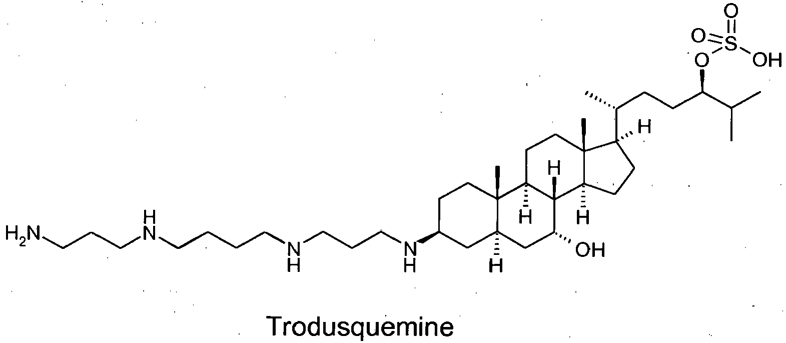

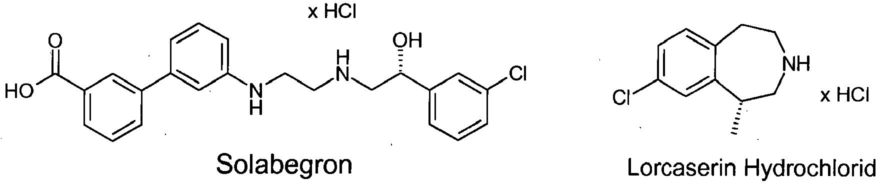

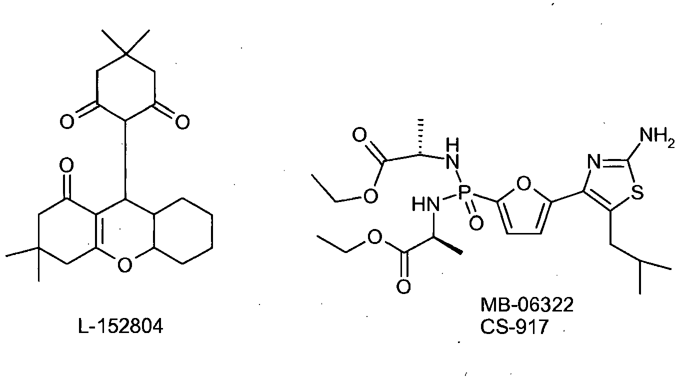

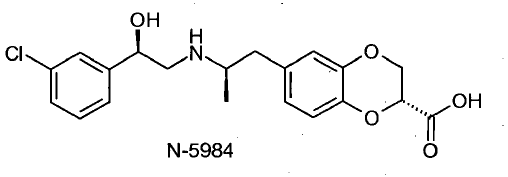

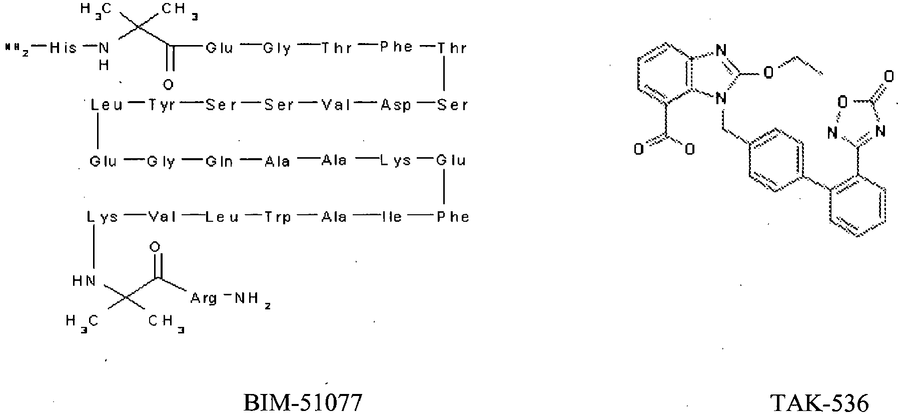

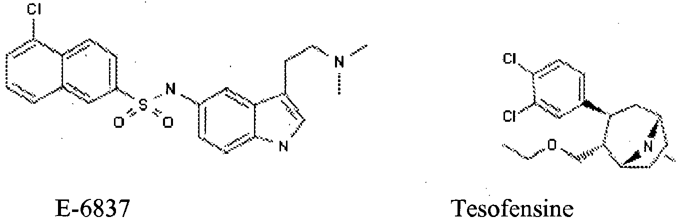

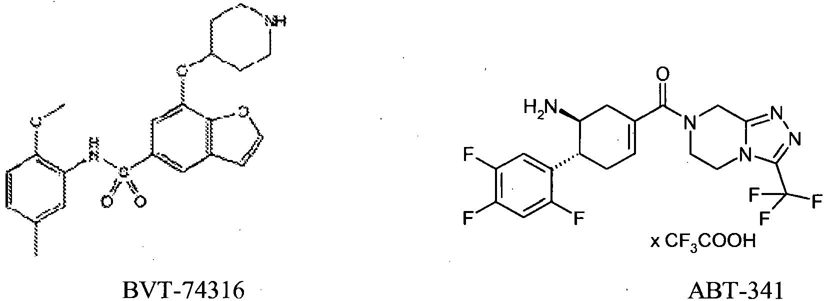

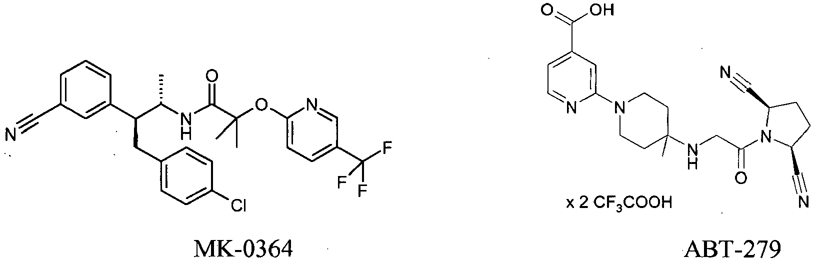

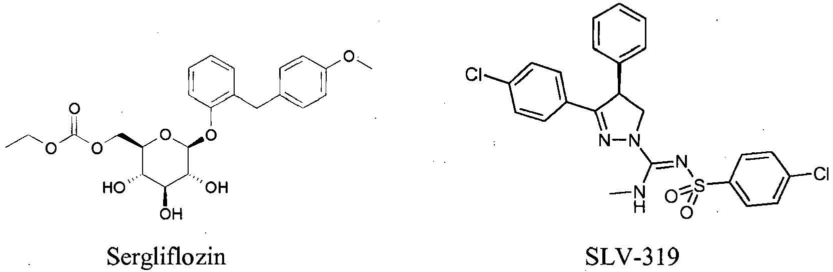

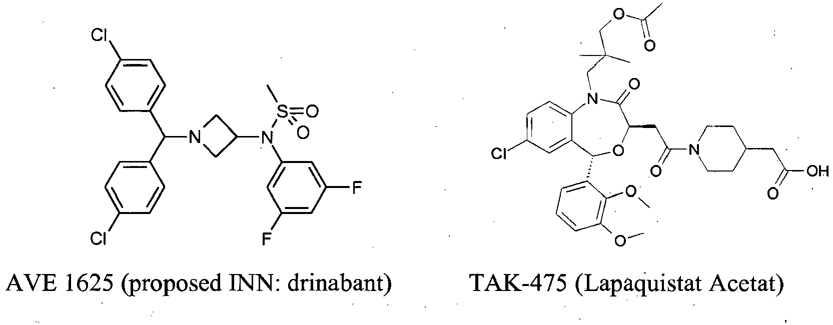

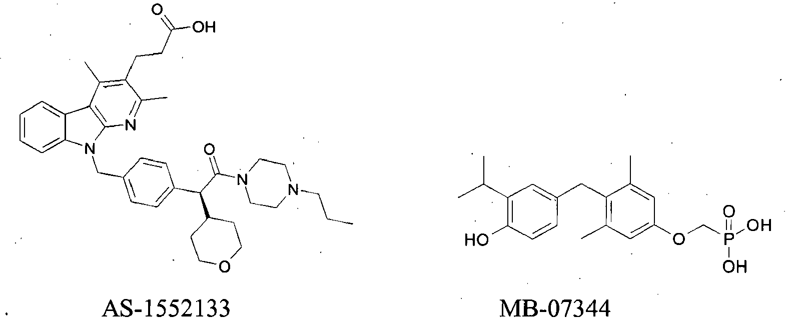

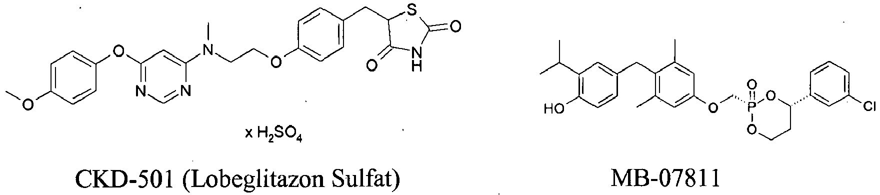

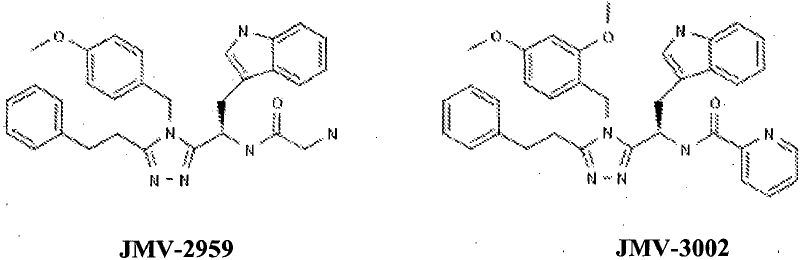

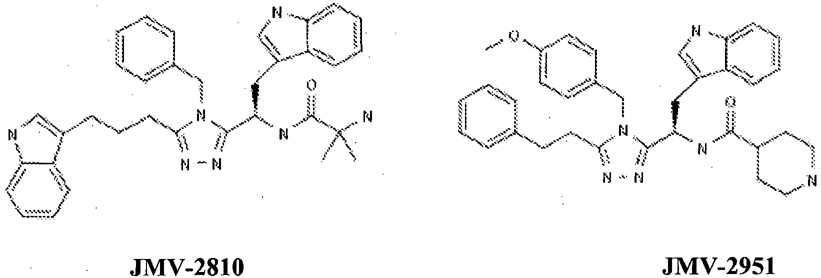

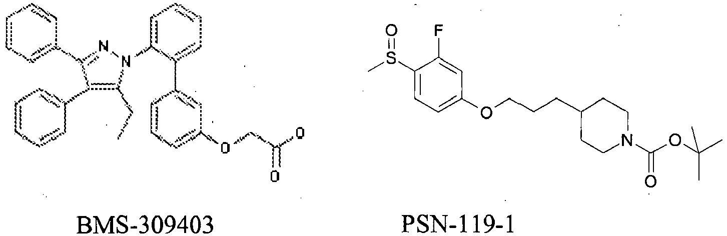

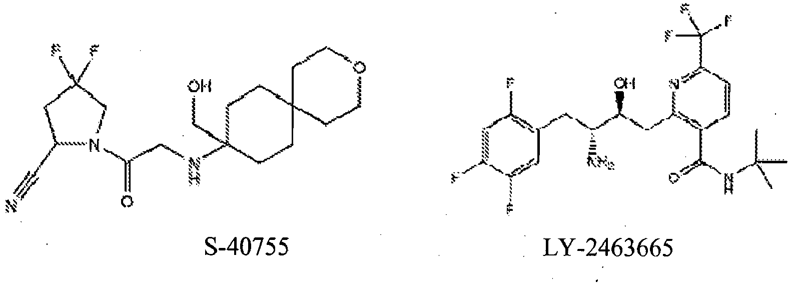

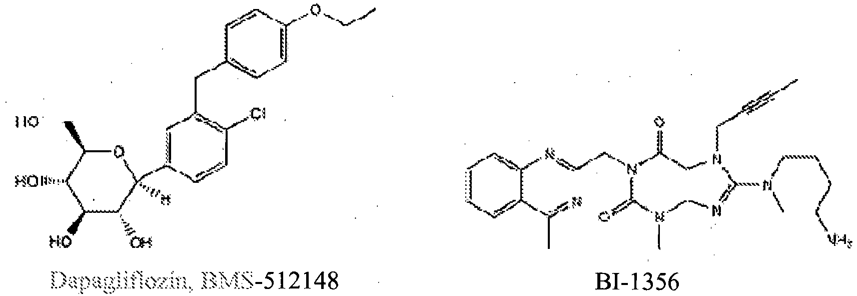

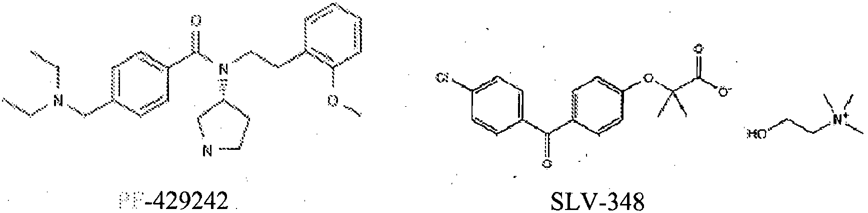

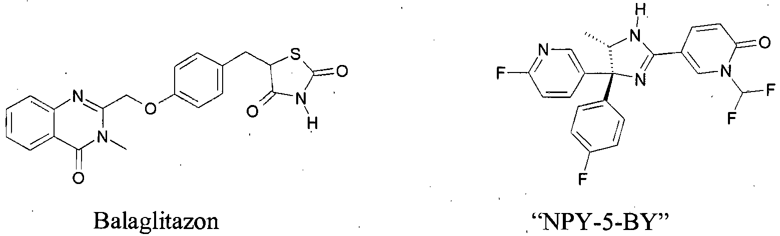

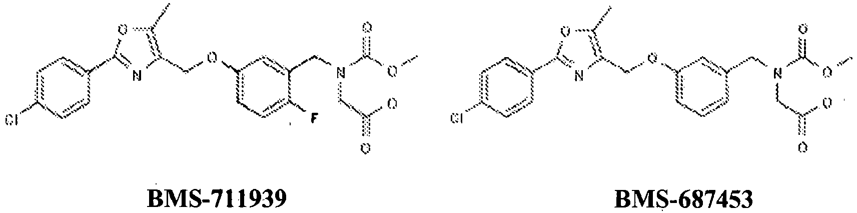

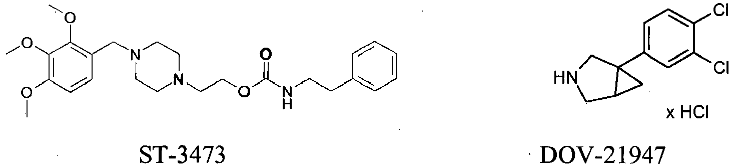

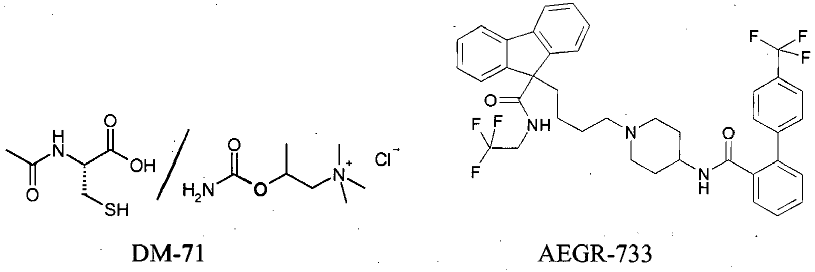

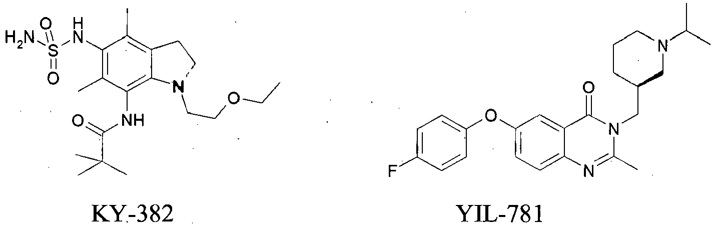

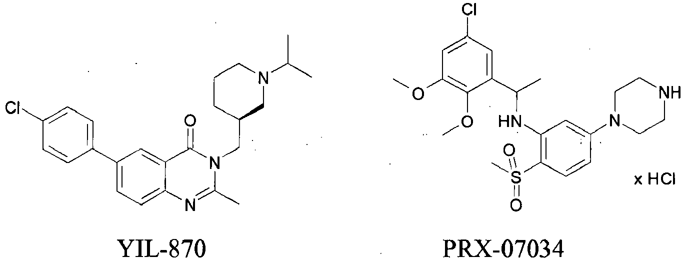

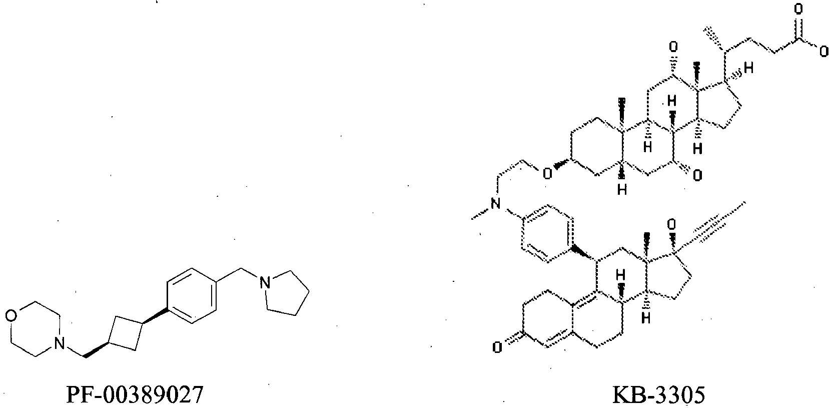

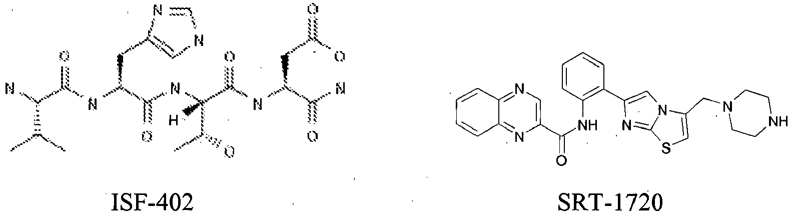

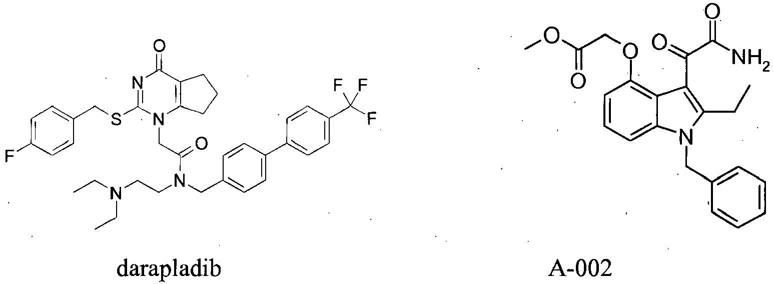

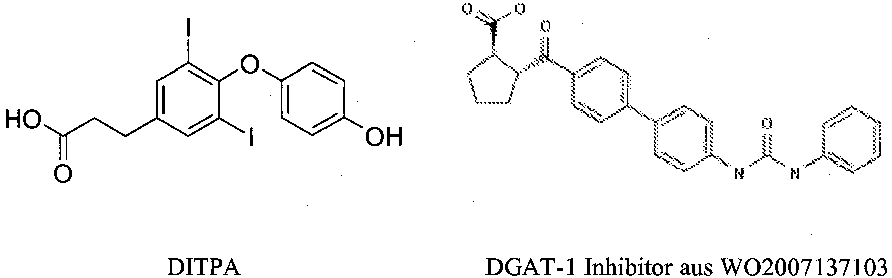

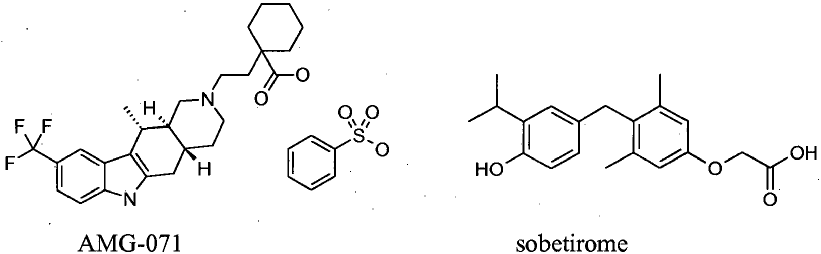

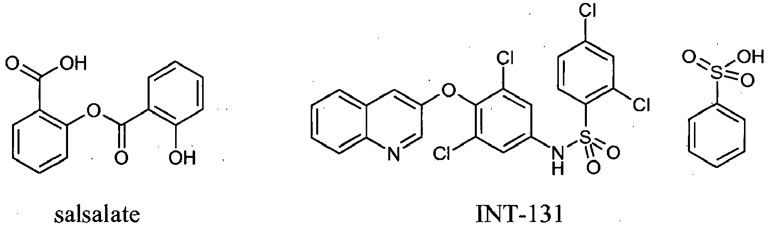

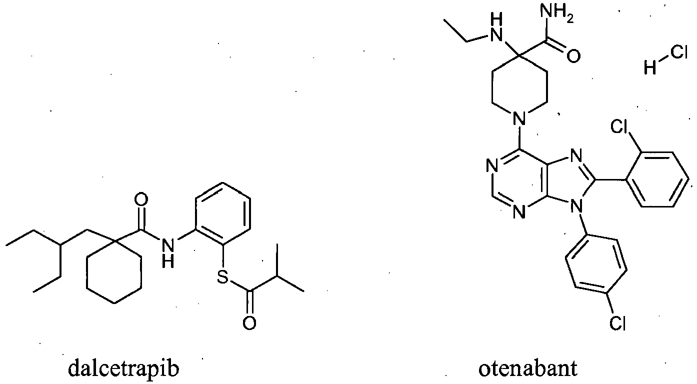

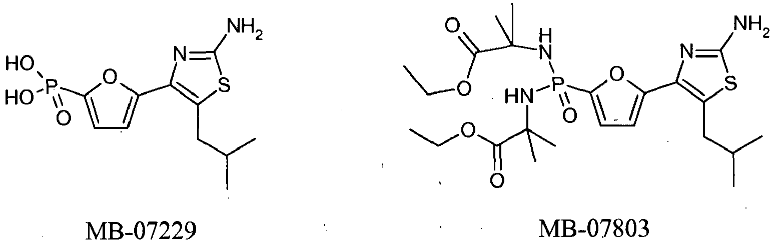

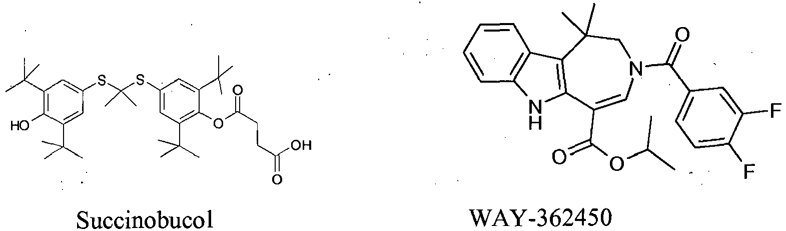

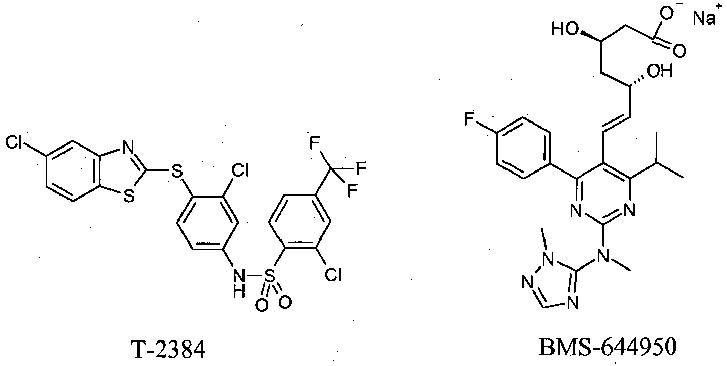

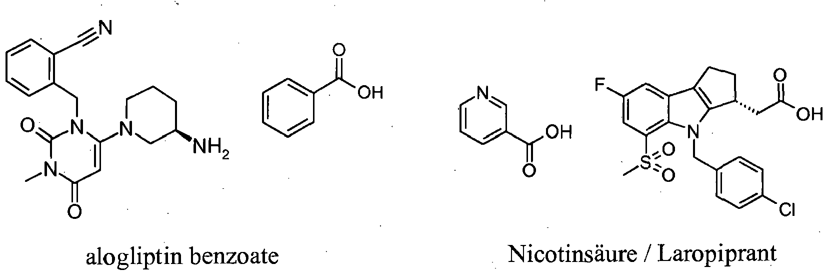

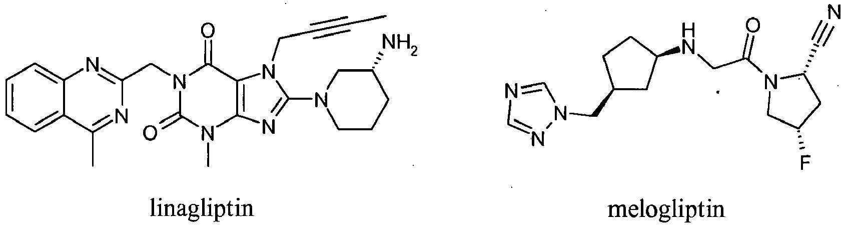

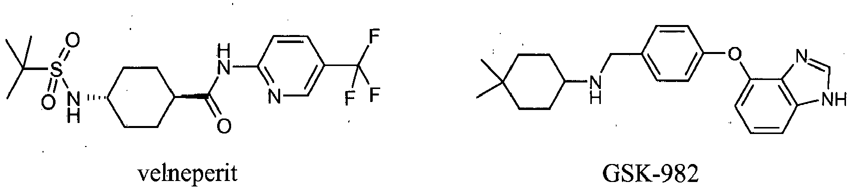

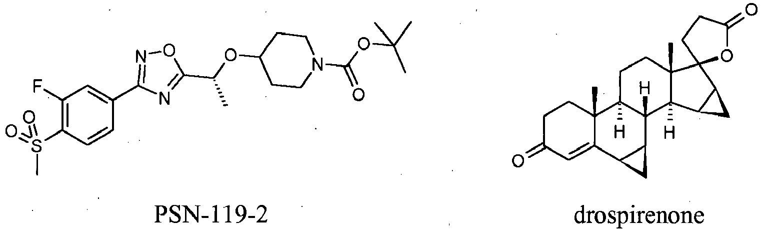

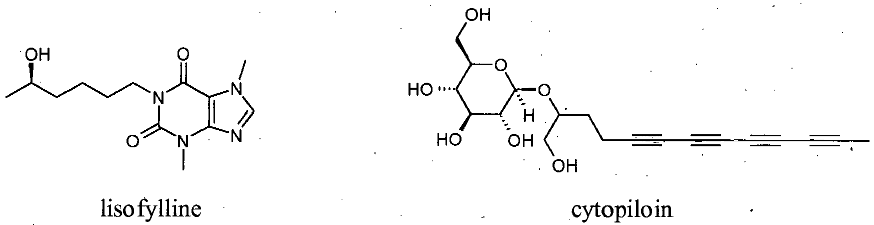

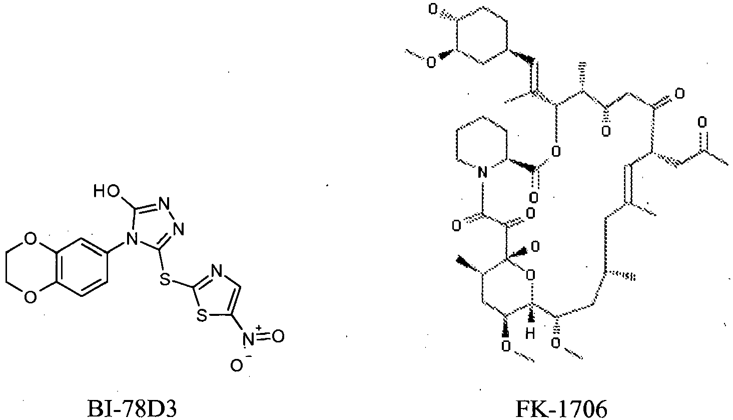

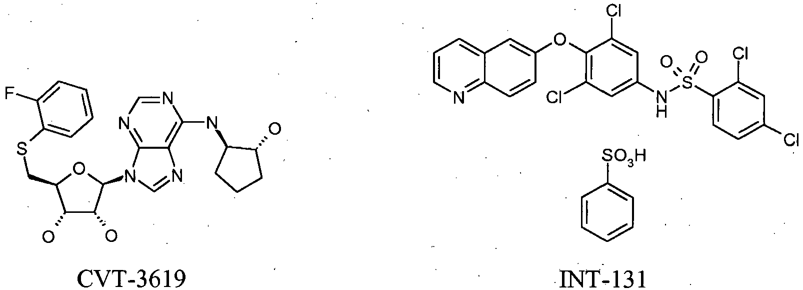

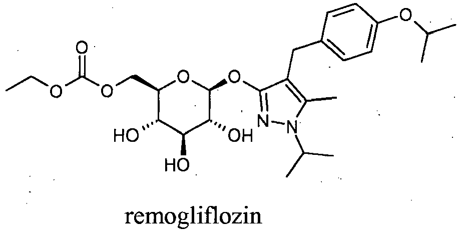

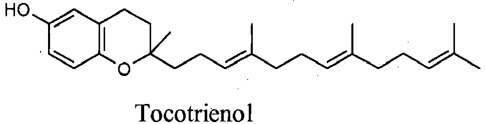

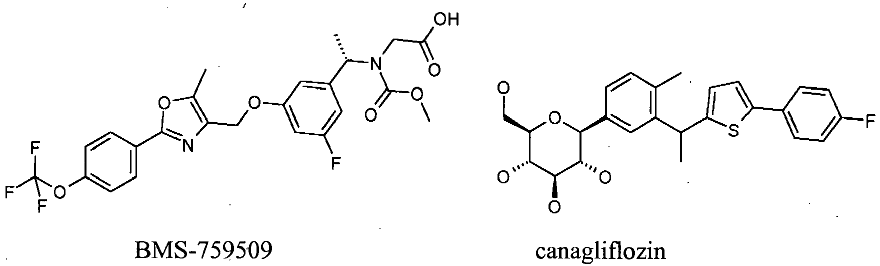

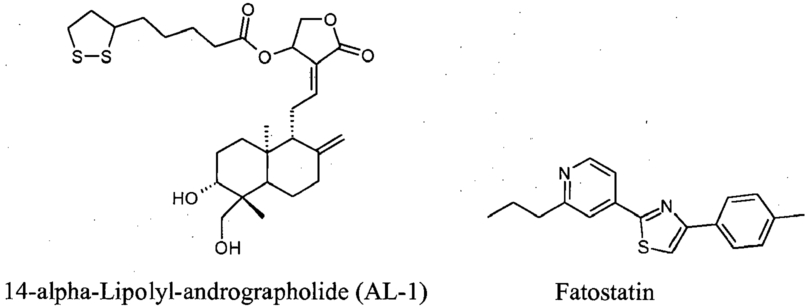

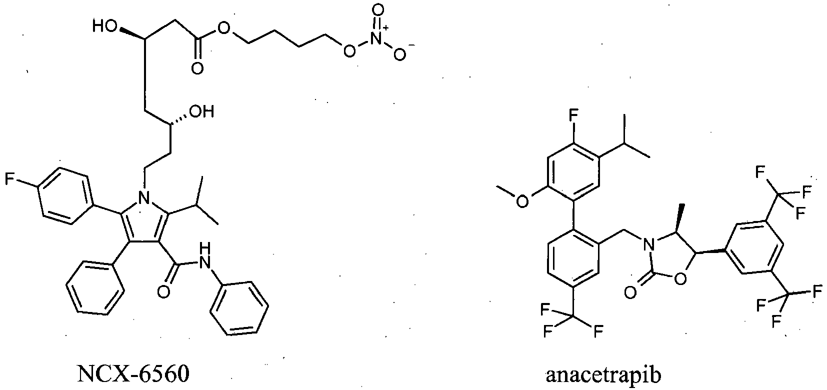

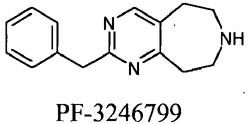

Weiterhin sind folgende Wirkstoffe für Kombinationspräparate geeignet:
Alle Antiepileptika, die in der Roten Liste 2010, Kapitel 15 genannt sind;
alle Antihypertonika, die in der Roten Liste 2010, Kapitel 17 genannt sind;
alle Hypotonika, die in der Roten Liste 2010, Kapitel 19 genannt sind;
alle Antikoagulantia, die in der Roten Liste 2010, Kapitel 20 genannt sind;
alle Arteriosklerosemittel, die in der Roten Liste 2010, Kapitel 25 genannt sind;
alle Betarezeptoren-, Calciumkanalblocker und Hemmstoffe des Renin-Angiotensin-Systems, die in der Roten Liste 2010, Kapitel 27 genannt sind;
alle Diuretika und Durchblutungsfördernde Mittel, die in der Roten Liste 2010, Kapitel 36 und 37 genannt sind;
alle Entwöhnungsmittel/Mittel zur Behandlung von Suchterkrankungen, die in der Roten Liste 2010, Kapitel 39 genannt sind;
alle Koronarmittel und Magen-Darm-Mittel, die in der Roten Liste 2010, Kapitel 55 und 60 genannt sind;
alle Migränemittel, Neuropathiepräparate und Parkinsonmittel, die in der Roten Liste 2010, Kapitel 61, 66 und 70 genannt sind.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

### Allgemeines Herstellungsverfahren

Die erfindungsgemäßen Verbindungen der Formel I können entsprechend des folgenden Reaktionsschemas hergestellt werden:

### Verfahren A:

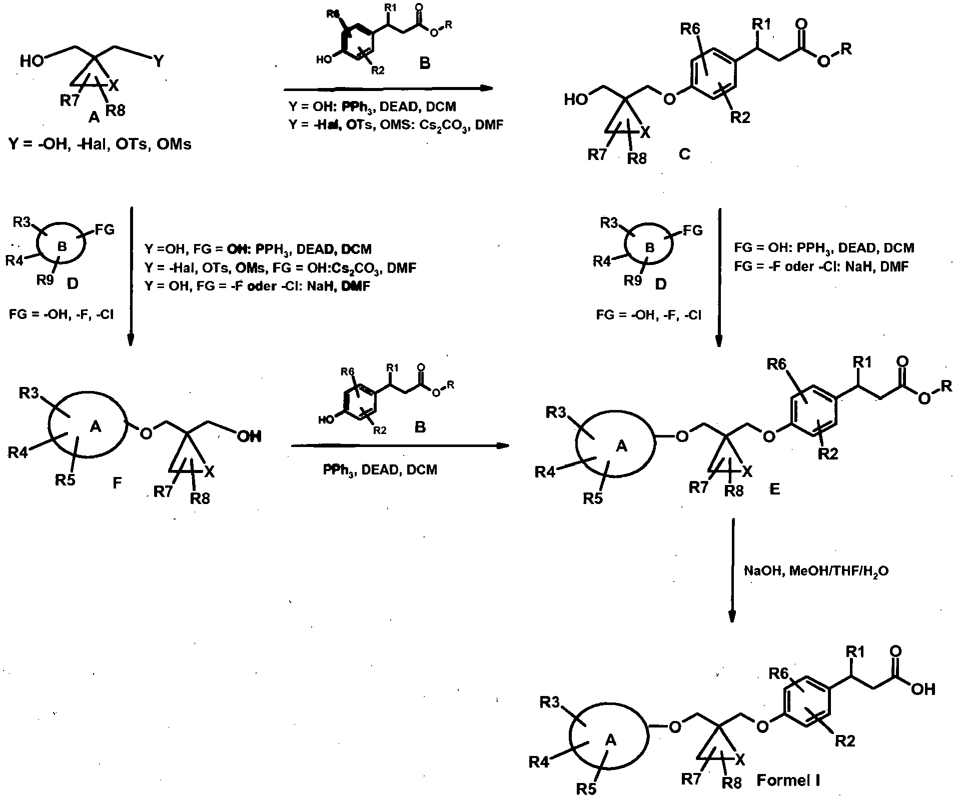

Eine Spiroverbindung der allgemeinen Formel A, worin X, R7 und R8 die oben beschriebene Bedeutungen haben, wird mit einem Phenol der allgemeinen Formel B, worin R1, R2 und R6 die oben beschriebenen Bedeutungen haben und R eine Alkylgruppe wie Methyl oder Ethyl bedeutet, für den Fall, dass Y eine Hydroxylgruppe ist unter Mitsunobu Bedingungen, in Gegenwart von zum Beispiel Triphenylphosphin und Diethyldiazodicarboxylate in einem aprotischen Lösungsmittel wie zum Beispiel Dichlormethan zum spirocyclischen substituierten 1,3-Propandioxidderivat der allgemeinen Formel C umgesetzt. Für den Fall, dass Y ein Halogenid wie zum Beispiel Bromid oder eine Abgangsgruppe wie zum Beispiel Mesylat oder Tosylat bedeutet, findet die Umsetzung zur Verbindung der allgemeinen Formel C in einem polar aprotischen Lösungsmittel wie zum Beispiel Dimethylformamid in Gegenwart einer Base wie zum Beispiel Cäsiumcarbonat statt. Die Verbindung der allgemeinen Formel C wird entweder unter Mitsunobu Bedingungen, in Gegenwart von zum Beispiel Triphenylphosphin und Diethyldiazodicarboxylate in einem aprotischen Lösungsmittel wie zum Beispiel Dichlormethan, mit einer Verbindung der allgemeinen Formel D, worin B, R3, R4 und R5 die oben beschriebenen Bedeutungen haben und FG eine Hydroxylgruppe bedeutet, oder unter den Bedingungen einer aromatischen nucleophilen Substitution, in einem polar aprotischen Lösungsmittel wie zum Beispiel Dimethylformamid in Gegenwart einer Base wie zum Beispiel Natriumhydrid, mit einer Verbindung der allgemeinen Formel D, worin B, R3, R4 und R5 die oben beschriebenen Bedeutungen haben und FG ein Fluor- oder Chloratom bedeutet, zum spirocyclische substituierten 1,3-Propandioxidderivat der allgemeinen Formel E umgesetzt. Das spirocyclisch substituierten 1,3-Propandioxidderivat der allgemeinen Formel E kann man alternativ auch erhalten, indem man zuerst die Spiroverbindung der allgemeinen Formel A, worin X, R7 und R8 die oben beschriebenen Bedeutungen haben, entweder unter Mitsunobu Bedingungen für den Fall, dass Y eine Hydroxylgruppe bedeutet, in Gegenwart von zum Beispiel Triphenylphosphin und Diethyldiazodicarboxylate in einem aprotischen Lösungsmittel wie zum Beispiel Dichlormethan, mit einer Verbindung der allgemeinen Formel D, worin B, R3, R4 und R5 die oben beschriebenen Bedeutungen haben und FG eine Hydroxylgruppe bedeutet, oder unter den Bedingungen einer aromatischen nucleophilen Substitution, für den Fall, dass Y eine Hydroxylgruppe bedeutet, in einem polar aprotischen Lösungsmittel wie zum Beispiel Dimethylformamid in Gegenwart einer Base wie zum Beispiel Natriumhydrid, mit einer Verbindung der allgemeinen Formel D, worin B, R3, R4 und R5 die oben beschriebenen Bedeutungen haben und FG ein Fluor- oder Chloratom bedeutet, zum spirocyclische substituierten 1,3-Propandioxidderivat der allgemeinen Formel F umsetzt. Für den Fall, dass Y ein Halogenid wie zum Beispiel Bromid oder eine Abgangsgruppe wie zum Beispiel Mesylat oder Tosylat und FG eine Hydroxylgruppe bedeutet, findet die Umsetzung zur Verbindung der allgemeinen Formel F in einem polar aprotischen Lösungsmittel wie zum Beispiel Dimethylformamid in Gegenwart einer Base wie zum Beispiel Cäsiumcarbonat statt. Die Verbindung der allgemeinen Formel F wird dann unter Mitsunobu Bedingungen, in Gegenwart von zum Beispiel Triphenylphosphin und Diethyldiazodicarboxylate in einem aprotischen Lösungsmittel wie zum Beispiel Dichlormethan, mit einem Phenol der allgemeinen Formel B, worin R1, R2 und R6 die oben beschriebenen Bedeutungen haben und R eine Alkylgruppe wie Methyl oder Ethyl bedeutet, zum spirocyclischen substituierten 1,3-Propandioxidderivat der allgemeinen Formel E umgesetzt. Unter Einwirkung einer Base wie zum Beispiel Natriumhydroxid in einem Lösungsmittelgemisch wie zum Beispiel Methanol, Tetrahydrofuran und Wasser wird der Ester der allgemeinen Formel E gespalten und man erhält die freie Carbonsäure der allgemeinen Formel I.
Nach diesem Verfahren wurden die Beispiele 1 - 37 hergestellt.

Die nachfolgend aufgeführten Beispiele dienen zur Erläuterung der Erfindung, ohne diese jedoch einzuschränken.

**Tabelle 1:**

| | | | | | | | | | | |
|---|---|---|---|---|---|---|---|---|---|---|
| 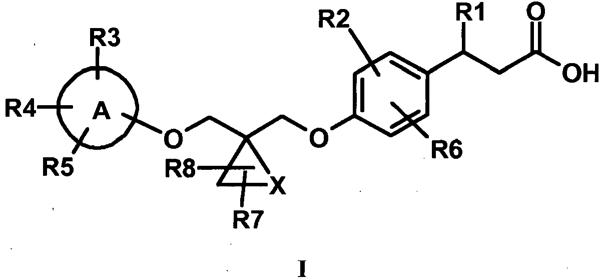 | | | | | | | | | | |

| **Bsp.** | **R1** | **R2** | **R6** | **R3** | **R4** | **R5** | **R7** | **R8** | **X** | **A** |
|---|---|---|---|---|---|---|---|---|---|---|
| **1** | -C≡C-CH₃ | H | H | 3-C(CH₃)₃ | H | H | H | H | -CH₂- | Phenyl |
| **2** | -C≡C-CH₃ | H | H | 4-CF₃ | H | H | H | H | -CH₂- | Phenyl |
| **3** | -C≡C-CH₃ | H | H | 3-Cl | H | H | H | H | -CH₂- | Phenyl |
| **4** | -C≡C-CH₃ | H | H | 4-CF₃ | 2-Cl | H | H | H | -OCH₂- | Phenyl |
| **5** | -C≡C-CH₃ | H | H | 3-C(CH₃)₃ | H | H | H | H | -CH₂CH₂- | Phenyl |
| **6** | -C≡C-CH₃ | H | H | 4-CF₃ | H | H | H | H | -CH₂CH₂- | Phenyl |
| **7** | -C≡C-CH₃ | H | H | 3-CH₃ | H | H | H | H | -CH₂CH₂- | Phenyl |
| **8** | -C≡C-CH₃ | H | H | H | H | H | H | H | -CH₂CH₂- | Phenyl |
| **9** | -C≡C-CH₃ | H | H | 3-CF₃ | 5-CF₃ | H | H | H | -CH₂- | Phenyl |
| **10** | -C≡C-CH₃ | H | H | H | H | H | H | H | -CH₂- | Phenyl |
| **11** | -C≡C-CH₃ | H | H | 3-CH₃ | H | H | H | H | -CH₂- | Phenyl |
| **12** | -C≡C-CH₃ | H | H | 3-Cl | 4-CN | H | H | H | -CH₂- | Phenyl |
| **13** | -C≡C-CH₃ | H | H | 6-CF₃ | H | H | H | H | -CH₂- | 3-Pyridyl |
| **14** | -C≡C-CH₃ | H | H | 5-CF₃ | H | H | H | H | -CH₂- | 2-Pyridyl |
| **15** | -C≡C-CH₃ | H | H | 2-Cl | 4-CF₃ | H | H | H | -CH₂- | Phenyl |
| **16** | -OCH₃ | H | H | 5-CF₃ | H | H | H | H | -CH₂- | 2-Pyridyl |
| **17** | -OCH₃ | H | H | 6-CF₃ | H | H | H | H | -CH₂- | 3-Pyridyl |
| **18** | -C≡C-CH₃ | H | H | 2-CH₃ | H | H | H | H | -CH₂- | Phenyl |
| **19** | -C≡C-CH₃ | H | H | 4-F | H | H | H | H | -CH₂- | Phenyl |
| **20** | -C≡C-CH₃ | H | H | 3-F | H | H | H | H | -CH₂- | Phenyl |
| **21** | -C≡C-CH₃ | H | H | 2-F | H | H | H | H | -CH₂- | Phenyl |
| **22** | -C≡C-CH₃ | H | H | 3-Cl | 4-F | H | H | H | -CH₂- | Phenyl |
| **23** | -C≡C-CH₃ | H | H | 2-CH₃ | 4-F | H | H | H | -CHF₂- | Phenyl |
| **24** | -C≡C-CH₃ | H | H | 3-CH₃ | 4-F | H | H | H | -CH₂- | Phenyl |
| **25** | -C≡C-CH₃ | H | H | 2-Cl | 4-F | H | H | H | -CH₂- | Phenyl |
| **26** | -C≡C-CH₃ | H | H | 3-CF₃ | H | H | H | H | -CH₂- | Phenyl |
| **27** | -C≡C-CH₃ | H | H | H | H | H | H | H | -CH₂- | 2-Pyridyl |
| **28** | -OCH₃ | H | H | 3-Cl | 5-Cl | H | H | H | -CH₂- | 2-Pyridyl |
| **29** | -OCH₃ | H | H | H | H | H | H | H | -CH₂- | Phenyl |
| **30** | -OCH₃ | H | H | 4-F | H | H | H | H | -CH₂- | Phenyl |
| **31** | -OCH₃ | H | H | 3-F | H | H | H | H | -CH₂- | Phenyl |
| **32** | -C≡C-CH₃ | H | H | 3-Cl | 5-Cl | H | H | H | -CH₂- | 2-Pyridyl |
| **33** | -C≡C-CH₃ | H | H | 6-CF₃ | H | H | H | H | -CH₂- | 2-Pyridyl |
| **34** | -C≡C-CH₃ | H | H | 5-F | H | H | H | H | -CH₂- | 2-Pyridyl |
| **35** | -C≡C-CH₃ | H | H | 2-CF₃ | H | H | H | H | -CH₂- | Phenyl |
| **36** | -C≡C-CH₃ | H | H | 3-Cl | 5-CF₃ | H | H | H | -CH₂- | 2-Pyridyl |
| **37** | -C≡C-CH₃ | H | H | 6-F | H | H | H | H | -CH₂- | 2-Pyridyl |

Die Verbindungen wurden durch LC/MS wie folgt näher charakterisiert:
LC/MS Methoden

### Methode 1:

| | |
|---|---|
| Säule: | Waters UPLC BEH C18 2,1*50 mm; 1.7 µm |
| Lösungsmittel: | H2O+0.1% FA:AcN+0.08% FA |
| Gradient: | 95:5 (0min) to 5:95 (1.1 min) to 5:95 (1.7 min) to 95:5 (1.8 min) to 95:5 (2 min) |
| Flow, Temperature: | 0.9 ml/min 55°C |

### Methode 2:

| | |
|---|---|
| Säule: | Waters XBridge C18 4.6*50 mm; 2,5 µm |
| Lösungsmittel: | H2O+0.1% FA:AcN+0.1% FA |
| Gradient: | 97:3 (0 min) to 40:60 (3.5 min) to 2:98 (4 min) to 2:98 (5 min) to 97:3 (5.2 min) to 97:3 (6.5 min) |
| Flow, Temperature: | 1,3 ml/min 45°C |

### Methode 3:

| | |
|---|---|
| Säule: | Waters UPLC BEH C18 2,1 *50 mm; 1.7 µm |
| Lösungsmittel: | H2O+0.05% FA:AcN+0.035% FA |
| Gradient: | 95:5 (0 min) to 5:95 (1.1 min) to 5:95 (1.7 min) to 95:5 (1.9 min) to 95:5 (2 min) |
| Flow, Temperature: | 0.9 ml/min 55°C |

### Methode 4:

| | |
|---|---|
| Säüle: | Waters UPLC BEH C18 2,1*50 mm; 1.7 µm |
| Lösungsmittel: | H2O+0.05% FA:AcN+0.035% FA |
| Gradient: | 95:5 (0 min) to 5:95 (1.1 min) to 5:95 (1.7 min) to 95:5 (1.8 min) to 95:5 (2 min) |
| Flow, Temperatur: | 0.9 ml/min 55°C |

**Tabelle 3:**

| Bsp. | MW [g/mol] | LCMS Methode | Retenti onzeit [Min] | Ioni-sierung | Gemessene Masse [m/e] | Gemessenes Ion | Formel |
|---|---|---|---|---|---|---|---|
| 1 | 420,55 | 1 | ES- | 419.55 | [M-H]- | 1,4 | C27H32O4 |
| 2 | 432,44 | 1 | ES- | 431.37 | [M-H]- | 1,38 | C24H23F3O4 |
| 3 | 398,89 | 1 | ES- | 397.38 | [M-H]- | 1,4 | C23H23ClO4 |
| 4 | 482,88 | 1 | ES- | 481.32 | [M-H]- | 1,35 | C24H22ClF3O5 |
| 5 | 434,58 | 1 | ES- | 433.42 | [M-H]- | 1,5 | C28H34O4 |
| 6 | 446,46 | 1 | ES- | 445.32 | [M-H]- | 1,45 | C25H25F3O4 |
| 7 | 392,49 | 1 | ES+ | 393.15 | [M+H]+ | 1,44 | C25H28O4 |
| 8 | 378,47 | 1 | ES- | 377.31 | [M-H]- | 1,41 | C24H26O4 |
| 9 | 500,43 | 1 | ES+ | 499.4 | [M-H]- | 1,43 | C25H22F6O4 |
| 10 | 364,44 | 1 | ES- | 363.25 | [M-H]- | 1,35 | C23H24O4 |
| 11 | 378,47 | 1 | ES- | 377.31 | [M-H]- | 1,38 | C24H26O4 |
| 12 | 423,90 | 1 | ES+ | 422.31 | [M-H]- | 1,29 | C24H22ClNO4 |
| 13 | 433,43 | 1 | ES+ | 434.29 | [M+H]+ | 1,32 | C23H22F3NO4 |
| 14 | 433,43 | 1 | ES+ | 434.12 | [M+H]+ | 1,36 | C23H22F3NO4 |
| 15 | 466,88 | 2 | ES- | 465.21 | [M-H]- | 4,98 | C24H22ClF3O4 |
| 16 | 425,40 | 1 | ES+ | 426.21 | [M+H]+ | 1,34 | C21H22F3NO5 |
| 17 | 425,40 | 1 | ES+ | 426.25 | [M+H]+ | 1,28 | C21H22F3NO5 |
| 18 | 378,47 | 1 | ES+ | 401.23 | [M+Na]+ | 1,39 | C24H26O4 |
| 19 | 382,43 | 3 | ES- | 381.19 | [M-H]- | 1,22 | C23H23FO4 |
| 20 | 382,43 | 3 | ES- | 381.19 | [M-H]- | 1,23 | C23H23FO4 |
| 21 | 382,43 | 3 | ES- | 381.18 | [M-H]- | 1,21 | C23H23FO4 |
| 22 | 416,88 | 4 | ES- | 415.25 | [M-H]- | 1,25 | C23H22ClFO4 |
| 23 | 396,46 | 1 | ES+ | 397.3 | [M+H]+ | 1,38 | C24H25FO4 |
| 24 | 396,46 | 1 | ES+ | 397.01 | [M+H]+ | 1,38 | C24H25FO4 |
| 25 | 416,88 | 4 | ES- | 415.22 | [M-H]- | 1,24 | C23H22ClFO4 |
| 26 | 432,44 | 4 | ES- | 431.35 | [M-H]- | 1,26 | C24H23F3O4 |
| 27 | 365,43 | 2 | ES+ | 366.3 | [M+H]+ | 4,57 | C22H23NO4 |
| 28 | 426,30 | 4 | ES- | 424.21 | [M-H]- | 1,25 | C20H21Cl2NO5 |
| 29 | 356,42 | 4 | ES- | 355.19 | [M-H]- | 1,18 | C21H24O5 |
| 30 | 374,41 | 4 | ES- | 373.16 | [M-H]- | 1,18 | C21H23FO5 |
| 31 | 374,41 | 4 | ES- | 373.2 | [M-H]- | 1,19 | C21H23FO5 |
| 32 | 434,32 | 4 | ES+ | 434.25 | [M+H]+ | 1,12 | C22H21Cl2NO4 |
| 33 | 433,43 | 4 | ES+ | 434.19 | [M+H]+ | 1,24 | C23H22F3NO4 |
| 34 | 383,42 | 4 | ES+ | 384.17 | [M+H]+ | 1,19 | C22H22FNO4 |
| 35 | 432,44 | 1 | ES- | 431.4 | [M-H]- | 1,37 | C24H23F3O4 |
| 36 | 467,87 | 4 | ES+ | 468.2 | [M+H]+ | 1,15 | C23H21ClF3NO4 |
| 37 | 383,42 | 4 | ES+ | 384.23 | [M+H]+ | 1,19 | C22H22FNO4 |

Die Wirksamkeit der Verbindungen wurde wie folgt getestet:
In-vitro FLIPR-Assay mit rekombinanten Zellen, die den GPCR GPR40 (Ratte) exprimieren Funktionsüberprüfende Assays wurden mittels der FLIPR-Technik ("Fluorescence Imaging Plate Reader", Molecular Devices Corp.) durchgeführt. Hierzu wurden agonist-induzierte Änderungen der intrazellulären Konzentration von Ca²⁺ in rekombinanten HEK293 Zellen bestimmt, die den GPCR GPR40 (Ratte) exprimierten.
   Für die Untersuchungen wurden Zellen in 96-well-Mikrotiterplatten (60000 Zellen/well) ausgesät und über Nacht wachsen gelassen. Das Medium wurde entfernt und die Zellen in Puffer inkubiert, der den Fluoreszenzfarbstoff Fluo-4 enthielt. Nach dieser Beladung mit Farbstoff wurden die Zellen gewaschen, Testsubstanz zugegeben und Änderungen in der intrazellulären Ca²⁺-Konzentration im FLIPR-Gerät gemessen. Ergebnisse wurden als prozentuale Änderung relativ zur Kontrolle dargestellt (0 %: keine Testsubstanz addiert; 100 %: 10 µM Referenzagonist Linolsäure addiert), zur Berechnung von Dosis/Wirkungskurven verwendet und EC₅₀-Werte bestimmt.

**Tabelle 2: Biologische Aktivität**

| **Beispiel** | **EC₅₀ [µM] (Ratte GPR40)** |
|---|---|
| **1** | 0,73 |
| **2** | 0,37 |
| **3** | 0,29 |
| **4** | 2,55 |
| **5** | 2,56 |
| **6** | 1,32 |
| **7** | 1,54 |
| **8** | 0,45 |
| **9** | 1,66 |
| **10** | 0,07 |
| **11** | 0,15 |
| **12** | 0,27 |
| **13** | 0,71 |
| **14** | 0,17 |
| **15** | 1,58 |
| **16** | 2,46 |
| **17** | 52,00 |
| **18** | 0,06 |
| **19** | 0,07 |
| **20** | 0,07 |
| **21** | 0,11 |
| **22** | 0,34 |
| **23** | 0,44 |
| **24** | 0,28 |
| **25** | 0,47 |
| **26** | 0,46 |
| **27** | 0,67 |
| **28** | 2,97 |
| **29** | 11,40 |
| **30** | 1,53 |
| **31** | 4,62 |
| **32** | 1,08 |
| **33** | 0,23 |
| **34** | 0,34 |
| **35** | 0,55 |
| **36** | 1,08 |
| **37** | 0,20 |

Aus der Tabelle ist abzulesen, dass die Verbindungen der Formel I den GPR40 Rezeptor aktivieren und dadurch gut zur Behandlung von Hyperglykämie und von Diabetes geeignet sind. Durch die Verbindungen der Formel I wird die Insulinausschüttung erhöht (siehe Itoh et al., Nature 2003, 422, 173-176).

Aufgrund der Aktivierung des GPR40 Rezeptors können die Verbindungen der Formel I auch zur Behandlung bzw. Prävention weiterer Krankheiten angewendet werden.
Die Verbindungen der vorliegenden Erfindung eignen sich insbesondere zur Behandlung und/oder Prävention von:
1. - Störungen des Fettsäurestoffwechsels und Glucoseverwertungsstörungen
   - Störungen, bei denen Insulinresistenz eine Rolle spielt
2. Diabetes mellitus, insbesondere Typ-2-Diabetes, einschließlich der Prävention der damit verbundenen Folgeerkrankungen.
   - Besondere Aspekte in diesem Zusammenhang sind
   - Hyperglykämia,
   - Verbesserung der Insulinresistenz,
   - Verbesserung der Glucosetoleranz,
   - Schutz der β-Zellen der Bauchspeicheldrüse
   - Prävention makro- und mikrovaskulärer Erkrankungen
3. Verschiedene andere Leiden, die mit dem metabolischen Syndrom bzw. Syndrom X assoziiert sein können, wie
   - Obesitas (erhöhter body mass index -BMI)
      Zunahme des Bauchumfangs (viscerale Adipositas)
   - Fettleber (non-alcoholic fatty liver disease (NAFLD) und NASH)
   - Dyslipidämie (z.B. Hypertriglyceridämie und/oder niedriges HDL)
   - Insulinresistenz
   - Hyperkoagulabilität
   - Hyperurikämie
   - Mikroalbuminämie
   - Thrombosen, hyperkoagulabile und prothrombotische Zustände (arteriell und venös)
   - Bluthochdruck
   - Herzinsuffizienz, beispielsweise (jedoch nicht darauf beschränkt), nach Herzinfarkt, hypertensiver Herzkrankheit oder Kardiomyopathie
4. Gedächtnisstörungen, geistige Defekte, ZNS-Erkrankungen wie
   - Altersdemenzen
   - Alzheimer'schen Krankheit
   - Behandlung verminderter Aufmerksamkeit oder Wachsamkeit
   - Schizophrenie
5. Gastrointestinale (GI) Störungen
   - GI-Dyskinesien (Reizdarmsyndrom = Irritables Darmsyndrom (IDS) bzw. Irritable Bowel Syndrome (IBS), Reizkolon, Colon irritabile und "nervöser Darm")

Die verwendeten Abkürzungen stehen für:
- Ac: Acetyl
- Bn: Benzyl
- iBu: Isobutyl
- tBu: tert-Butyl
- BuLi: n-Butyllithium
- DC: Dünnschichtchromatographie
- DEAD: Diethylazodicarboxylat
- DCI: direkte chemische Ionisation (bei MS)
- DCM: Dichlormethan
- DMAP: 4-N,N-Dimethylaminopyridin
- DMF: N,N-Dimethylformamid
- DMSO: Dimethylsulfoxid
- EE: Essigsäureethylester
- ent: Enantiomer / enantiomerenrein
- EI: Elektronenstoß-Ionisation (bei MS)
- eq: Äquivalent
- ESI: Elektronenspray-Ionisation (bei MS)
- FG: Funktionelle Gruppe
- Hal: Halogen
- HPLC: Hochdruck-, Hochleistungsflüssigchromatographie
- LC-MS: Flüssigchromatographie-gekoppelte Massenspektroskopie
- m: meta
- Me: Methyl
- MeOH: Methanol
- MS: Massenspektroskopie
- Ms: Mesyl
- NMR: Kernresonanzspektroskopie
- o: ortho
- p: para
- Pd/C: Palladium auf Kohle
- iPr: Isopropyl
- nPr: n-Propyl
- rac: Racemisch / racemisches Gemisch
- Rf: Retentionszeit (bei DC)
- RP: Reverse phase
- THF: Tetrahydrofuran
- Ts: Tosyl
- FA: Ameisensäure

### Experimenteller Teil:

Beispielsynthesen nach Verfahren A.

### Beispiel 1

### 3- {4-[1-(3-tert-Butyl-phenoxymethyl)-cyclopropylmethoxy]-phenyl}-hex-4-ynoylsäure

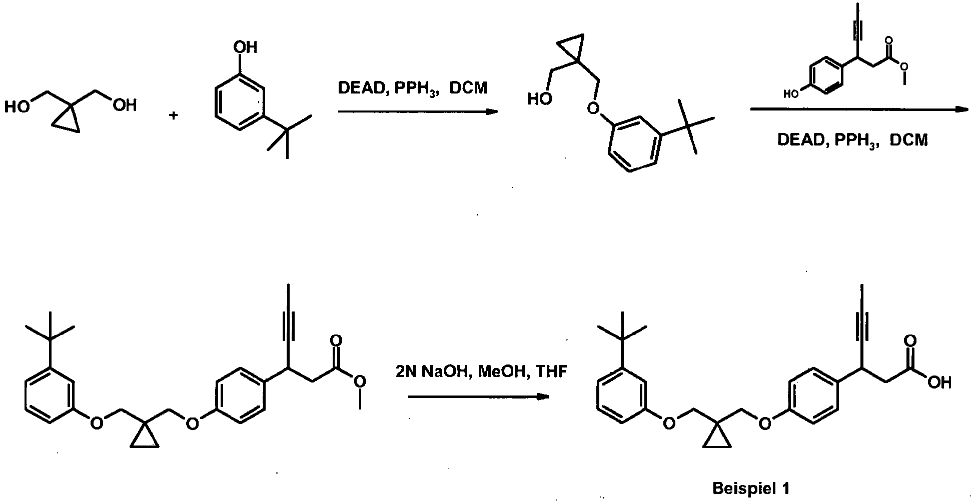

### 1-(3-tert-Butyl-phenoxymethyl)-cyclopropyl]-methanol

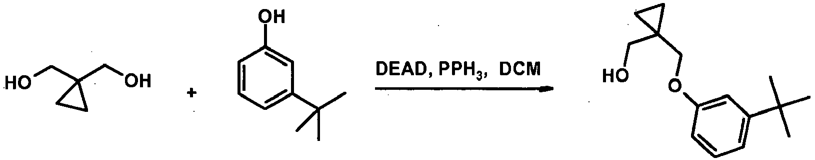

0.96 ml 1,1-Bis(hydroxymethylcyclopropan), 1.0 g 3-tert.-Butylphenol und 1.75g Triphenylphosphin wurden in 100 ml Dichlormethan gelöst. Unter Eiskühlung wurden 0.88 ml Diethylazodicarboxylat zugetropft. Das Eisbad wurde entfernt und das Reaktionsgemisch 4 Stunden bei Raumtemperatur gerührt. Dann wurden vorsichtig 5 ml Wasser zugegeben und das Reaktionsgemisch im Vakuum eingeengt. Der Rückstand wurde in 50 ml Wasser und 50 ml Essigsäuretehylester aufgenommen. Die organische Phase wurde abgetrennt, über MgSO₄ getrocknet und anschließend im Vakuum eingeengt. Der Rückstand wurde an Kiesegel mit dem Lösungsmittelgemisch n-Heptan/Essigsäureethylester als linearer Gradient 100 % n-Heptan => 100% Essigsäureethylester gereinigt. Man erhielt 580 mg 1-(3-tert-Butyl-phenoxymethyl)-cyclopropyl]-methanol.
C₁₅H₂₂O₂ (234.34), LCMS(ESI-pos): 217.2 (M-H₂0+H⁺).

### 3-{4-[1-(3-tert-Butyl-phenoxymethyl)-cyclopropylmethoxy]-phenyl}-hex-4-ynoylsäure methylester

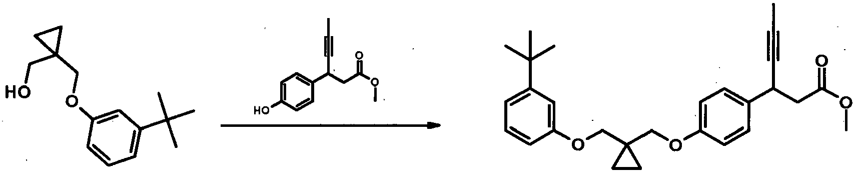

537 mg 1-(3-tert-Butyl-phenoxymethyl)-cyclopropyl]-methanol, 500 mg 3-(4-Hydroxyphenyl)-hex-4-ynoylsäuremethylester und 600 mg Triphenylphosphin wurden in 80 ml Dichlormethan gelöst. Unter Eiskühlung wurden 0.31 ml Diethylazodicarboxylat zugetropft. Das Eisbad wurde entfernt und das Reaktionsgemisch 24 Stunden bei Raumtemperatur gerührt. Dann wurden vorsichtig 5 ml Wasser zugegeben und das Reaktionsgemisch im Vakuum eingeengt. Der Rückstand wurde in 50 ml Wasser und 50 ml Essigsäuretehylester aufgenommen. Die organische Phase wurde abgetrennt, über MgSO₄ getrocknet und anschließend im Vakuum eingeengt. Der Rückstand wurde an Kiesegel mit dem Lösungsmittelgemisch n-Heptan/Essigsäureethylester als linearer Gradient 100 % n-Heptan => 100% Essigsäureethylester gereinigt. Man erhielt 410 mg 3-{4-[1-(3-tert-Butylphenoxymethyl)-cyclopropylmethoxy]-phenyl}-hex-4-ynoylsäuremethylester.
C₂₈H₃₄O₄ (434.58), LCMS(ESI-pos): 435.3 (M+H⁺).

### 3-{4-[1-(3-tert-Butyl-phenoxymethyl)-cyclopropylmethoxy]-phenyl}-hex-4-ynoylsäure

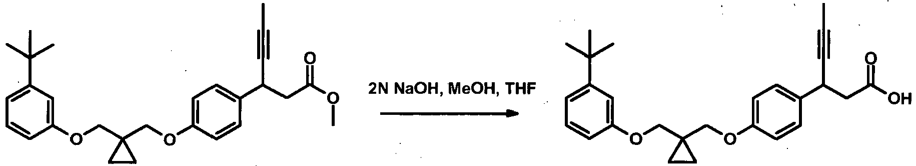

410 mg 3-{4-[1-(3-tert-Butyl-phenoxymethyl)-cyclopropylmethoxy]-phenyl}-hex-4-ynoylsäuremethylester wurde in einem Gemisch aus THF/MeOH/2N NaOH = 1:1:1 (je 5ml) gelöst und bei Raumtemperatur gerührt. Nach 45 min wurde durch Zugabe von 4N HCl auf pH1 angesäuert. Es wurden 50 ml Wasser zugegeben und dreimal mit je 50 ml Essigsäureethylester extrahiert. Die vereinigten organischen Phasen über MgSO₄ getrocknet und anschließend im Vakuum eingeengt. Der Rückstand wurde über RP-HPLC gereinigt. Man erhielt 148 mg 3-{4-[1-(3-tert-Butyl-phenoxymethyl)-cyclopropylmethoxy]-phenyl}-hex-4-ynoylsäure.

### Beispiel 2

### 3-{4-[1-(4-Trifluoromethyl-phenoxymethyl)-cyclopropylmethoxy]-phenyl}-hex-4-ynoylsäure

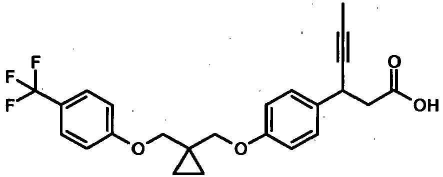

Analog zu Beispiel 1 erhielt man aus 1,1-Bis(hydroxymethylcyclopropan), 4-Hydroxybenzotrifluorid und 3-(4-Hydroxy-phenyl)-hex-4-ynoylsäuremethylester 3-{4-[1-(4-Trifluoromethyl-phenoxymethyl)-cyclopropylmethoxy]-phenyl}-hex-4-ynoylsäure.

### Beispiel 3

### 3-{4-[1-(3-Chloro-phenoxymethyl)-cyclopropylmethoxy]-phenyl}-hex-4-ynoylsäure

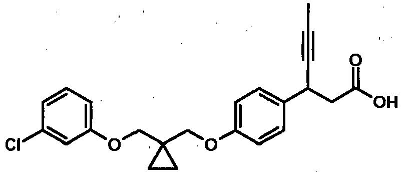

Analog zu Beispiel 1 erhielt man aus 1,1-Bis(hydroxymethylcyclopropan), 3-Chlorphenol und 3-(4-Hydroxy-phenyl)-hex-4-ynoylsäuremethylester 3-{4-[1-(3-Chloro-phenoxymethyl)-cyclopropylmethoxy]-phenyl}-hex-4-ynoylsäure.

### Beispiel 4

### 3-{4-[3-(2-Chloro-4-trifluoromethyl-phenoxymethyl)-oxetan-3-ylmethoxy]-phenyl}-hex-4-ynoylsäure

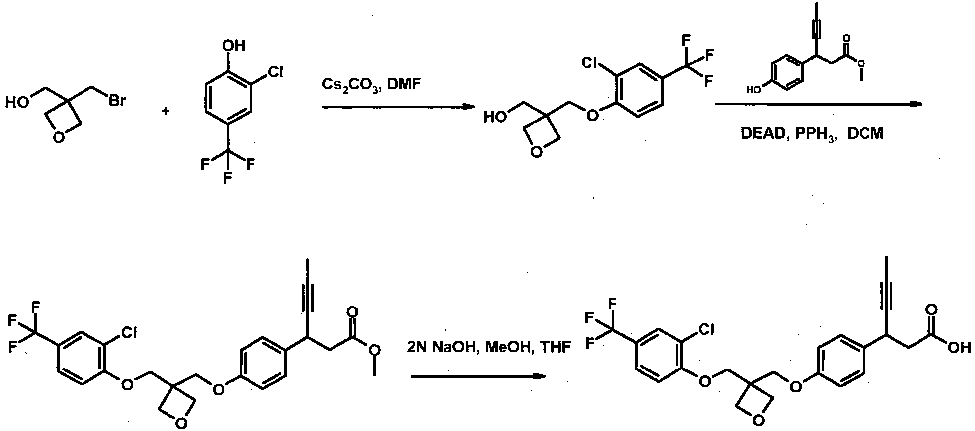

### [3-(2-Chloro-4-trifluoromethyl-phenoxymethyl)-oxetan-3-yl]-methanol

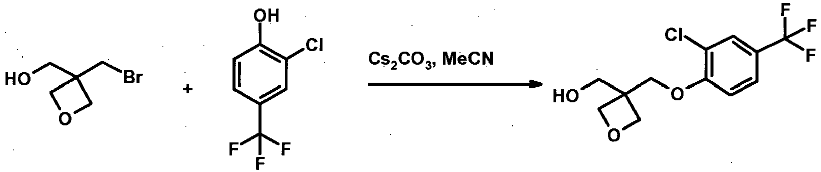

600 mg 3-Chloro-4-hydroxy-benzotrifluorid und 829 mg 3-Bromomethyl-3-oxetanmethanol wurden in 20 ml Acetonitril gelöst und mit 1.49 g Cäsiumcarbonat versetzt. Das Reaktionsgemisch wurde auf 110°C erhitzt und drei Stunden bei dieser Temperatur gerührt. Zum abgekühlten Reaktuionsgemisch wurden 50 ml Wasser und 50 ml Essigsäuretehylester hinzugefügt. Die organische Phase wurde abgetrennt, über MgSO₄ getrocknet und anschließend im Vakuum eingeengt. Der Rückstand wurde an Kiesegel mit dem Lösungsmittelgemisch n-Heptan/Essigsäureethylester als linearer Gradient 100 % n-Heptan => 100% Essigsäureethylester gereinigt. Man erhielt 990 mg [3-(2-Chloro-4-trifluoromethyl-phenoxymethyl)-oxetan-3-yl]-methanol.
C₁₂H₁₂ClF₃O₃. (296.68), LCMS(ESI-neg): 341.0 (M+ HCOO).

### 3-{4-[3-(2-Chloro-4-trifluoromethyl-phenoxymethyl)-oxetan-3-ylmethoxy]-phenyl}-hex-4-ynoylsäure

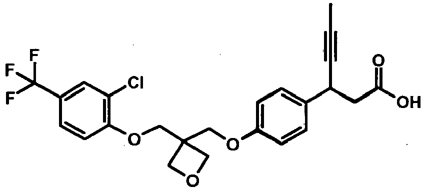

Analog zu Beispiel 1 erhielt man aus [3-(2-Chloro-4-trifluoromethyl-phenoxymethyl)-oxetan-3-yl]-methanol und 3-(4-Hydroxy-phenyl)-hex-4-ynoylsäuremethylester 3-{4-[3-(2-Chloro-4-trifluoromethyl-phenoxymethyl)-oxetan-3-ylmethoxy]-phenyl}-hex-4-ynoylsäure.

### Beispiel 5

### 3-{4-[1-(3-tert-Butyl-phenoxymethyl)-cyclobutylmethoxy]-phenyl}-hex-4-ynoylsäure

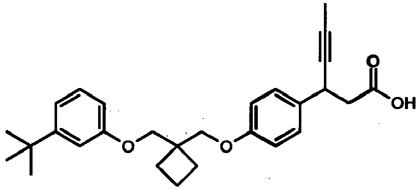

Analog zu Beispiel 1 erhielt man aus Cyclobutan-1,1-diyldimethanol, 3-tert.-Butylphenol und 3-(4-Hydroxy-phenyl)-hex-4-ynoylsäuremethylester 3-{4-[1-(3-tert-Butyl-phenoxymethyl)-cyclobutylmethoxy]-phenyl}-hex-4-ynoylsäure.

### Beispiel 6

### 3-{4-[1-(4-Trifluoromethyl-phenoxymethyl)-cyclobutylmethoxy]-phenyl}-hex-4-ynoylsäure

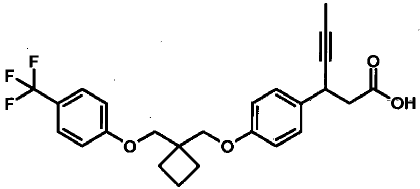

Analog zu Beispiel 1 erhielt man aus Cyclobutan-1,1-diyldimethanol, 4-Hydroxybenzotrifluorid und 3-(4-Hydroxy-phenyl)-hex-4-ynoylsäuremethylester 3- {4-[1-(4-Trifluoromethyl-phenoxymethyl)-cyclobutylmethoxy]-phenyl}-hex-4-ynoylsäure.

### Beispiel 7

### 3-[4-(1-m-Tolyloxymethyl-cyclobutylmethoxy)-phenyl]-hex-4-ynoylsäure

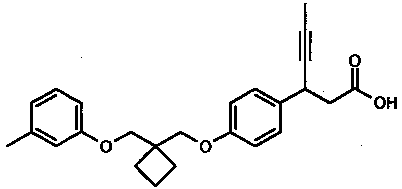

Analog zu Beispiel 1 erhielt man aus Cyclobutan-1,1-diyldimethanol, 3-Methylphenol und 3-(4-Hydroxy-phenyl)-hex-4-ynoylsäuremethylester 3-[4-(1-m-Tolyloxymethyl-cyclobutyl methoxy)-phenyl]-hex-4-ynoylsäure.

### Beispiel 8

### 3-[4-(1-Phenoxymethyl-cyclobutylmethoxy)-phenyl]-hex-4-ynoylsäure

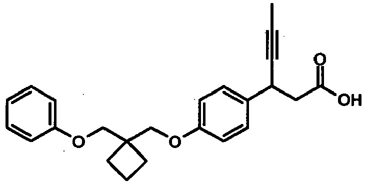

Analog zu Beispiel 1 erhielt man aus Cyclobutan-1,1-diyldimethanol, Phenol und 3-(4-Hydroxy-phenyl)-hex-4-ynoylsäuremethylester 3-[4-(1-Phenoxymethyl-cyclobutylmethoxy)-phenyl]-hex-4-ynoylsäure.

### Beispiel 9

### 3- {4-[1-(3,5-Bis-trifluoromethyl-phenoxymethyl)-cyclopropylmethoxy]-phenyl}-hex-4-ynoylsäure

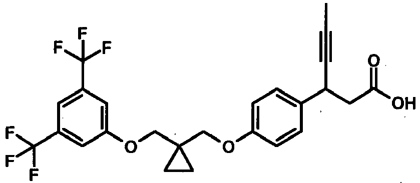

Analog zu Beispiel 1 erhielt man aus 1,1-Bis(hydroxymethylcyclopropan), 3,5-Bistrifluoromethyl-phenol und 3-(4-Hydroxy-phenyl)-hex-4-ynoylsäuremethylester 3-{4-[1-(3,5-Bis-trifluoromethyl-phenoxymethyl)-cyclopropylmethoxy]-phenyl}-hex-4-ynoylsäure.

### Beispiel 10

### 3-[4-(1-Phenoxymethyl-cyclopropylmethoxy)-phenyl]-hex-4-ynoylsäure

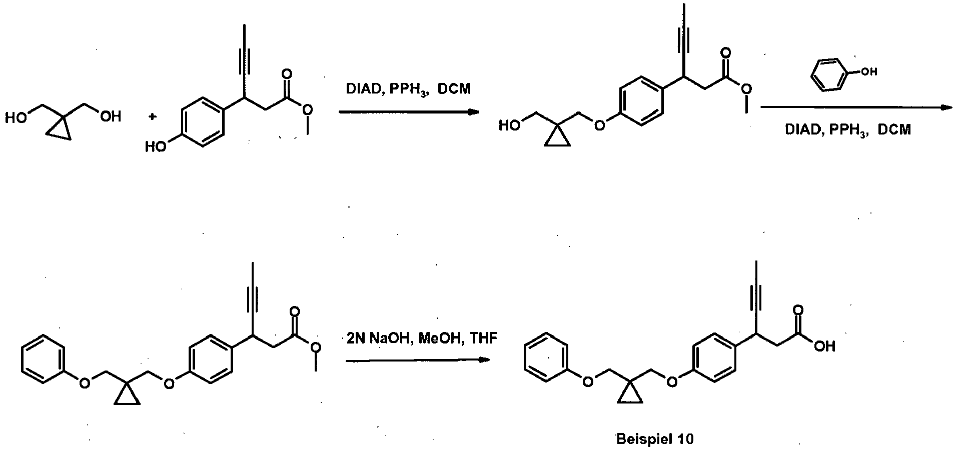

### 3-[4-(1-Hydroxymethyl-cyclopropylmethoxy)-phenyl]-hex-4-ynoylsäuremethylester

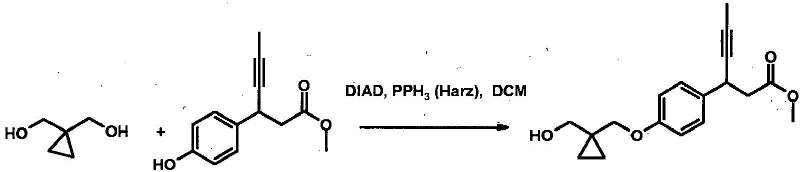

300 mg 3-(4-Hydroxy-phenyl)-hex-4-ynoylsäuremethylester wurden in 10 ml DCM gelöst und in einem Mirkowellen-Gefäß vorgelegt, dann wurden 234 mg 1,1-Bis(hydroxymethylcyclopropan) und 541 mg Triphenylphosphin (polymergebunden, Reagentplus 99%) zugegeben und zum Quellen des Harzes 10 min nachgerührt. Anschließend wurden 0.41 ml Diisopropylazodicarboxylat zugegeben und 15 min bei 120 °C unter Mikrowelleneinstrahlung gerührt. Das abgekühlte Reaktionsgemisch wurde filtriert und der Filterkuchen 3 x mit je 10 ml DCM nachgewaschen. Die vereinigten Filtrate wurden mit 30 ml Wasser gewaschen, danach über eine ChemElut-Kartusche getrocknet und im Vakuum eingeengt. Der Rückstand wurde mittels RP-HPLC gereinigt. Man erhielt 200 mg 3-[4-(1-Hydroxymethyl-cyclopropylmethoxy)-phenyl]-hex-4-ynoylsäuremethylester.
C₁₈H₂₂O₄. (302.37), LCMS(ESI-pos): 285.1 (M-H₂O+H⁺).

### 3-[4-(1-Phenoxymethyl-cyclopropylmethoxy)-phenyl]-hex-4-ynoylsäuremethylester

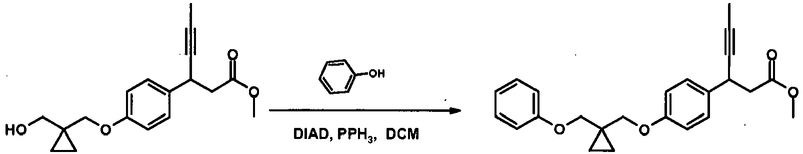

100 mg 3-[4-(1-Hydroxymethyl-cyclopropylmethoxy)-phenyl]-hex-4-ynoylsäuremethylester wurden in 10 ml DCM gelöst und in einem Mikrowellen-Gefäß vorgelegt, dann wurden 46.7 mg Phenol und 130 mg Triphenylphosphin (polymergebunden, Reagentplus 99%) zugegeben und zum Quellen des Harzes 10 min nachgerührt. Anschließend wurden 98 µl Diisopropylazodicarboxylat zugegeben und 15 min bei 120 °C unter Mikrowelleneinstrahlung gerührt. Das abgekühlte Reaktionsgemisch wurde filtriert und der Filterkuchen 3 x mit je 10 ml DCM nachgewaschen. Die vereinigten Filtrate wurden mit 30 ml Wasser gewaschen, danach über eine ChemElut-Kartusche getrocknet und im Vakuum eingeengt. Der Rückstand wurde mittels RP-HPLC gereinigt. Man erhielt 68 mg 3-[4-(1-Phenoxymethyl-cyclopropylmethoxy)-phenyl]-hex-4-ynoylsäuremethylester.
C₂₄H₂₆O₄. (378.47), LCMS(ESI-pos): 396.2 (M+NH₄⁺).

### 3-[4-(1-Phenoxymethyl-cyclopropylmethoxy)-phenyl]-hex-4-ynoylsäure

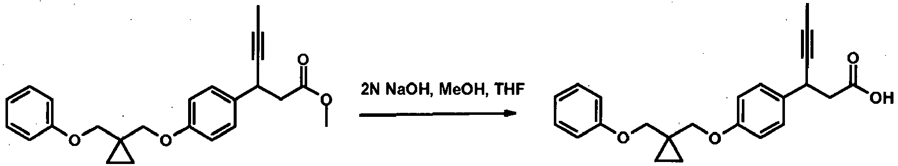

Analog zu Beispiel 1 erhielt man aus 3-[4-(1-Phenoxymethyl-cyclopropylmethoxy)-phenyl]-hex-4-ynoylsäuremethylester 3-[4-(1-Phenoxymethyl-cyclopropylmethoxy)-phenyl]-hex-4-ynoylsäure.

### Beispiel 11

### 3-[4-(1-m-Tolyloxymethyl-cyclopropylmethoxy)-phenyl]-hex-4-ynoylsäure

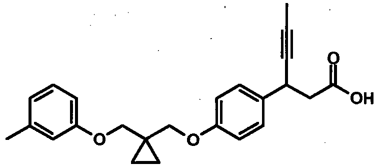

Analog zu Beispiel 10 erhielt man aus 1,1-Bis(hydroxymethylcyclopropan), 3-Methylphenol und 3-(4-Hydroxy-phenyl)-hex-4-ynoylsäuremethylester 3-[4-(1-m-Tolyloxymethyl-cyclopropylmethoxy)-phenyl]-hex-4-ynoylsäure.

### Beispiel 12

### 3-{4-[1-(3-Chloro-4-cyano-phenoxymethyl)-cyclopropylmethoxy]-phenyl} -hex-4-ynoylsäure

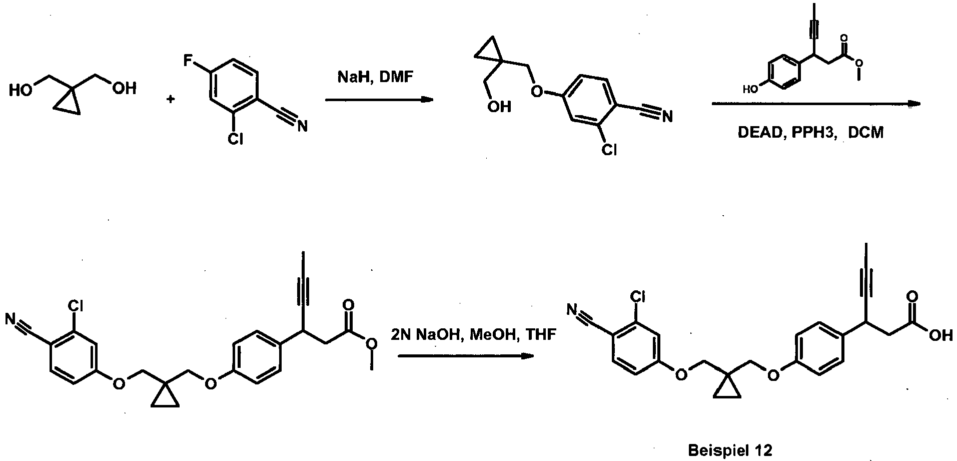

### 2-Chloro-4-(1-hydroxymethyl-cyclopropylmethoxy)-benzonitrile

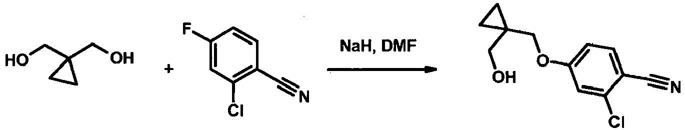

15.0 g 1,1-Bis(hydroxymethylcyclopropan) und 7.4 g 2-Chloro-4-fluorobenzonitril wurden in werden in 350 ml N-Methylpyttolidon gelöst und unter Eiskühlung wurden 1.26 g NaH portionsweise eingetragen. Nach 30 Minuten wurde das Kältebad entfernt und das Reaktionsgemisch 2 Stunden bei raumtemperatur gerüht. Dann wurde durch vorsichtige Zugabe von 250 ml Wasser das Reaktionsgemisch abgelöscht und dreimal mit Portionen zu je 200 ml Essigsäureethylester extrahiert. Die vereinigten organischen Phasen wurden über MgSO₄ getrocknet und anschließend im Vakuum eingeengt. Der Rückstand wurde an Kiesegel mit dem Lösungsmittelgemisch n-Heptan/Essigsäureethylester als linearer Gradient 100 % n-Heptan => 50% n-Heptan + 50% Essigsäureethylester gereinigt. Man erhielt 10.5 g 2-Chloro-4-(1-hydroxymethyl-cyclopropylmethoxy)-benzonitrile.
C₁₂H₁₂ClNO₂ (237.69), LCMS(ESI-pos): 238.0 (M+H⁺); DC in EE:n-Heptan = 1:1; R_{f}= 0,29.

### 3-{4-[1-(3-Chloro-4-cyano-phenoxymethyl)-cyclopropylmethoxy]-phenyl}-hex-4-ynoylsäure

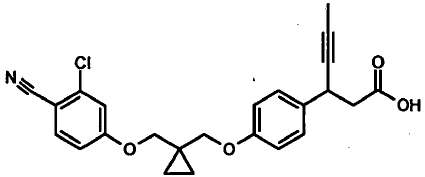

Analog zu Beispiel 1 erhielt man aus 2-Chloro-4-(1-hydroxymethyl-cyclopropylmethoxy)-benzonitrile und 3-(4-Hydroxy-phenyl)-hex-4-ynoylsäuremethylester 3-{4-[1-(3-Chloro-4-cyano-phenoxymethyl)-cyclopropylmethoxy]-phenyl}-hex-4-ynoylsäure.

### Beispiel 13

### 3-{4-[1-(6-Trifluoromethyl-pyridin-3-yloxymethyl)-cyclopropylmethoxy]-phenyl}-hex-4-ynoylsäure

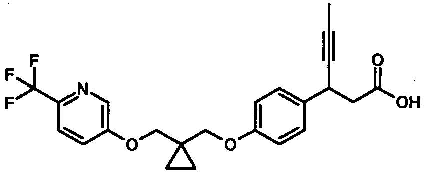

Analog zu Beispiel 12 und Beispiel 1 erhielt man aus 1,1-Bis(hydroxymethylcyclopropan), 5-Fluoro-2-(trifluoromethyl)pyridin und 3-(4-Hydroxy-phenyl)-hex-4-ynoylsäuremethylester 3-{4-[1-(6-Trifluoromethyl-pyridin-3-yloxymethyl)-cyclopropylmethoxy]-phenyl-hex-4-ynoylsäure.

### Beispiel 14

### 3-{4-[1-(5-Trifluoromethyl-pyridin-2-yloxymethyl)-cyclopropylmethoxy]-phenyl}-hex-4-ynoylsäure

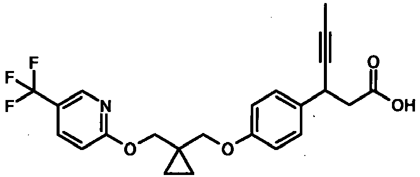

Analog zu Beispiel 12 und Beispiel 1 erhielt man aus 1,1-Bis(hydroxymethylcyclopropan), 2-Fluoro-5-(trifluoromethyl)pyridin und 3-(4-Hydroxy-phenyl)-hex-4-ynoylsäuremethylester 3-{4-[1-(5-Trifluoromethyl-pyridin-2-yloxymethyl)-cyclopropylmethoxy]-phenyl}-hex-4-ynoylsäure.

### Beispiel 15

### 3-{4-[1-(2-Chloro-4-trifluoromethyl-phenoxymethyl)-cyclopropylmethoxy]-phenyl}-hex-4-ynoylsäure

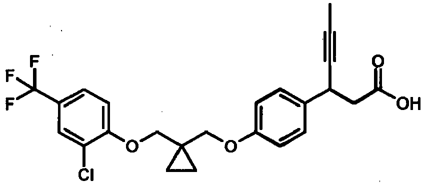

Analog zu Beispiel 12 und Beispiel 1 erhielt man aus 1,1-Bis(hydroxymethylcyclopropan), 3-Chloro-4-fluoro-benzotrifluorid und 3-(4-Hydroxy-phenyl)-hex-4-ynoylsäuremethylester 3-{4-[1-(2-Chloro-4-trifluoromethyl-phenoxymethyl)-cyclopropylmethoxy]-phenyl}-hex-4-ynoylsäure.

### Beispiel 16

### 3-Methoxy-3-{4-[1-(5-trifluoromethyl-pyridin-2-yloxymethyl)-cyclopropylmethoxy]-phenyl}-propionsäure

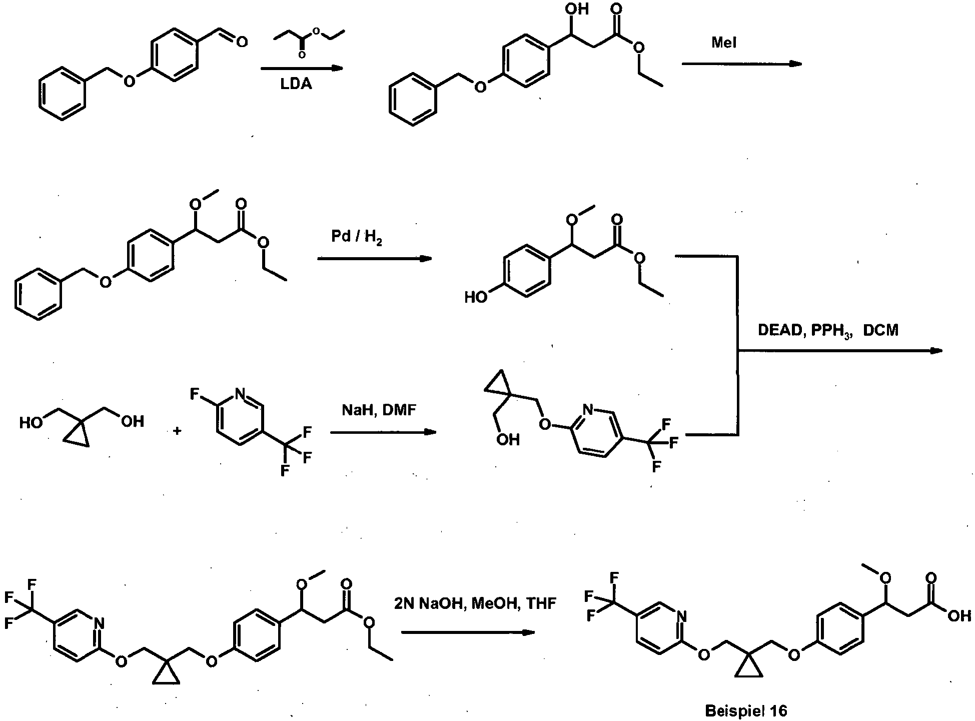

### 3-(4-Benzyloxy-phenyl)-3-hydroxy-propionic acid ethyl ester

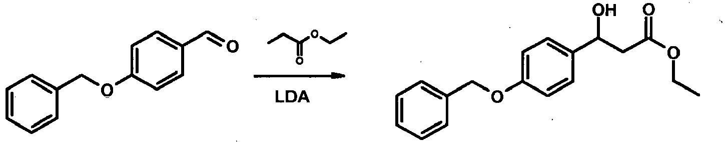

23.8 ml Diisopropylamin wurden unter Argon in 250ml THF vorgelegt, auf 0°C abgekühlt und mit 70 ml n-Butyllithiumlösung (2.5M in Toluol) versetzt. Die Mischung wurdefür 30 Minuten bei 0°C gerührt, dann auf -75°C abgkühlt und langsam mit 16.2 ml Ethylacetat vesetzt und für 30 Minuten nachgerührt. Dann wurden bei -75°C 30 g p-Benzyloxybenzaldehyd, gelöst in 250ml THF, zugetropft (Innentemp. max. -68°C; 1 h). Es wurde 20 Minuten nachgerührt und danach das Reaktionsgemisch mit 200ml ges. NH₄Cl-Lösung und 20 ml 1N HCl gequencht (Innentemp. max. -50°C). Man ließ auf Raumtemperatur auftauen, die Phasen wurden getrennt und die organische Phase dreimal mit je 100 ml ges. NH₄Cl-Lsg und einmal mit 100 ml ges. NaCl-Lsg. gewaschen. Das Lösungsmittel wurde im Vakuum entfernt. Der Rückstand wurde mit n-Heptan/2%THF ausgerührt und der ausgefallene Niederschlag abgesaugt und im Vakuum bei 40 °C getrocknet. Man erhielt 41 g 3-(4-Benzyloxy-phenyl)-3-hydroxy-propionsäureethylester als farblosen Feststoff.
C₁₈H₂₀O₄ (300.36), LCMS(ESI-pos): 283.1(M-H₂O+H⁺).

### 3-(4-Benzyloxy-phenyl)-3-methoxy-propionsäureethylester

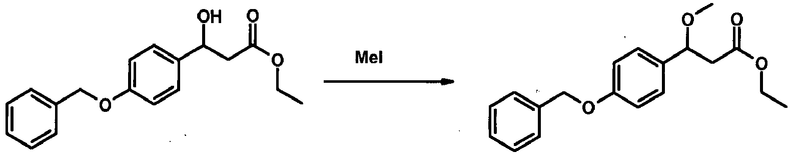

40 g 3-(4-Benzyloxy-phenyl)-3-hydroxy-propionsäureethylester, 75 g Silberoxid und 83 ml Iodmethan wurden in 350 ml Toluol vorgelegt und in einem geschlossenen Gefäß 24 Stunden bei 70°C Badtemperatur gerührt. Danach wurden weitere 20 ml Iodmethan zugeben und weitere 48 h bei 70°C Badtemperatur gerührt. Das Reaktionsgemisch wurde über eine kurze Kieselgelschicht filtriert, mit Toluol nachwaschen und anschließend das Toluol im Vakuum entfernt. Der Rückstand wurde an Kieselgel mit dem Eluens Essigsäureethylester : n-Heptan = 1:2 gereinigt. Man erhielt 22.9 g 3-(4-Benzyloxy-phenyl)-3-methoxy-propionsäureethylester. C₁₉H₂₂O₄ (314.38), LCMS(ESI-pos): 283.1 (M-CH₃OH+H⁺).

### 3-(4-Hydroxy-phenyl)-3-methoxy-propionsäureethylester

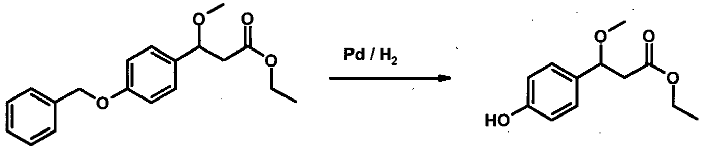

9.3g 3-(4-Benzyloxy-phenyl)-3-methoxy-propionsäureethylester wurden in 200 ml Ethanol gelöst und mit 1.1g Palladium auf Aktivkohle (10%) versetzt. Es wurde zwei Stundenunter einer Wasserstoffatmosphäre bei Raumtemperatur gerührt. Das Reaktionsgemisch wurde über einen Nylonmembranfilter (0,45 µm) filtriert, mit 200 ml Ethanol nachwaschen und das Filtrat im Vakuum eingeengt Man erhielt 6.7g 3-(4-Hydroxy-phenyl)-3-methoxy-propionsäureethylester. C₁₂H₁₆O₄ (224.26), LCMS(ESI-pos): 193.1(M-CH₃OH +H⁺). ¹H NMR (500 MHz, DMSO-d₆) 9.39 (s, 1H), 7.12 (d, 2H), 6.73 (d, 2H), 4.44 (dd, 1H), 4.02 (q, 2H), 3.03 (s, 3H), 2.68 (dd, 2H), 2.53 (dd, 2H), 1.14 (t, 3H).

### 3-Methoxy-3-{4-[1-(5-trifluoromethyl-pyridin-2-yloxymethyl)-cyclopropylmethoxy]-phenyl}-propionsäure

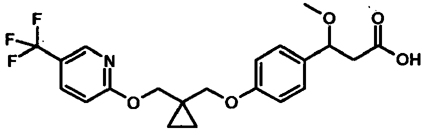

Analog zu Beispiel 12 und Beispiel 1 erhielt man aus 1,1-Bis(hydroxymethylcyclopropan), 2-Fluoro-5-(trifluoromethyl)pyridin und 3-(4-Hydroxy-phenyl)-3-methoxy-propionsäureethylester 3-Methoxy-3-{4-[1-(5-trifluoromethyl-pyridin-2-yloxymethyl)-cyclopropylmethoxy]-phenyl} -propionsäure.

### Beispiel 17

### 3-Methoxy-3-{4-[1-(6-trifluoromethyl-pyridin-3-yloxymethyl)-cyclopropylmethoxy]-phenyl}-propionsäure

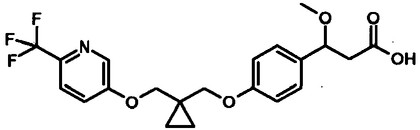

Analog zu Beispiel 16 und Beispiel 1 erhielt man aus 1,1-Bis(hydroxymethylcyclopropan), 5-Fluoro-2-(trifluoromethyl)pyridin und 3-(4-Hydroxy-phenyl)-3-methoxy-propionsäureethylester 3-Methoxy-3-{4-[1-(6-trifluoromethyl-pyridin-3-yloxymethyl)-cyclopropylmethoxy]-phenyl}-propionsäure.

### Beispiel 18

### 3-[4-(1-o-Tolyloxymethyl-cyclopropylmethoxy)-phenyl]-hex-4-ynoylsäure

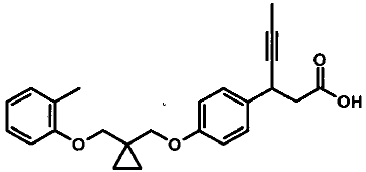

Analog zu Beispiel 10 erhielt man aus 1,1-Bis(hydroxymethylcyclopropan), 2-Methylphenol und 3-(4-Hydroxy-phenyl)-hex-4-ynoylsäuremethylester 3-[4-(1-o-Tolyloxymethyl-cyclopropylmethoxy)-phenyl]-hex-4-ynoylsäure.

### Beispiel 19

### 3-{4-[1-(4-Fluoro-phenoxymethyl)-cyclopropylmethoxy]-phenyl}-hex-4-ynoylsäure

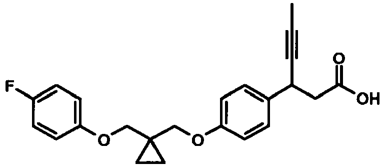

Analog zu Beispiel 10 erhielt man aus 1,1-Bis(hydroxymethylcyclopropan), 4-Fluorphenol und 3-(4-Hydroxy-phenyl)-hex-4-ynoylsäuremethylester 3-{4-[1-(4-Fluoro-phenoxymethyl)-cyclopropylmethoxy]-phenyl}-hex-4-ynoylsäure.

### Beispiel 20

### 3-{4-[1-(3-Fluoro-phenoxymethyl-cyclopropylmethoxy]-phenyl}-hex-4-ynoylsäure

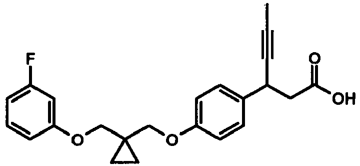

Analog zu Beispiel 10 erhielt man aus 1,1-Bis(hydroxymethylcyclopropan), 3-Fluorphenol und 3-(4-Hydroxy-phenyl)-hex-4-ynoylsäuremethylester 3-{4-[1-(3-Fluoro-phenoxymethyl)-cyclopropylmethoxy]-phenyl}-hex-4-ynoylsäure.

### Beispiel 21

### 3-{4-[1-(2-Fluoro-phenoxymethyl)-cyclopropylmethoxy]-phenyl}-hex-4-ynoylsäure

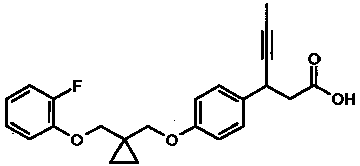

Analog zu Beispiel 10 erhielt man aus 1,1-Bis(hydroxymethylcyclopropan), 2-Fluorphenol und 3-(4-Hydroxy-phenyl)-hex-4-ynoylsäuremethylester 3-{4-[1-(2-Fluoro-phenoxymethyl)-cyclopropylmethoxy]-phenyl}-hex-4-ynoylsäure.

### Beispiel 22

### 3-{4-[1-(3-Chlor-4-fluor-phenoxymethyl)-cyclopropylmethoxy]-pheny}-hex-4-ynoylsäure

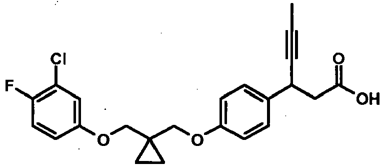

Analog zu Beispiel 10 erhielt man aus 1,1-Bis(hydroxymethylcyclopropan), 3-Chlor-4-fluorphenol und 3-(4-Hydroxy-phenyl)-hex-4-ynoylsäuremethylester 3-{4-[1-(3-Chlor-4-fluor-phenoxymethyl)-cyclopropylmethoxy]-phenyl]-hex-4-ynoylsäure.

### Beispiel 23

### 3-{4-[1-(4-fluor-2-methyl-phenoxymethyl)-cyclopropylmethoxy]-phenyl}-hex-4-ynoylsäure

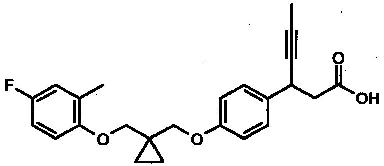

Analog zu Beispiel 10 erhielt man aus 1,1-Bis(hydroxymethylcyclopropan), 4-Fluor-2-methylphenol und 3-(4-Hydroxy-phenyl)-hex-4-ynoylsäuremethylester 3-{4-[1-(4-fluor-2-methyl-phenoxymethyl)-cyclopropylmethoxy]-phenyl}-hex-4-ynoylsäure.

### Beispiel 24

### 3-{4-[1-(4-fluor-3-methyl-phenoxymethyl)-cyclopropylmethoxy]-phenynl}-hex-4-ynoylsäure

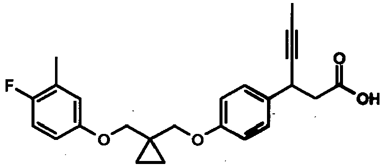

Analog zu Beispiel 10 erhielt man aus 1,1-Bis(hydroxymethylcyclopropan), 4-Fluor-3-methylphenol und 3-(4-Hydroxy-phenyl)-hex-4-ynoylsäuremethylester 3-{4-[1-(4-fluor-3-methyl-phenoxymethyl)-cyclopropylmethoxy]-phenyl}-hex-4-ynoylsäure.

### Beispiel 25

### 3-{4-[1-(2-chlor-4-fluor-phenoxymethyl)-cyclopropylmethoxyl]-phenyl}-hex-4-ynoylsäure

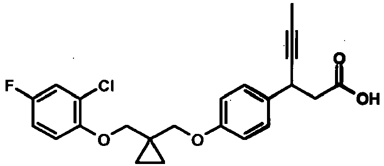

Analog zu Beispiel 10 erhielt man aus 1,1-Bis(hydroxymethylcyclopropan), 2-Chlor-4-fluor-phenol und 3-(4-Hydroxy-phenyl)-hex-4-ynoylsäuremethylester 3-{4-[1-(2-Chlor-4-fluor-phenoxymethyl)-cyclopropylmethoxy]-phenyl}-hex-4-ynoylsäure.

### Beispiel 26

### 3-{4-[1-(3-Trifluormethyl-phenoxymethyl)-cyclopropylmethoxy]-phenyl}-hex-4-ynoylsäure

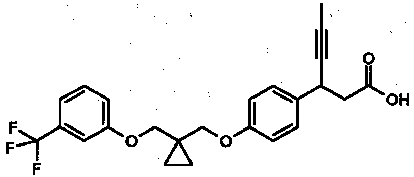

Analog zu Beispiel 10 erhielt man aus 1,1-Bis(hydroxymethylcyclopropan), 3-Trifluormethyl-phenol und 3-(4-Hydroxy-phenyl)-hex-4-ynoylsäuremethylester 3-{4-[1-(3-Trifluormethyl-phenoxymethyl)-cyclopropylmethoxy]-phenyl}-hex-4-ynoylsäure.

### Beispiel 27

### 3-{4-[1-(Pyridin-2-yloxymethyl)-cyclopropylmethoxy]-phenyl}-hex-4-ynoylsäure

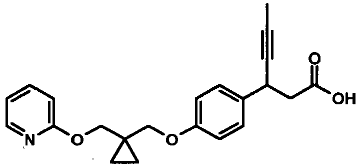

Analog zu Beispiel 10 erhielt man aus 1,1-Bis(hydroxymethylcyclopropan), 2-Hydroxypyridin und 3-(4-Hydroxy-phenyl)-hex-4-ynoylsäuremethylester 3- {4-[1-(Pyridin-2-yloxymethyl)-cyclopropylmethoxy]-phenyl}-hex-4-ynoylsäure.

### Beispiel 28

### 3-{4-[1-(3,5-Dichlor-pyridin-2-yloxymethyl)-cyclopropylmethoxy]-phenyl}-3-methoxy-propionsäure

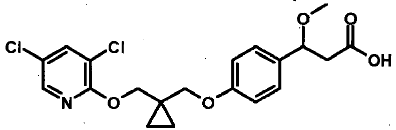

Analog zu Beispiel 16 erhielt man aus 1,1-Bis(hydroxymethylcyclopropan); 3,5-Dichlor-2-fluor-pyridin und 3-(4-Hydroxy-phenyl)-3-methoxy-propionsäureethylester 3-{4-[1-(3,5-Dichlor-pyridin-2-yloxymethyl)-cyclopropylmethoxy]-phenyl}-3-methoxy-propionsäure

### Beispiel 29

### 3-Methoxy-3-[4-(1-phenoxymethyl-cyclopropylmethoxy)-phenyl]-propionsäure

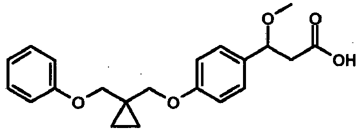

Analog zu Beispiel 10 erhielt man aus 1,1-Bis(hydroxymethylcyclopropan, Phenol und 3-(4-Hydroxy-phenyl)-3-methoxy-propionsäureethylester 3-Methoxy-3-[4-(1-phenoxymethylcyclopropylmethoxy)-phenyl]-propionsäure

### Beispiel 30

### 3-{4-[1-(4-Fluor-phenoxymethyl)-cyclopropylmethoxy]-phenyl}-3-methoxy-propionsäure

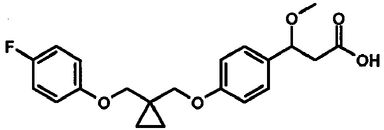

Analog zu Beispiel 10 erhielt man aus 1,1-Bis(hydroxymethylcyclopropan, 4-Fluor-phenol und 3-(4-Hydroxy-phenyl)-3-methoxy-propionsäureethylester 3- {4-[1-(4-Fluor-phenoxymethyl)-cyclopropylmethoxy]-phenyl}-3-methoxy-propionsäure

### Beispiel 31

### 3-{4-[1-(3-Fluor-phenoxymethyl)-cyclopropylmethoxy]-phenyl]-3-methoxy-propionsäure

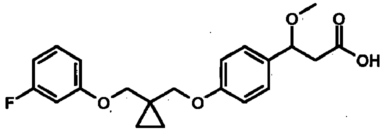

Analog zu Beispiel 10 erhielt man aus 1,1-Bis(hydroxymethylcyclopropan, 3-Fluor-phenol und 3-(4-Hydroxy-phenyl)-3-methoxy-propionsäureethylester 3-{4-[1-(3-Fluor-phenoxymethyl)-cyclopropylmethoxy]-phenyl}-3-methoxy-propionsäure

### Beispiel 32

### 3-{4-[1-(3,5-Dichlor-pyridin-2-yloxymethyl)-cyclopropylmethoxy]-phenyl}-hex-4-ynoylsäure

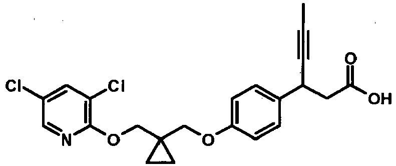

Analog zu Beispiel 10 erhielt man aus 1,1-Bis(hydroxymethylcyclopropan), 3,5-Dichlor-2-hydroxypyridin und 3-(4-Hydroxy-phenyl)-hex-4-ynoylsäuremethylester 3-{4-[1-(3,5-Dichlorpyridin-2-yloxymethyl)-cyclopropylmethoxy]-phenyl}-hex-4-ynoylsäure.

## Patentansprüche

1. Verbindungen der Formel I, 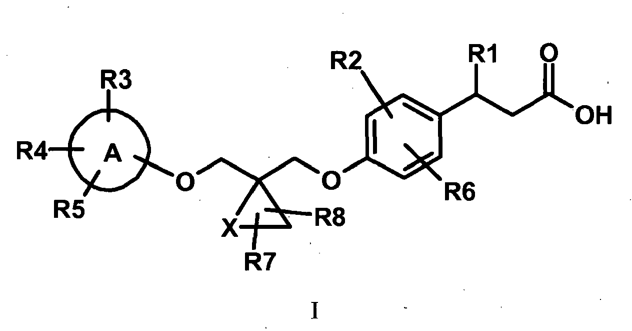 worin bedeuten
R1 O-(C₁-C₆)-Alkyl, (C₂-C₆)-Alkinyl, (C₂-C₆)-Alkinylen-(C₃-C₁₀)-Cycloalkyl, wobei der O-(C₁-C₆)-Alkylrest, der (C₂-C₆)-Alkinylrest und der (C₂-C₆)-Alkinylen-(C₃-C₁₀)-Cycloalkylrest einfach oder mehrfach mit Fluor substituiert sein können;
R2, R6 unabhängig voneinander H, F, Cl, Br, CN, (C₁-C₆)-Alkyl, O-(C₁-C₆)-Alkyl, CO-(C₁-C₆)-Alkyl, wobei der (C₁-C₆)-Alkylrest, O-(C₁-C₆)-Alkylrest und der CO-(C₁-C₆)-Alkylrest einfach oder mehrfach mit Fluor substituiert sein können;
R3, R4, R5 unabhängig voneinander H, F, Cl, Br, I, NO₂, CN, O-(C₁-C₆)-Alkyl, (C₁-C₆)-Alkyl, (C₁-C₃)-Alkylen-(C₃-C₆)-cycloalkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₁₀)-Cycloalkyl, oder ein 4 bis 12-gliedriger Heterocyclus, wobei der (C₁-C₆)-Alkylrest, der O-(C₁-C₆)-Alkylrest, der (C₁-C₃)-Alkylen-(C₃-C₆)-cycloalkylrest, der NH(C₁-C₆)-Alkylrest, der N((C₁-C₆)-Alkyl)₂rest, der SO₂-CH₃-rest, der SO₂-NH(C₁-C₆)-Alkylrest, SO₂-N((C₁-C₆)-Alkyl)₂-rest, COO-(C₁-C₆)-Alkylrest, der CONH(C₁-C₆)-Alkylrest, und der CON((C₁-C₆)-Alkyl)₂rest jeweils ein oder mehrfach mit F substituiert sein können und wobei der (C₆-C₁₀)-Arylrest, der (C₃-C₁₀)-Cycloalkylrest und der 4 bis 12-gliedrige Heterocyclusrest jeweils ein bis 3-fach substituiert sein können mit
F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, S O₂-CH₃ SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂ oder SF₅;
R7, R8 unabhängig voneinander H, F;
A (C₆-C₁₀)-Aryl, (C₃-C₁₀)-Cycloalkyl, oder ein 4 bis 12-gliedriger Heterocyclus;
X -CH₂-, -CH₂-CH₂-, -CH₂O-, -OCH₂-, -CH₂S, -SCH₂-, -CH₂SO-, -SOCH₂-, -CH₂SO₂-, -SO₂CH₂-;
und deren physiologisch verträgliche Salze.

2. Verbindungen gemäß Anspruch 1, **dadurch gekennzeichnet, dass** darin bedeuten
R1 (C₂-C₆)-Alkinyl;
R2, R6 unabhängig voneinander H, F, Cl, Br, CN, (C₁-C₆)-Alkyl, O-(C₁-C₆)-Alkyl, CO-(C₁-C₆)-Alkyl, wobei der (C₁-C₆)-Alkylrest, O-(C₁-C₆)-Alkylrest und der CO-(C₁-C₆)-Alkylrest einfach oder mehrfach mit Fluor substituiert sein können;
R3, R4, R5 unabhängig voneinander H, F, Cl, Br, I, NO₂, CN, O-(C₁-C₆)-Alkyl, (C₁-C₆)-Alkyl, (C₁-C₃)-Alkylen-(C₃-C₆)-cycloalkyl, NH₂, NH(C₁-C₆)-Alkyl, N((C₁-C₆)-Alkyl)₂, SO₂-CH₃, SO₂-NH₂, SO₂-NH(C₁-C₆)₋Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₁₀)-Cycloalkyl, oder ein 4 bis 12-gliedriger Heterocyclus, wobei der (C₁-C₆)-Alkylrest, der O-(C₁-C₆)-Alkylrest, der (C₁-C₃)-Alkylen-(C₃-C₆)-cycloalkylrest, der NH(C₁-C₆)-Alkylrest, der N((C₁-C₆)-Alkyl)₂rest, der SO₂-CH₃-rest, der SO₂-NH(C₁-C₆)-Alkylrest, SO₂-N((C₁-C₆)-Alkyl)₂-rest, COO-(C₁-C₆)-Alkylrest, der CONH(C₁-C₆)-Alkylrest, und der CON((C₁-C₆)-Alkyl)₂rest jeweils ein oder mehrfach mit F substituiert sein können und wobei der (C₆-C₁₀)-Arylrest, der (C₃-C₁₀)-Cycloalkylrest und der 4 bis 12-gliedrige Heterocyclusrest jeweils ein bis 3-fach substituiert sein können mit
F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, S O₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂ oder SF₅;
R7, R8 unabhängig voneinander H, F;
A (C₆-C₁₀)-Aryl, (C₃-C₁₀)-Cycloalkyl, oder ein 4 bis 12-gliedriger Heterocyclus;
X -CH₂-, -CH₂-CH₂-, -CH₂O-, -OCH₂-, -CH₂S, -SCH₂-, -CH₂SO-, -SOCH₂-, -CH₂SO₂-, -SO₂CH₂-;
und deren physiologisch verträgliche Salze.

3. Verbindungen gemäß Anspruch 1 oder 2, **dadurch gekennzeichnet, dass** darin bedeuten
R1 (C₂-C₆)-Alkinyl;
R2, R6 unabhängig voneinander H, F, Cl, Br, CN, (C₁-C₆)-Alkyl, O-(C₁-C₆)-Alkyl, CO-(C₁-C₆)-Alkyl, wobei der (C₁-C₆)-Alkylrest, O-(C₁-C₆)-Alkylrest und der CO-(C₁-C₆)-Alkylrest einfach oder mehrfach mit Fluor substituiert sein können;
R3, R4, R5 unabhängig voneinander H, F, Cl, Br, I, NO₂, CN, O-(C₁-C₆)-Alkyl, (C₁-C₆)-Alkyl, (C₁-C₃)-Alkylen-(C₃-C₆)-Cycloalkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₁₀)-Cycloalkyl, oder ein 4 bis 12-gliedriger Heterocyclus, wobei der (C₁-C₆)-Alkylrest, der O-(C₁-C₆)-Alkylrest, der (C₁-C₃)-Alkylen-(C₃-C₆)-cycloalkylrest, der NH(C₁-C₆)-Alkylrest, der N((C₁-C₆)-Alkyl)₂rest, der SO₂-CH₃-rest, der SO₂-NH(C₁-C₆)-Alkylrest, SO₂-N((C₁-C₆)-Alkyl)₂-rest, COO-(C₁-C₆)-Alkylrest, der CONH(C₁-C₆)-Alkylrest, und der CON((C₁-C₆)-Alkyl)₂rest jeweils ein oder mehrfach mit F substituiert sein können und wobei der (C₆-C₁₀)-Arylrest, der (C₃-C₁₀)-Cycloalkylrest und der 4 bis 12-gliedrige Heterocyclusrest jeweils ein bis 3-fach substituiert sein können mit
F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, S O₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂ oder SF₅;
R7, R8 unabhängig voneinander H, F;
A Phenyl, Pyridyl;
X -CH₂-, -CH₂-CH₂-, -CH₂O-, -OCH₂-, -CH₂S, -SCH₂-, -CH₂SO-, -SOCH₂-, -CH₂SO₂-, -SO₂CH₂-;
und deren physiologisch verträgliche Salze.

4. Verbindungen gemäß einem oder mehreren der Ansprüche 1 bis 3, **dadurch gekennzeichnet, dass** darin bedeuten
R1 (C₂-C₆)-Alkinyl;
R2, R6 H;
R3, R4 unabhängig voneinander H, F, Cl, Br, I, CF₃, CN, CF₃, OCF₃, (C₁-C₆)-Alkyl;
R5 H;
R7, R8 H;
A Phenyl, Pyridyl;
X -CH₂-, -CH₂-CH₂-;
und deren physiologisch verträgliche Salze.

5. Verbindungen gemäß einem oder mehreren der Ansprüche 1 bis 4, **dadurch gekennzeichnet, dass** darin bedeuten
R1 (C₂-C₆)-Alkinyl;
R2, R6 H;
R3, R4 unabhängig voneinander H, F, Cl, Br, I, CF₃, CN, CF₃, OCF₃, (C₁-C₆)-Alkyl;
R5 H;
R7, R8 H;
A Phenyl;
X -CH₂-, -CH₂-CH₂-;
und deren physiologisch verträgliche Salze.

6. Verbindungen gemäß einem oder mehreren der Ansprüche 1 bis 4, **dadurch gekennzeichnet, dass** darin bedeuten
R1 (C₂-C₆)-Alkinyl;
R2, R6 H;
R3, R4 unabhängig voneinander H, F, Cl, Br, I, CF₃, CN, CF₃, OCF₃, (C₁-C₆)-Alkyl;
R5 H;
R7, R8 H;
A Pyridyl;
X -CH₂-, -CH₂-CH₂-;
und deren physiologisch verträgliche Salze.

7. Verbindungen gemäß Anspruch 1, **dadurch gekennzeichnet, dass** darin bedeuten
R1 O-(C₁-C₆)-Alkyl, (C₂-C₆)-Alkinyl;
R2, R6 H;
R3, R4 unabhängig voneinander H, F, Cl, Br, I, CF₃, CN, CF₃, OCF₃, (C₁-C₆)-Alkyl;
R5 H;
R7, R8 H;
A Phenyl, 2-Pyridyl, 3-Pyridyl;
X -CH₂-, -CH₂-CH₂-;
und deren physiologisch verträgliche Salze.

8. Verbindungen gemäß einem oder mehreren der Ansprüche 1 bis 7, zur Anwendung als Arzneimittel.

9. Arzneimittel enthaltend eine oder mehrere der Verbindungen gemäß einem oder mehreren der Ansprüche 1 bis 7.

10. Arzneimittel, gemäß Anspruch 9, **dadurch gekennzeichnet, dass** es mindestens einen weiteren Wirkstoff enthält.

11. Arzneimittel, gemäß Anspruch 10, **dadurch gekennzeichnet, dass** es als weiteren Wirkstoff eine oder mehrere Antidiabetika, hypoglykämische Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Modulator, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Modulatoren, NPY-Agonisten, MC4-Agonisten, Orexin-Rezeptor 1 Antagonisten, Orexin-Rezeptor 2 Antagonisten, H3-Agonisten, TNF-Agonisten, CRF-Antagonisten, CRF BP-Antagonisten, Urocortin-Agonisten, β3-Agonisten, CB1-Rezeptor Antagonisten, MSH (Melanocyt-stimulierendes Hormon)-Agonisten, CCK-A Modulatoren, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten, Lipase/Amylase-Inhibitoren, PPAR-Modulatoren, RXR-Modulatoren oder TR-β-Agonisten oder Amphetamine enthält.

12. Arzneimittel, gemäß Anspruch 10, **dadurch gekennzeichnet, dass** es als weiteren Wirkstoff Metformin, Arcabose, Glibenclamid, Glimepirid, Gliclazid, Gliquidon, Pioglitazon, Rosiglitazon, Exenatid, Miglitol, Vildagliptin, Sitagliptin, Repaglinid, Nateglinid oder Mitiglinid enthält.

13. Arzneimittel, gemäß Anspruch 10, **dadurch gekennzeichnet, dass** es als weiteren Wirkstoff Lixisenatide enthält.

14. Verbindungen gemäß einem oder mehreren der Ansprüche 1 bis 7 zur Blutzuckersenkung.

15. Verbindungen gemäß einem oder mehreren der Ansprüche 1 bis 7 zur Behandlung des Diabetes.

16. Verbindungen gemäß einem oder mehreren der Ansprüche 1 bis 7 zur Erhöhung der Insulinausschüttung.

17. Verfahren zur Herstellung eines Arzneimittels enthaltend eine oder mehrere der Verbindungen gemäß einem oder mehreren der Ansprüche 1 bis 7, **dadurch gekennzeichnet, dass** der Wirkstoff mit einem pharmazeutisch geeigneten Träger vermischt wird und diese Mischung in eine für die Verabreichung geeignete Form gebracht wird.

18. Set (Kit) bestehend aus getrennten Packungen von
a) einer wirksamen Menge einer Verbindung der Formel I, gemäß einem oder mehreren der Ansprüche 1 bis 7 und
b) einer wirksamen Menge eines weiteren Arzneimittelswirkstoffs.

## Claims

1. A compound of the formula I 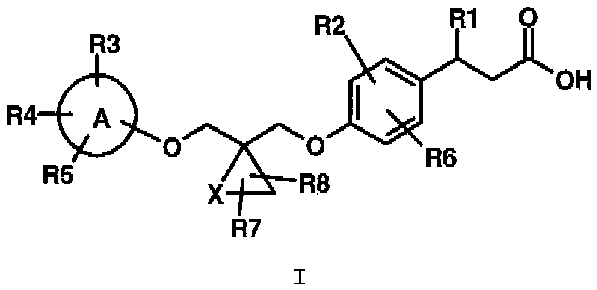 in which
R1 is O-(C₁-C₆)-alkyl, (C₂-C₆) -alkynyl, (C₂-C₆)-alkynylene-(C₁-C₁₀)-cycloalkyl, where the O-(C₁-C₆) -alkyl radical, the (C₂-C₆)-alkynyl radical and the (C₂-C₆)-alkynylene- (C₃-C₁₀) -cycloalkyl radical may be mono- or polysubstituted by fluorine;
R2, R6 are each independently H, F, Cl, Br, CN, (C₁-C₆)-alkyl, O-(C₁-C₆) -alkyl, CO- (C₁-C₆) -alkyl, where the (C₁-C₆)-alkyl radical, O-(C₁-C₆)-alkyl radical and the CO-(C₁-C₆)-alkyl radical may be mono- or polysubstituted by fluorine;
R3, R4, R5 are each independently H, F, Cl, Br, I, NO₂, CN, O- (C₁-C₆) -alkyl, (C₁-C₆) -alkyl, (C₁-C₃) - alkylene-(C₃-C₆)-cycloalkyl, NH₂, NH(C₁-C₆)-alkyl, N((C₁-C₆)-alkyl)₂, SO₂-CH₃, SO₂-NH₂, SO₂-NH(C₁-C₆) -alkyl, SO₂-N((C₁-C₆)-alkyl)₂, COOH, COO-(C₁-C₆)-alkyl, CONH₂, CONH(C₁-C₆) -alkyl, CON((C₁-C₆)-alkyl)₂, SF₅, (C₆-C₁₀)-aryl, (C₃-C₁₀)-cycloalkyl, or a 4- to 12-membered heterocycle, where the (C₁-C₆)-alkyl radical, the O-(C₁-C₆)-alkyl radical, the (C₁-C₃)-alkylene-(C₃-C₆)-cycloalkyl radical, the NH(C₁-C₆)-alkyl radical, the N((C₁-C₆)-alkyl)₂ radical, the SO₂-CH₃ radical, the SO₂-NH(C₁-C₆)-alkyl radical, SO₂-N((C₁-C₆)-alkyl)₂ radical, COO-(C₁-C₆)-alkyl radical, the CONH(C₁-C₆)-alkyl radical and the CON((C₁-C₆)-alkyl)₂ radical may each be mono- or polysubstituted by F, and where the (C₆-C₁₀)-aryl radical, the (C₃-C₁₀)-cycloalkyl radical and the 4- to 12-membered heterocycle radical may each be mono- to trisubstituted by
F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆) -alkyl, CON((C₁-C₆)-alkyl)₂ or SF₅;
R7, R8 are each independently H, F;
A is (C₆-C₁₀) -aryl, (C₃-C₁₀)-cycloalkyl, or a 4- to 12-membered heterocycle;
X is -CH₂-, -CH₂-CH₂-, -CH₂O-, -OCH₂-, -CH₂S, -SCH₂--CH₂SO-, -SOCH₂-, -CH₂SO₂-, -SO₂CH₂-;
and physiologically compatible salts thereof.

2. A compound as claimed in claim 1, wherein
R1 is (C₂-C₆)-alkynyl ;
R2, R6 are each independently H, F, Cl, Br, CN, (C₁-C₆) -alkyl, O-(C₁-C₆)-alkyl, CO-(C₁-C₆)-alkyl, where the (C₁-C₆)-alkyl radical, O-(C₁-C₆) -alkyl radical and the CO-(C₁-C₆)-alkyl radical may be mono- or polysubstituted by fluorite;
R3, R4, R5 are each independently H, F, Cl, Br, I, NO₂, CN, O-(C₁-C₆) -alkyl, (C₁-C₆) -alkyl, (C₁-C₃) - alkylene-(C₃-C₆)-cycloalkyl, NH₂, NH(C₁-C₆)-alkyl, N((C₁-C₆)-alkyl)₃, SO₂-CH₃, SO₂-NH₂, SO₂-NH(C₁-C₆) -alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅, (C₆-C₁₀)-aryl, (C₃-C₁₀)-cycloalkyl, or a 4- to 12-membered heterocycle, where the (C₁-C₆) -alkyl radical, the O-(C₁-C₆)-alkyl radical, the (C₁-C₃)-alkylene-(C₃-C₆)-cycloalkyl radical, the NH(C₁-C₆)-alkyl radical, the N((C₁-C₆)-alkyl)₂ radial, the SO₂-CH₃ radical, the SO₂-NH(C₁-C₆)-alkyl radical, SO₂-N((C₁-C₆)-alkyl)₃ radial, COO-(C₁-C₆)-alkyl radical, the CONH(C₁-C₆)-alkyl radical and the CON((C₁-C₆)-alkyl)₂ radical may each be mono- or polysubstituted by F, and where the (C₆-C₁₀)-aryl radical, the (C₃-C₁₀)-cycloalkyl radical and the 4- to 12-membered heterocycle radical may each be mono- to trisubstituted by
F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆) -alkyl, N((C₁-C₆)-alkyl)₃, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂ or SF₅;
R7, R8 are each independently H, F;
A is (C₆-C₁₀)-aryl, (C₃-C₁₀)-cycloalkyl, or a 4- to 12-membered heterocycle;
X is -CH₃-, -CH₂-CH₂-, -CH₂O-, -OCH₂-, -CH₂S, -SCH₂-, -CH₂SO-, -SOCH₂-, -CH₂SO₂-, -SO₂CH₂-;
and physiologically compatible salts thereof.

3. A compound as claimed in claim 1 or 2, wherein
R1 is (C₂-C₆) -alkynyl;
R2, R6 are each independently H, F, Cl, Br, CN, (C₁-C₆)-alkyl, O-(C₁-C₆)-alkyl, CO-(C₁-C₆)-alkyl, where the (C₁-C₆)-alkyl radical, O-(C₁-C₆)-alkyl radical and the CO-(C₁-C₆)-alkyl radical may be mono- or polysubstituted by fluorine;
R3, R4, R5 are each independently H, F, Cl, Br, I, NO₂, CN, O-(C₁-C₆)-alkyl, (C₁-C₆)-alkyl, (C₁-C₃)-alkylene-(C₃-C₆)-cycloalkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆) -alkyl)₂, COOH, COO-(C₁-C₆)-alkyl, CONH₂, CONH(C₁-C₆)-alkyl, CON((C₁-C₆)-alkyl)₂, SF₅, (C₆-C₁₀)-aryl, (C₃-C₁₀) - cycloalkyl, or a 4- to 12-membered heterocycle, where the (C₁-C₆) -alkyl radical, the O-(C₁-C₆)-alkyl radical, the (C₁-C₃)-alkylene-(C₃-C₆)-cycloalkyl radical, the NH(C₁-C₆) -alkyl radical, the N((C₁-C₆)-alkyl)₂ radical, the SO₂-CH₃ radical, the SO₂-NH(C₁-C₆)-alkyl radical, SO₂-N((C₁-C₆)-alkyl)₂ radical, COO-(C₁-C₆)-alkyl radical, the CONH(C₁-C₆)-alkyl radical and the CON((C₁-C₆)-alkyl)₂ radical may each be mono- or polysubstituted by F, and where the (C₆-C₁₀)-aryl radical, the (C₃-C₁₀)-cycloalkyl radical and the 4- to 12-membered heterocycle radical may each be mono- to trisubstituted by
F, Cl, Br, I, OH, CF₃, CHF₂, CH₂F, NO₂, CN, OCF₃, OCHF₃, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂ or SF₅;
R7, R8 are each independently H, F;
A is phenol, pyridyl;
X is -CH₂-, -CH₂-CH₂-, -CH₂O-, -OCH₂-, -CH₂S, -SCH₂-, -CH₂SO-, -SOCH₂-, -CH₂SO₂-, -SO₂CH₂- ;
and physiologically compatible salts thereof.

4. A compound as claimed in one or more of claims 1 to 3, wherein
R1 is (C₂-C₆)-alkynyl ;
R2, R6 is H;
R3, R4 are each independently H, F, Cl, Br, I, CF₃, CN, CF₃ , OCF₃, (C₁-C₆)-alkyl;
R5 is H;
R7, R8 is H;
A is phenyl, pyridyl;
X is -CH₂-, -CH₂-CH₂-;
and physiologically compatible salts thereof.

5. A compound as claimed in one or more of claims 1 to 4, wherein
R1 is (C₂-C₆)-alkynyl;
R2, R6 is H;
R3, R4 are each independently H, F, Cl, Br, I, CF₃, CN, CF₃, OCF₃, (C₁-C₆)-alkyl;
R5 is H;
R7, R8 is H;
A is phenyl;
X is -CH₂-, -CH₂-CH₂- ;
and physiologically compatible salts thereof.

6. A compound as claimed in one or more of claims 1 to 4, wherein
R1 is (C₂-C₆)-alkynyl;
R2, R6 is H;
R3, R4 are each independently H, F, Cl, Br, I, CF₃, CN, CF₃, OCF₃, (C₁-C₆)-alkyl;
R5 is H;
R7, R8 is H;
A is pyridyl
X is -CH₂-, -CH₂-CH₂-;
and physiologically compatible salts thereof.

7. A compound as claimed in claim 1, wherein
R1 is O- (C₁-C₆) -alkyl, (C₂-C₆)-alkynyl;
R2, R6 is H;
R3, R4 are each independently H, F, Cl, Br, I, CF₃, CN, CF₃, OCF₃ , (C₁-C₆)-alkyl;
R5 is H;
R7, R8 is H;
A is phenol, 2-pyridyl, 3-pyridyl;
X is -CH₂-, -CH₂-CH₂-;
and physiologically compatible salts thereof.

8. A compound as claimed in one or more of claims 1 to 7 for use as a medicament.

9. A medicament comprising one or more compounds as claimed in one or more of claims 1 to 7.

10. The medicament as claimed in claim 9, which comprises at least one further active ingredient.

11. The medicament as claimed in claim 10, which comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbers, LDL receptor inducers, ACAT inhibitors, antioxidants, lipoprotein lipase modulators, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose 1,6-biphosphatase, modulators of glucose transporter 4, inhibitors of glutamine:fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART modulators, NPY agonists, MC4 agonists, orexin receptor 1 antagonists, orexin receptor 2 antagonists, H3 agonists, TNF agonists, CRF antagonists, CRF BP antagonists, urocortin agonists, β3 agonists, CB1 receptor antagonists, MSH (melanocyte-stimulating hormone) agonists, CCK-A modulators, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists, lipase/amylase inhibitors, PPAR modulators, RXR modulators or TR-β-agonists or amphetamines

12. The medicament as claimed in claim 10, which comprises, as a further active ingredient, metformin, arcabose, glibenclamide, glimepiride, gliclazide, gliquidone, pioglitazone, rosiglitazone, exenatide, miglitol, vildagliptin, sitagliptin, repaglinide, nateglinide or mitiglinide.

13. The medicament as claimed in claim 10, which comprises, as a further active ingredient, lixisenatide.

14. The compound as claimed in one or more of claims 1 to 7 for lowering blood glucose.

15. The compound as claimed in one or more of claims 1 to 7 for treatment of diabetes.

16. The compound as claimed in one or more of claims 1 to 7 for increasing insulin excretion.

17. A process for producing a medicament comprising one or more of the compounds as claimed in one or more of claims 1 to 7, wherein the active ingredient is mixed with a pharmaceutically suitable carrier and this mixture is converted to a form suitable for administration.

18. A kit composed of separate packages of
a) an effective amount of a compound of the formula I as claimed in one or more of claims 1 to 7 and
b) an effective amount of a further medicinal active ingredient.

## Revendications

1. Composés de formule I 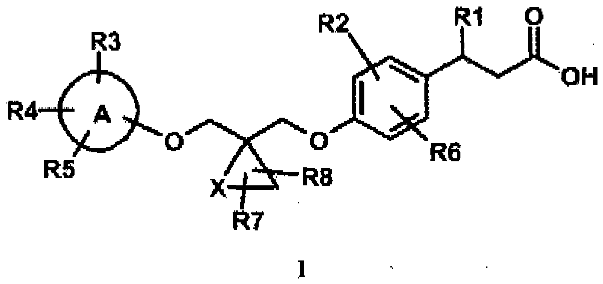 dans laquelle
R1 signifie O-alkyle en (C₁-C₆), alcynyle en (C₂-C₆), alcynylène en (C₂-C₆)-cycloalkyle en (C₃-C₁₀), le radical O-allyle en (C₁-C₆), le radical alcynyle en (C₂-C₆) et le radical alcynylène en (C₂-C₆)-cycloalkyle en (C₃-C₁₀) pouvant être substitués une ou plusieurs fois avec du fluor ;
R2, R6 signifient indépendamment l'un de l'autre H, F, Cl, Br, CN, alkyle en (C₁-C₆), O-alkyle en (C₁-C₆), CO-alkyle en (C₁-C₆), le radical alkyle en (C₁-C₆), le radical O-alkyle en (C₁-C₆) et le radical CO-alkyle en (C₁-C₆) pouvant être substitués une ou plusieurs fois avec du fluor ;
R3, R4, R5 signifient indépendamment les uns des autres H, F, Cl, Br, I, NO₂, CN, O-alkyle en (C₁-C₆), alkyle en (C₁-C₆), alkylène en (C₁-C₃)-cycloalkyle en (C₃-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON-(alkyle en (C₁-C₆))₂, SF₅, aryle en (C₆-C₁₀), cycloalkyle en (C₃-C₁₀) ou un hétérocycle de 4 à 12 éléments, le radical alkyle en (C₁-C₆), le radical O-alkyle en (C₁-C₆), le radical alkylène en (C₁-C₃)-cycloalkyle en (C₃-C₆), le radical NH-alkyle en (C₁-C₆), le radical N-(alkyle en (C₁-C₆))₂, le radical SO₂-CH₃, le radical SO₂-NH-alkyle en (C₁-C₆), le radical SO₂-N(alkyle en (C₁-C₆))₂, le radical COO-alkyle en (C₁-C₆), le radical CONH-alkyle en (C₁-C₆) et le radical CON(alkyle en (C₁-C₆))₂ pouvant chacun être substitués une ou plusieurs fois avec F, et le radical aryle en (C₆-C₁₀), le radical cycloalkyle en (C₃-C₁₀) et le radical hétérocycle de 4 à 12 éléments pouvant chacun être substitués une à 3 fois avec
F, Cl, Br, I, OH, CF₃, CHF₂, CH₂F, NO₂, CN, OCF₃, OCHF₂, O-alkyle en (C₁-C₆) , alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂ ou SF₅ ;
R7, R8 signifient indépendamment l'un de l'autre H, F ;
A signifie aryle en (C₆-C₁₀), cycloalkyle en (C₃-C₁₀) ou un hétérocycle de 4 à 12 éléments ;
X signifie -CH₂-, -CH₂-CH₂-, -CH₂O-, -OCH₂-, -CH₂S, - SCH₂-, -CH₂SO-, -SOCH₂-, -CH₂SO₂-, -SO₂CH₂- ;
et leurs sels physiologiquement acceptables.

2. Composés selon la revendication 1, **caractérisés en ce que**
R1 signifie alcynyle en (C₂-C₆) ;
R2, R6 signifient indépendamment l'un de l'autre H, F, Cl, Br, CN, alkyle en (C₁-C₆), O-allyle en (C₁-C₆), CO-alkyle en (C₁-C₆), le radical alkyle en (C₁-C₆), le radical O-alkyle en (C₁-C₆) et le radical CO-alkyle en (C₁-C₆) pouvant être substitués une ou plusieurs fois avec du fluor ;
R3, R4, R5 signifient indépendamment les uns des autres H, F, Cl, Br, I, NO₂, CN, O-alkyle en (C₁-C₆), alkyle en (C₁-C₆), alkylène en (C₁-C₃)-cycloalkyle en (C₃-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₃-N(alkyle en (C₁-C₆))₂, COOH, COO-alkyle en (C₂-C₆), CONH₂, CONH-alkyle en (C₁-C₆), CON-(alkyle en (C₁-C₆))₂, SF₅, aryle en (C₆-C₁₀), cycloalkyle en (C₃-C₁₀) ou un hétérocycle de 4 à 12 éléments, le radical alkyle en (C₁-C₆), le radical O-alkyle en (C₁-C₆), le radical alkylène en (C₁-C₃)-cycloalkyle en (C₃-C₆), le radical NH-alkyle en (C₁-C₆), le radical N(alkyle en (C₁-C₆))₂, le radical SO₂-CH₃, le radical SO₂-NH-alkyle en (C₁-C₆), le radical SO₂-N(alkyle en (C₁-C₆))₂, le radical COO-alkyle en (C₁-C₆), le radical CONH¬alkyle en (C₁-C₆) et le radical CON(alkyle en (C₁-C₆))₂ pouvant chacun être substitués une ou plusieurs fois avec F, et le radical aryle en (C₆-C₁₀), le radical cycloalkyle en (C₃-C₁₀) et le radical hétérocycle de 4 à 12 éléments pouvant chacun être substitués une à 3 fois avec
F, Cl, Br, I, OH, CF₃, CHF₂, CH₂F, NO₂, CN, OCF₃, OCHF₂, O-allyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂¬NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂ ou SF₅ ;
R7, R8 signifient indépendamment l'un de l'autre H, F ;
A signifie aryle en (C₆-C₁₀), cycloalkyle en (C₃-C₁₀) ou un hétérocycle de 4 à 12 éléments ;
X signifie -CH₂-, -CH₂-CH₂-, -CH₂O-, -OCH₂-, -CH₂S, -SCH₂-, -CH₂SO-, -SOCH₂-, -CH₂SO₂-, -SO₂CH₂- ;
et leurs sels physiologiquement acceptables.

3. Composés selon la revendication 1 ou 2, **caractérisés en ce que**
R1 signifie alcynyle en (C₂-C₆) ;
R2, R6 signifient indépendamment l'un de l'autre H, F, Cl, Br, CN, alkyle en (C₁-C₆) , O-alkyle en (C₁-C₆), CO-alkyle en (C₁-C₆) , le radical alkyle en (C₁-C₆), le radical O-alkyle en (C₁-C₆) et le radical CO-alkyle en (C₁-C₆) pouvant être substitués une ou plusieurs fois avec du fluor ;
R3, R4, R5 signifient indépendamment les uns des autres H, F, Cl, Br, I, NO₂, CN, O-alkyle en (C₁-C₆), alkyle en (C₁-C₆), alkylène en (C₁-C₃)-cycloalkyle en (C₃-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON-(alkyle en (C₁-C₆))₂, SF₅, aryle en (C₆-C₁₀), cycloalkyle en (C₃-C₁₀) ou un hétérocycle de 4 à 12 éléments, le radical alkyle en (C₁-C₆), le radical O-alkyle en (C₁-C₆) , le radical alkylène en (C₁-C₃)-cycloalkyle en (C₃-C₆), le radical NH-alkyle en (C₁-C₆), le radical N-(alkyle en (C₁-C₆))₂, le radical SO₂-CH₃, le radical SO₂-NH-alkyle en (C₁-C₆), le radical SO₂-N(alkyle en (C₁-C₆))₂, le radical COO-alkyle en (C₁-C₆), le radical CONH-alkyle en (C₁-C₆) et le radical CON(alkyle en (C₁-C₆))₂ pouvant chacun être substitués une ou plusieurs fois avec F, et le radical aryle en (C₆-C₁₀), le radical cycloalkyle en (C₃-C₁₀) et le radical hétérocycle de 4 à 12 éléments pouvant chacun être substitués une à 3 fois avec
F, Cl, Br, I, OH, CF₃, CHF₂, CH₂F, NO₂, CN, OCF₃, OCHF₂, O-alkyle en (C₁-C₆), alkyle en (C₁-C₆) , NH₂, NH-alkyle en (C₁-C₆), N(alkyle en (C₁-C₆))₂, SO₂-CH₃, SO₂-NH₂, SO₂-NH-alkyle en (C₁-C₆), -SO₂-N(alkyle en (C₁-C₆))₂, COOH, COO-alkyle en (C₁-C₆), CONH₂, CONH-alkyle en (C₁-C₆), CON(alkyle en (C₁-C₆))₂ ou SF₅;
R7, R8 signifient indépendamment l'un de l'autre H, F ;
A signifie phényle, pyridyle ;
X signifie -CH₂-, -CH₂-CH₂-, -CH₂O-, -OCH₂-, -CH₂S, -SCH₂-, -CH₂SO-, -SOCH₂-, -CH₂SO₂-, -SO₂CH₂- ;
et leurs sels physiologiquement acceptables.

4. Composés selon une ou plusieurs des revendications 1 à 3, **caractérisés en ce que**
R1 signifie alcynyle en (C₂-C₆);
R2, R6 signifient H ;
R3, R4 signifient indépendamment les uns des autres H, F, Cl, Br, I, CF₃, CN, CF₃, OCF₃, alkyle en (C₁-C₆) ;
R5 signifie H ;
R7, R8 signifient H ;
A signifie phényle, pyridyle ;
X signifie -CH₂-, -CH₂-CH₂- ;
et leurs sels physiologiquement acceptables.

5. Composés selon une ou plusieurs des revendications 1 à 4, **caractérisés en ce que**
R1 signifie alcynyle en (C₂-C₆) ;
R2, R6 signifient H ;
R3, R4 signifient indépendamment l'un de l'autre H, F, Cl, Br, I, CF₃, CN, CF, OCF₃, alkyle en (C₁-C₆) ;
R5 signifie H ;
R7, R8 signifient H ;
A signifie phényle ;
X signifie -CH₂-, -CH₂-CH₂- ;
et leurs sels physiologiquement acceptables.

6. Composés selon une ou plusieurs des revendications 1 à 4, **caractérisés en ce que**
R1 signifie alcynyle en (C₂-C₆) ;
R2, R6 signifient H ;
R3, R4 signifient indépendamment l'un de l'autre H, F, Cl, Br, I, CF₃, CN, CF₃, OCF₃, alkyle en (C₁-C₆);
R5 signifie H ;
R7, R8 signifient H ;
A signifie pyridyle ;
X signifie -CH₂-, -CH₂-CH₂- ;
et leurs sels physiologiquement acceptables.

7. Composés selon la revendication 1, **caractérisés en ce que**
R1 signifie O-alkyle en (C₁-C₆), alcynyle en (C₂-C₆) ;
R2, R6 signifient H ;
R3, R4 signifient indépendamment l'un de l'autre H, F, Cl, Br, I, CF₃, CN, CF₃, OCF₃, alkyle en (C₁-C₆) ;
R5 signifie H ;
R7, R8 signifient H ;
A signifie phényle, 2-pyridyle, 3-pyridyle ;
X signifie -CH₂-, -CH₂-CH₂- ;
et leurs sels physiologiquement acceptables.

8. Composés selon une ou plusieurs des revendications 1 à 7, destinées à une utilisation en tant que médicament.

9. Médicament contenant un ou plusieurs des composés selon une ou plusieurs des revendications 1 à 7.

10. Médicament selon la revendication 9, **caractérisé en ce qu'**il contient au moins un agent actif supplémentaire.

11. Médicament selon la revendication 10, **caractérisé en ce qu'**il contient en tant qu'agent actif supplémentaire un ou plusieurs antidiabétiques, agents actifs hypoglycémiques, inhibiteurs d'HMGCoA-réductase, inhibiteurs de la résorption de cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption d'acide biliaire, inhibiteurs de CETP, adsorbeurs polymères d'acide biliaire, inducteurs des récepteurs de LDL, inhibiteurs d'ACAT, antioxydants, modulateurs de lipoprotéine lipase, inhibiteurs d'ATP citrate lyase, inhibiteurs de squalène synthétase, antagonistes de lipoprotéine (a), agonisés du récepteur HM74A, inhibiteurs de lipase, insulines, sulfonylurées, biguanides, méglitinides, thiazolidinediones, inhibiteurs d'α-glucosidase, agents actifs agissant sur le canal potassique ATP-dépendant des cellules bêta, inhibiteurs de glycogène phosphorylase, antagonistes des récepteurs de glucagon, activateurs de glucokinase, inhibiteurs de gluconéogenèse, inhibiteurs de fructose 1,6-biphosphatase, modulateurs du transporteur de glucose 4, inhibiteurs de glutamine fructose 6-phosphate amidotransférase, inhibiteurs de dipeptidylpeptidase IV, inhibiteurs de 11-bêta-hydroxystéroïde déshydrogénase 1, inhibiteurs de protéine tyrosine phosphatase 1B, modulateurs du transporteur de glucose 1 ou 2 dépendant du sodium, inhibiteurs de la lipase hormonosensible, inhibiteurs d'acétyl-CoA carboxylase, inhibiteurs de phosphoénolpyruvate-carboxykinase, inhibiteurs de glycogène synthase kinase-3 bêta, inhibiteurs de protéine kinase C bêta, antagonistes du récepteur d'endothéline A, inhibiteurs de I kappaB kinase, modulateurs du récepteur de glucocorticoïdes, modulateurs de CART, agonistes de NPY, agonistes de MC4, antagonistes du récepteur d'orexine 1, antagonistes du récepteur d'orexine 2, agonistes d'H3, agonistes de TNF, antagonistes de CRF, antagonistes de CRF BP, agonistes d'urocortine, agonistes de β3, antagonistes du récepteur de CB1, agonistes de MSH (hormone mélanotrope), modulateurs de CCK-A, inhibiteurs du recaptage de la sérotonine, composés mixtes sérotoninergiques et noradrénergiques, agonistes de 5HT, agonistes de bombésine, antagonistes de galanine, hormones de croissance, composés libérant des hormones de croissance, agonistes de TRH, modulateurs 2 ou 3 des protéines découplantes, agonistes de leptine, agonistes de DA, inhibiteurs de lipase/amylase, modulateurs de PPAR, modulateurs de RXR ou agonistes de TR-β ou amphétamines.

12. Médicament selon la revendication 10, **caractérisé en ce qu'**il contient en tant qu'agent actif supplémentaire de la metformine, de l'arcabose, du glibenclamide, du glimépiride, du gliclazide, de la gliquidone, de la pioglitazone, de la rosiglitazone, de l'exénatide, du miglitol, de la vildagliptine, de la sitagliptine, du répaglinide, du natéglinide ou du mitiglinide.

13. Médicament selon la revendication 10, **caractérisé en ce qu'**il contient en tant qu'agent actif supplémentaire du lixisénatide.

14. Composés selon une ou plusieurs des revendications 1 à 7 pour la réduction de la glycémie.

15. Composés selon une ou plusieurs des revendications 1 à 7 pour le traitement du diabète.

16. Composés selon une ou plusieurs des revendications 1 à 7 pour l'augmentation de la sécrétion d'insuline.

17. Procédé de fabrication d'un médicament contenant un ou plusieurs des composés selon une ou plusieurs des revendications 1 à 7, **caractérisé en ce que** l'agent actif est mélangé avec un support pharmaceutiquement approprié et ce mélange est mis sous une forme appropriée pour l'administration.

18. Ensemble (kit) constitué d'emballages séparés de
a) une quantité efficace d'un composé de formule I selon une ou plusieurs des revendications 1 à 7 et
b) une quantité efficace d'un agent actif médicamenteux supplémentaire.
